(19) Europäisches Patentamt
European Patent Office
Office européen des brevets

(11) **EP 4 461 202 B1**

(12) **EUROPEAN PATENT SPECIFICATION**

(45) Date of publication and mention
of the grant of the patent:
**08.04.2026 Bulletin 2026/15**

(21) Application number: **22918774.5**

(22) Date of filing: **09.12.2022**

(51) International Patent Classification (IPC):
*A61B 1/045* (2006.01)   *A61B 1/00* (2006.01)
*A61B 1/06* (2006.01)   *A61B 1/05* (2006.01)
*A61B 5/1455* (2006.01)   *A61B 5/1459* (2006.01)

(52) Cooperative Patent Classification (CPC):
**A61B 1/045; A61B 1/00; A61B 1/000094;
A61B 1/05; A61B 1/06; A61B 1/0638;
A61B 1/0655; A61B 1/0661; A61B 5/14552;
A61B 5/1459**

(86) International application number:
**PCT/JP2022/045537**

(87) International publication number:
**WO 2023/132188 (13.07.2023 Gazette 2023/28)**

(54) **ENDOSCOPE SYSTEM AND OPERATION METHOD OF SAME**

ENDOSKOPSYSTEM UND BETRIEBSVERFAHREN DAFÜR

SYSTÈME D'ENDOSCOPIE ET SON PROCÉDÉ DE FONCTIONNEMENT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **05.01.2022 JP 2022000464
18.08.2022 JP 2022130626**

(43) Date of publication of application:
**13.11.2024 Bulletin 2024/46**

(73) Proprietor: **FUJIFILM Corporation
Tokyo 106-8620 (JP)**

(72) Inventor: **ENDO, Maiko
Ashigarakami-gun, Kanagawa 258-8538 (JP)**

(74) Representative: **Dehns Germany Partnerschaft
mbB
Theresienstraße 6-8
80333 München (DE)**

(56) References cited:
JP-A- 2013 188 365   JP-A- 2013 188 365
JP-A- 2015 160 012   JP-A- 2016 007 353
JP-A- 2017 018 503   JP-B2- 6 412 252
JP-B2- 6 412 252

**Description**

**BACKGROUND OF THE INVENTION**

1. Field of the Invention

The present invention relates to an endoscope system that implements exposure control in the case where multiple kinds of image signals are obtained and a method of operating the endoscope system.

2. Description of the Related Art

In the medical field in which an endoscope is used, oxygen saturation imaging is known as a technique for facilitating detection of a lesion portion. As for the oxygen saturation imaging, an object to be observed is irradiated with narrow-range light, oxygen saturation is calculated from an endoscope image by using a difference in light absorption coefficient between oxyhemoglobin and deoxyhemoglobin in tissue to be observed, and a lesion such as a malignant tumor that has relatively low oxygen saturation is detected. The tissue includes specific pigment such as yellow pigment that differs from that of the oxyhemoglobin and the deoxyhemoglobin. The influence of light absorption by the specific pigment causes a problem in that the precision of calculation of the oxygen saturation reduces. To solve the problem, correction illumination light for calculating the influence of the specific pigment is radiated, and an algorithm for calculating the oxygen saturation is corrected based on a signal obtained by correction image pick-up (see JP6412252B).

Under known control on the amount of light, the target amount of light is calculated by using information about the brightness of an endoscope image obtained by performing image pick-up by using narrow-range light that is used for calculating the oxygen saturation, and brightness of multiple kinds of endoscope image signals chronologically obtained in subsequent frames is corrected in the case where multiple kinds of illumination light are sequentially emitted toward an object to be observed (see JP2013-188365A).

**SUMMARY OF THE INVENTION**

The optimal brightness of the image signals differs between the case where the oxygen saturation is calculated and the case where a correction calculation is made for calculating the oxygen saturation. A signal value including brightness that an image signal has needs to be appropriate in order to ensure the precision of the calculation of the oxygen saturation and the correction thereof. For this reason, when the brightness of another kind of an image signal is adjusted by using a specific kind of an image signal, a problem such as being too dark or being too bright may occur depending on the kind of the image signal to be obtained. Therefore, in the case where multiple kinds of image signals are obtained, there is a need for a technique that can obtain new image signals that are more suitable for the purposes of obtaining the respective image signals and that have reliable signal values.

The invention is defined by the independent claims. The dependent claims define advantageous embodiments.

The control amount is preferably outputted based on a difference between the image signal brightness calculated from the image signals and predetermined target brightness.

The processor is configured to: obtain observation image signals as the image signals in the first exposure period; obtain correction image signals as the image signals in the second exposure period; output a first control amount as the control amount, based on a difference between first image signal brightness that is the image signal brightness calculated by using the observation image signals and first target brightness that is the target brightness; output a second control amount as the control amount that differs from the first control amount, based on a difference between second image signal brightness that is the image signal brightness calculated by using the correction image signals and second target brightness that is the image signal brightness; generate first exposure control signals as the exposure control signals based on the first control amount; and generate second exposure control signals as the exposure control signals based on the second control amount. The second control amount is preferably larger than the first control amount.

The processor is preferably configured to output the control amount by using a specific color signal among the image signals. The specific color signal is preferably a B signal.

The processor is preferably configured to: generate an analysis image by using the image signals; extract a specific region from the analysis image; and output the control amount by using the image signals in the specific region. The specific region is preferably extracted so as to follow a specific shape.

The processor is preferably configured to: extract a first region as the specific region from a first analysis image that is the analysis image generated by using the observation image signals; output a first region control amount as the control amount, based on a difference between first region brightness calculated as the image signal brightness by using the first region and the first target brightness; extract a second region as the specific region from a second analysis image that is the analysis image generated by using the correction image signals; output a second region control amount as the control amount, based on a difference between second region brightness calculated as the image signal brightness by using the second region and the second target brightness; generate first region exposure control signals as the exposure control signals based on the first region control amount; and generate second region exposure control signals as the exposure control signals based on the second region control amount.

The processor is preferably configured to: switch between an observation mode in which the observation image signals are obtained and a correction mode in which the observation image signals and the correction image signals are obtained; generate the first exposure control signals in the observation mode; and generate the first exposure control signals and the second exposure control signals in the correction mode.

The first exposure period preferably includes a first illumination period in which substantially white light included in the observation illumination light is emitted and a second illumination period in which calculation illumination light included in the observation illumination light is emitted. The second exposure period preferably includes a third illumination period in which the correction illumination light is obtained. The processor is preferably configured to: automatically switch between the first illumination period, the second illumination period, and the third illumination period; obtain substantially white light image signals as the image signals in the first illumination period; obtain calculation image signals as the image signals in the second illumination period; obtain the correction image signals in the third illumination period; generate first A exposure control signals as the exposure control signals based on the substantially white light image signals; generate first B exposure control signals as the exposure control signals based on the calculation image signals; generate the second exposure control signals based on the correction image signals; and control the at least one or more light sources and the image pick-up optical system depending on the first A exposure control signals, the first B exposure control signals, and the second exposure control signals.

Control on the at least one or more light sources preferably includes control on an amount of light, and control on the image pick-up optical system preferably includes control on an aperture diaphragm value, control on an exposure time, and control on a gain.

A method of operating an endoscope system according to the present invention is a method of operating an endoscope system that illuminates an object to be observed and that performs image pick-up of reflection light from the object to be observed where a light source device, an image pick-up optical system, and a processor are included. The method includes the steps of: the light source device causing at least one or more light sources to produce light and sequentially emitting observation illumination light and correction illumination light having a spectrum that differs from a spectrum of the observation illumination light toward the object to be observed; the image pick-up optical system performing image pick-up of the reflection light; and the processor obtaining image signals of multiple kinds different from each other in a first exposure period in which the observation illumination light is emitted and in a second exposure period in which the correction illumination light is emitted, calculating image signal brightness from image signals that differ from each other among the image signals, outputting a control amount that changes depending on the image signal brightness, generating exposure control signals of multiple kinds having the control amounts different from each other, and controlling the at least one or more light sources and the image pick-up optical system depending on the exposure control signals.

According to the present invention, a reliable image signal can be obtained for every kind of image signals that are obtained in the case where multiple kinds of illumination light are emitted, and multiple kinds of the image signals are obtained.

## BRIEF DESCRIPTION OF THE DRAWINGS

Fig. 1 schematically illustrates an endoscope system;
Fig. 2 is a block diagram illustrating the function of the endoscope system;
Fig. 3 is a block diagram illustrating the structure of a light source unit;
Fig. 4 is a graph illustrating the spectrum of normal light;
Fig. 5 is a graph illustrating the spectrum of substantially white light;
Fig. 6 is a graph illustrating the spectrum of calculation illumination light;
Fig. 7 is a graph illustrating the spectrum of correction illumination light;
Fig. 8 illustrates a first light emission pattern;
Fig. 9 illustrates a second light emission pattern;
Fig. 10 illustrates a third light emission pattern;
Fig. 11 is a graph illustrating a blue transmittance range, a green transmittance range, and a red transmittance range;
Fig. 12 is a block diagram illustrating the function of a tissue oxygen saturation image generation section;
Fig. 13 is a graph illustrating the position of a contour for oxygen saturation in a two-dimensional space where an X-axis represents ln(R1/G1), and a Y-axis represents ln(B2/G1);

Fig. 14 is a graph illustrating light absorption coefficients of oxyhemoglobin and deoxyhemoglobin;

Fig. 15 illustrates a method of calculating the oxygen saturation;

Fig. 16 is a graph illustrating light absorption coefficient of the yellow pigment;

Fig. 17 is a graph illustrating the positions of curved surfaces in a three-dimensional space where an X-axis is $\ln(R1/G1)$, a Y-axis is $\ln(B2/G1)$, and a Z-axis is $\ln(B3/G3)$;

Fig. 18 is a block diagram illustrating the function of an exposure control unit;

Fig. 19 illustrates generation of exposure control signals;

Fig. 20 illustrates a method of calculating image signal brightness and a brightness difference and outputting a control amount;

Fig. 21 is a block diagram illustrating the function of a brightness calculation section;

Fig. 22 illustrates an endoscope image in a region in which analysis precision is high and a region in which the analysis precision is low;

Fig. 23 illustrates an image in the case where a specific region is a region obtained by removing outermost peripheral regions from a brightness calculation image region;

Fig. 24 illustrates an image in the case where the specific region is a donut-shaped region in the brightness calculation image region;

Fig. 25 illustrates an image in the case where the specific region is a region near the center of the brightness calculation image region;

Fig. 26 illustrates an example in which a first region is extracted from a first analysis image, and exposure control is implemented;

Fig. 27 illustrates an example in which a second region is extracted from a second analysis image, and the exposure control is implemented;

Fig. 28 illustrates the case where a first A exposure control signal, a first B exposure control signal, and a second exposure control signal are generated, and the exposure control is implemented;

Fig. 29 illustrates a specific content of the exposure control;

Fig. 30 is a flowchart illustrating the flow of the exposure control;

Fig. 31 illustrates an image that represents an example of a correction image;

Fig. 32 is a block diagram illustrating the functions of a correction image generation section, a reliability calculation section, and a correction determination section;

Fig. 33 is a graph illustrating a first reliability calculation table;

Fig. 34 is a graph illustrating a second reliability calculation table;

Fig. 35 is a graph illustrating a third reliability calculation table;

Fig. 36 illustrates an image that represents an example of the correction image in the case where the saturation of the correction image is changed depending on reliability;

Fig. 37 illustrates an image that represents an example of the correction image in the case where a correction specific region is surrounded by a border depending on the reliability;

Fig. 38 illustrates an image that represents an example of the correction image in the case where it is represented that a correction process can be appropriately performed;

Fig. 39 illustrates an image that represents an example of the correction image in the case where a warning is displayed;

Fig. 40 is a block diagram illustrating the function of an endoscope system according to a second embodiment;

Fig. 41 is a plan view of a rotation filter;

Fig. 42 illustrates a difference value $\Delta Z$ used in a process of correcting a calculated value;

Fig. 43 schematically illustrates an endoscope system according to a third embodiment;

Fig. 44 is a graph illustrating the spectrum of mixed light including first blue light, second blue light, green light, and red light;

Fig. 45 illustrates the function of a camera head according to the third embodiment;

Fig. 46 is a graph illustrating the spectrum of light that enters an image pick-up sensor;

Fig. 47 is a graph illustrating the spectrum of light that enters an image pick-up sensor;

Fig. 48 is a graph illustrating the spectrum of light that enters an image pick-up sensor;

Fig. 49 is a graph illustrating the spectrum of light that enters an image pick-up sensor;

Fig. 50 illustrates the function of a camera head according to a fourth embodiment;

Fig. 51A is a graph illustrating the spectrum of second mixed light, Fig. 51B is a graph illustrating a relationship among the reflectance and light transmittance of light that enters a dichroic mirror according to the fourth embodiment and the wavelength of light, and Fig. 51C is a graph illustrating a relationship between the sensitivity of an image pick-up sensor and the wavelength of light;

Fig. 52A is a graph illustrating the spectrum of the second mixed light, Fig. 52B is a graph illustrating a relationship among the reflectance and light transmittance of light that enters the dichroic mirror according to the fourth embodiment and the wavelength of light, and Fig. 52C is a graph illustrating a relationship between the sensitivity of an image pick-up sensor and the wavelength of light;

Fig. 53 illustrates a light emission pattern in an oxygen saturation mode according to the fourth embodiment;

Fig. 54 illustrates a light emission pattern in a correction mode according to the fourth embodiment;

Fig. 55A is a graph illustrating the spectrum of the correction illumination light, Fig. 55B is a graph illustrating a relationship among the reflectance and light transmittance of light that enters the dichroic mirror according to the fourth embodiment and the wavelength of light, and Fig. 55C is a graph illustrating the sensitivity of an image pick-up sensor and the wavelength of light;

Fig. 56A is a graph illustrating the spectrum of the correction illumination light, Fig. 56B is a graph illustrating a relationship among the reflectance and light transmittance of light that enters the dichroic mirror according to the fourth embodiment and the wavelength of light, and Fig. 56C is a graph illustrating the sensitivity of an image pick-up sensor and the wavelength of light;

Fig. 57 is a block diagram illustrating the function of an endoscope system according to the fourth embodiment;

Fig. 58A illustrates an image that represents a correction region, and Fig. 58B is an enlarged view of the correction region illustrated in Fig. 58A;

Fig. 59 is a block diagram illustrating the functions of a reliability calculation section, a correction determination section, and an extended display control unit according to the fourth embodiment;

Fig. 60 is a graph illustrating the first reliability calculation table where a lateral axis represents the signal value of a G1 image signal;

Fig. 61 is a graph illustrating the third reliability calculation table where a vertical axis represents a signal ratio ln(B2/G1);

Fig. 62 illustrates a relationship among a light emission pattern, an endoscope image generated, and an image set in the correction mode according to the fourth embodiment;

Fig. 63 illustrates the respective correction regions in a substantially white light image, a second blue light image, and a correction illumination light image;

Fig. 64 illustrates a method of calculating a correlation coefficient; and

Fig. 65 illustrates an image that represents an example of display in the case where a warning is displayed according to the fourth embodiment.

## DESCRIPTION OF THE PREFERRED EMBODIMENTS

First Embodiment

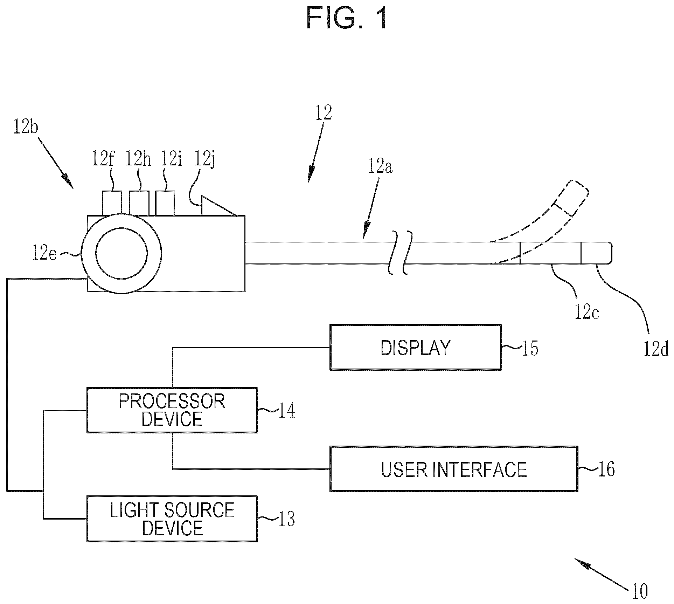

As illustrated in Fig. 1, an endoscope system 10 includes an endoscope 12, a light source device 13, a processor device 14, a display 15, and a user interface 16. The endoscope 12 is optically connected to the light source device 13 and is electrically connected to the processor device 14.

The light source device 13 supplies illumination light to the endoscope 12. The display 15 displays a normal light image, a substantially white light image, and/or a tissue oxygen saturation image (an oxygen saturation image and/or a correction oxygen saturation image). For example, the user interface 16 includes a keyboard, a mouse, a microphone, a tablet and a touch pen and receives an input operation of, for example, function settings. The processor device 14 implements control on the light source device 13 and control regarding image processing, analysis, and display on an image signal transmitted from the endoscope 12.

The endoscope 12 includes an insertion section 12a, an operation section 12b, a bending portion 12c, and a tip portion 12d. The insertion section 12a is inserted into the body of a photographic subject. The operation section 12b is provided at a proximal end portion of the insertion section 12a. The bending portion 12c and the tip portion 12d are provided at a distal end of the insertion section 12a. An angle knob 12e of the operation section 12b is operated, and consequently, the bending portion 12c bends. The bending portion 12c bends, and consequently, the tip portion 12d is directed in a desired direction. The tip portion 12d irradiates an object to be observed with the illumination light, receives reflection light from the object to be observed, and performs image pick-up of the object to be observed. A forceps channel (not illustrated) through which a treatment tool, for example, extends may be provided from the insertion section 12a to the tip portion 12d. The treatment tool is inserted into the forceps channel via a forceps port 12j.

The operation section 12b includes the angle knob 12e, a mode change switch 12f, a still image obtaining instruction switch 12h, and a zoom operation section 12i. The mode change switch 12f is used for an operation of switching a mode. The still image obtaining instruction switch 12h is used for an instruction for obtaining a still image of the photographic subject. The zoom operation section 12i is used to operate a zoom lens 42.

The endoscope system 10 has three modes of a normal observation mode (a normal mode), a tissue oxygen saturation observation mode (an observation mode), and a correction tissue oxygen saturation calculation mode (a correction mode). A user operates the mode change switch 12f, and consequently, the three modes are switched with a central control unit 50 used. In the normal mode, the object to be observed is irradiated with normal light that is wide-range white light, and an normal light image is generated based on a normal light image signal and is displayed. The normal light is illumination light suitable for screening observation.

In the observation mode, the object to be observed is irradiated with substantially white light and oxygen saturation calculation illumination light (calculation illumination light), a substantially white light image signal and an oxygen saturation calculation image signal (a calculation image signal) are obtained, the oxygen saturation of the object to be observed is calculated based on the calculation image signal, the calculated oxygen saturation is superimposed on a base image generated from the substantially white light image signal, and an oxygen saturation image is displayed.

The substantially white light and the calculation illumination light that are used in the observation mode are illumination light for the observation of the oxygen saturation image obtained by turning on multiple light sources and combining light in multiple colors as described later. For this reason, in the case where the substantially white light and the calculation illumination light are not particularly distinguished in description, these are collectively referred to below as observation illumination light. In the case where the substantially white light image signal and/or the calculation image signal is described, this is referred to as an observation image signal.

In the correction mode, the object to be observed is irradiated with correction oxygen saturation calculation illumination light (correction illumination light) in addition to the observation illumination light, and when the oxygen saturation is calculated, a calculation for reflecting the influence of a specific pigment included in the object to be observed is made based on a correction oxygen saturation calculation image signal (a correction image signal), and correction oxygen saturation is calculated. In the present specification "tissue oxygen saturation" represents the oxygen saturation calculated in the observation mode or the correction oxygen saturation calculated in the correction mode or both.

In the normal mode, the display 15 displays the normal light image that has natural hue. In the observation mode, the display 15 displays the substantially white light image and the oxygen saturation image obtained by imaging the calculated oxygen saturation in, for example, a pseudo color. In the correction mode, the display 15 displays the substantially white light image and the correction oxygen saturation image obtained by imaging and the correction oxygen saturation.

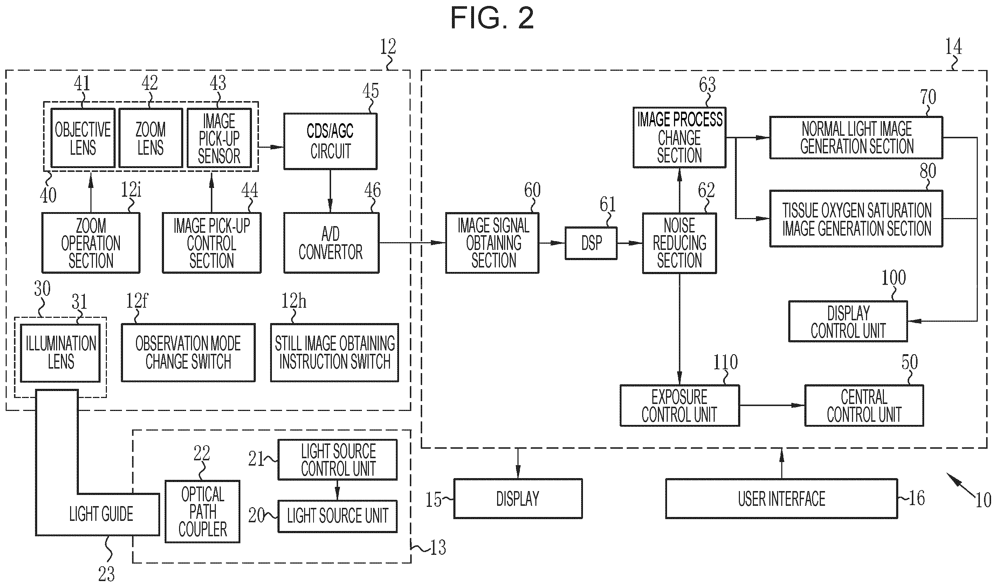

In Fig. 2, the light source device 13 includes a light source unit 20, a light source control unit 21, and an optical path coupler 22. For example, the light source unit 20 includes a semiconductor light source such as a light emitting diode (LED) for multiple colors, a laser light source, a combination of a laser diode and a fluorescent body, a xenon lamp, or a halogen light source. The light source unit 20 includes multiple light sources and emits illumination light with which the object to be observed is illuminated in a manner in which the multiple light sources are turned on or turned off, and the amounts of light that the light sources emit are controlled in the case where the light sources are turned on.

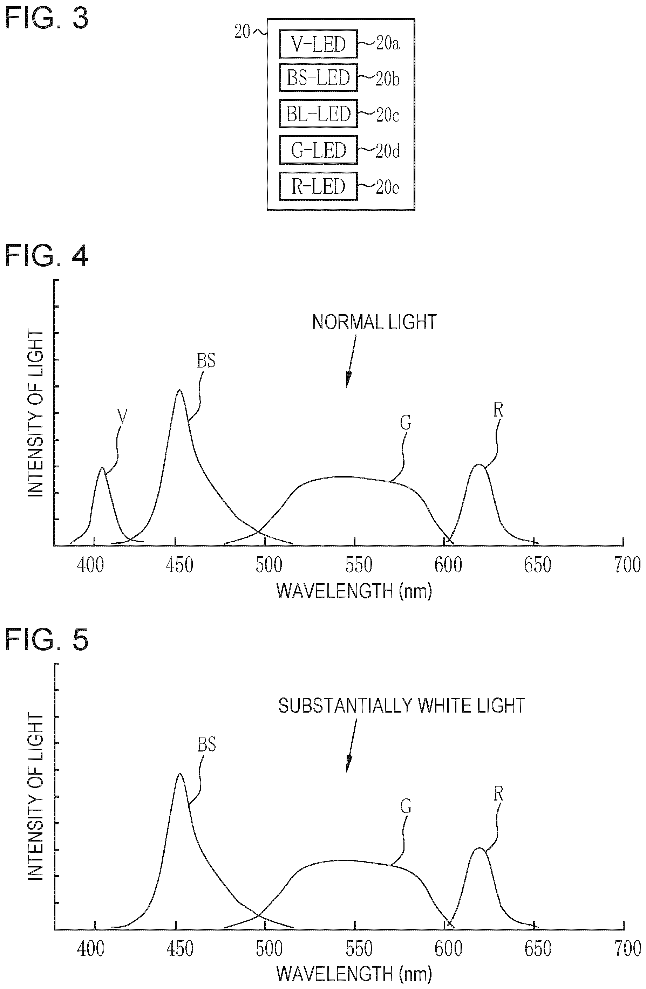

According to the first embodiment, as illustrated in Fig. 3, the light source unit 20 includes LEDs (semiconductor light sources) for five colors of a violet light emitting diode (V-LED) 20a, a blue short-wavelength light emitting diode BS-LED 20b, a blue long-wavelength light emitting diode (BL-LED) 20c, a green light emitting diode (G-LED) 20d, and a red light emitting diode (R-LED) 20e. A combination of the LEDs for the colors is not limited thereto.

The violet light emitting diode (V-LED) 20a emits purple light V the central wavelength of which is $410 \pm 10$ nm. The BS-LED 20b emits first blue light BS the central wavelength of which is $450$ nm $\pm 10$ nm. The BL-LED 20c emits second blue light BL the central wavelength of which is $470$ nm $\pm 10$ nm. The G-LED 20d preferably emits green light G the central wavelength of which is about $540$ nm in a green range. The R-LED 20e preferably emits red light R the central wavelength of which is about $630$ nm in a red range. As for the LEDs 20b to 20e, the central wavelengths and peak wavelengths may be equal to each other or may differ from each other.

The light source control unit 21 individually inputs a control signal into the LEDs 20a to 20e. At least one kind or more of the illumination light are emitted under light source control in which turning on or turning off the LEDs 20a to 20e and the amounts of light that the LEDs 20a to 20e emit when the LEDs 20a to 20e are turned on are individually controlled, and consequently, the normal light, the substantially white light, the calculation illumination light, or the correction illumination light is finally emitted in the set mode. Exposure control under control of the light source control unit 21 will be described later.

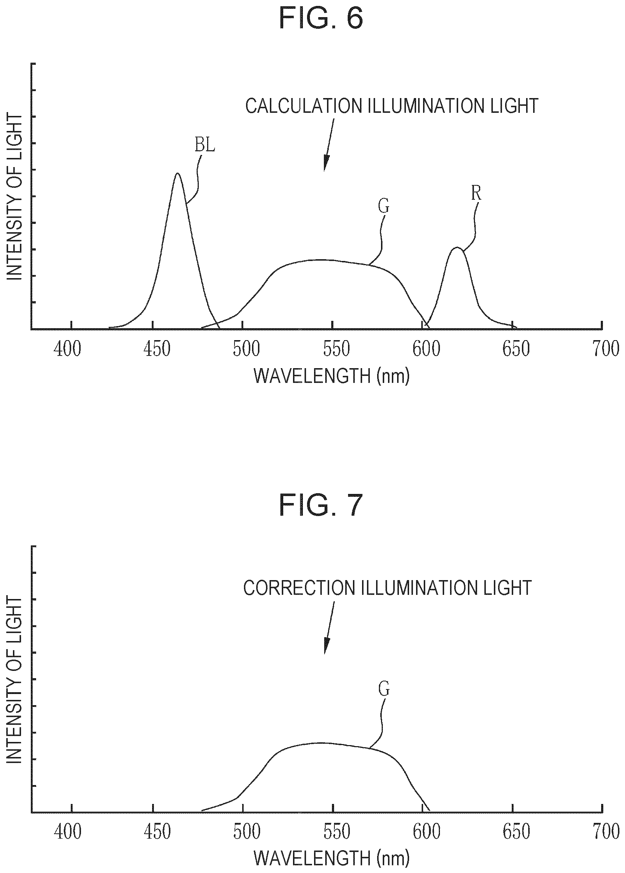

In the case where the normal light that has a spectrum illustrated in Fig. 4 is emitted, the V-LED 20a, the BS-LED 20b, the G-LED 20d, and the R-LED 20e are simultaneously turned on, and consequently, the purple light V, the first blue light BS, the green light G, and the red light R are combined. In the case where the substantially white light that has a spectrum illustrated in Fig. 5 is emitted, the BS-LED 20b, the G-LED 20d, and the R-LED 20e are simultaneously turned on, and consequently, the first blue light BS, the green light G, and the red light R are combined. In the case where the calculation illumination light that has a spectrum illustrated in Fig. 6 is emitted, the BL-LED 20c, the G-LED 20d, and the R-LED 20e are simultaneously turned on, and consequently, the second blue light BL, the green light G, and the red light R are combined. In the case where the correction illumination light that has a spectrum illustrated in Fig. 7 is emitted, the G-LED 20d is turned on, and the green light G is emitted. As illustrated in Fig. 4 to Fig. 7, the normal light, the observation illumination light (the substantially white light and the calculation illumination light), and the correction illumination light have the different spectrums.

The light source control unit 21 preferably changes the amounts of light of the V-LED 20a, the BS-LED 20b, the BL-LED 20c, the G-LED 20d, and the R-LED 20e for every frame F in accordance with a specific pattern that is set in each mode, and the normal light, the substantially white light, the calculation illumination light, or the correction illumination light is preferably emitted in each frame. A frame is the unit of a period that includes at least a period from a timing with which the illumination light is emitted to a timing with which an image pick-up sensor 43 finishes reading out the image signals. The light source control unit 21 automatically changes the illumination light emitted from the light source unit 20 in an illumination period for every frame.

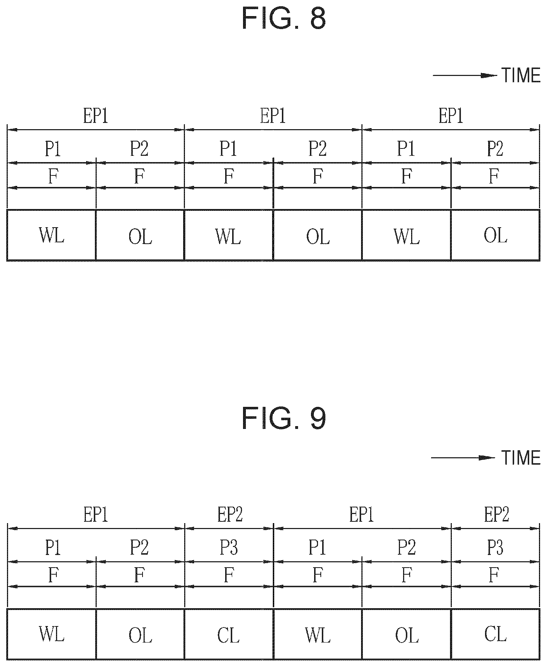

In the normal mode, a light emission pattern is repeated such that the normal light is emitted in a normal light illumination period in each frame F. In the observation mode, as illustrated in, for example, Fig. 8, a light emission pattern (a first light emission pattern) is repeated such that substantially white light WL for a single frame is emitted in a substantially white light illumination period (a first illumination period) P1 in which the object to be observed is illuminated with the substantially white light WL, and calculation illumination light OL for a single frame is subsequently emitted in a calculation illumination period (a second illumination period) P2 in which the object to be observed is illuminated with the calculation illumination light OL. A first exposure period EP1 described later includes a first illumination period P1 and a second illumination period P2. In the case where the first illumination period P1 and the second illumination period P2 are continuous, the first exposure period EP1 preferably includes the first illumination period P1 and the second illumination period P2 that are continuous.

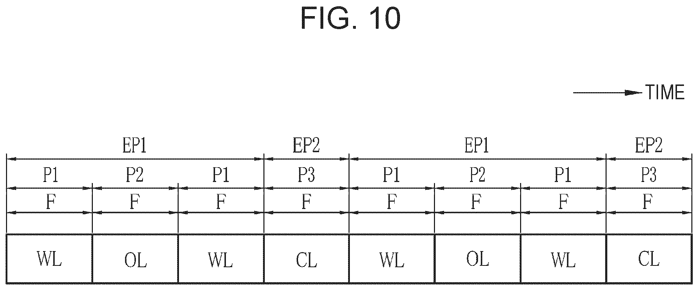

In the correction mode, as illustrated in, for example, Fig. 9, a light emission pattern (a second light emission pattern) is repeated such that the substantially white light WL for a single frame is emitted in the substantially white light illumination period P1, the calculation illumination light OL for a single frame is emitted in the calculation illumination period P2, and the correction illumination light CL for a single frame is emitted in a correction illumination period (a third illumination period) P3 in which the object to be observed is illuminated with the correction illumination light CL. A second exposure period EP2 described later includes the third illumination period P3. The light source control unit 21 automatically switches between the illumination periods. The light emission patterns are not limited thereto but can be freely set. As illustrated in, for example, Fig. 10, the substantially white light WL, the calculation illumination light OL, the substantially white light WL, and the correction illumination light CL may be sequentially emitted for every frame in a light emission pattern (a third light emission pattern). In such a case, the first exposure period is a period in which the first illumination period or the second illumination period is continuous such as the first illumination period, the second illumination period, and the first illumination period.

An illumination optical system 30 and an image pick-up optical system 40 are provided at the tip portion 12d of the endoscope 12. The illumination optical system 30 is an optical system for irradiating the object to be observed with the illumination light and includes an illumination lens 31. A light guide 23 causes the illumination light such as the normal light, the substantially white light, the calculation illumination light, and the correction illumination light to propagate, and the object to be observed is irradiated with the illumination light via the illumination lens 31.

In the case where the light source unit 20 is contained in the tip portion 12d of the endoscope 12, the illumination light such as the normal light, the substantially white light, the calculation illumination light, and the correction illumination light does not pass through the light guide 23 but passes through the illumination lens 31 of the illumination optical system 30 and is emitted. The image pick-up optical system 40 is an optical system that performs image pick-up of the reflection light from the object to be observed that is illuminated with the illumination light, and includes an objective lens 41, the zoom lens 42, and the image pick-up sensor 43. The image pick-up optical system 40 includes a shutter 48 and an aperture diaphragm 47 (not illustrated) described later. The aperture diaphragm 47 and the shutter 48 may not be components but may be an electronic aperture diaphragm and an electronic shutter that are electronically controlled. The reflection light from the object to be observed that is irradiated with the illumination light enters the image pick-up sensor 43 via the objective lens 41 and the zoom lens 42, and consequently, the image of the object to be observed is imaged on the image pick-up sensor 43. The zoom lens 42 is a lens for magnifying the object to be observed and moves between a telephoto end and a wide end as a result of an operation on the zoom operation section 12i.

The image pick-up sensor 43 is a color image pick-up sensor that performs image pick-up of the object to be observed being illuminated with the illumination light. Each of pixels of the image pick-up sensor 43 is a B pixel (a blue pixel) that includes a B (blue) color filter, a G pixel (a green pixel) that includes a G (green) color filter, or a R pixel (a red pixel) that includes a R (red) color filter. For example, the image pick-up sensor 43 is preferably a color image pick-up sensor that has a Bayer array in which the proportion of the number of the B pixels, the G pixels, and the R pixels is 1:2:1.

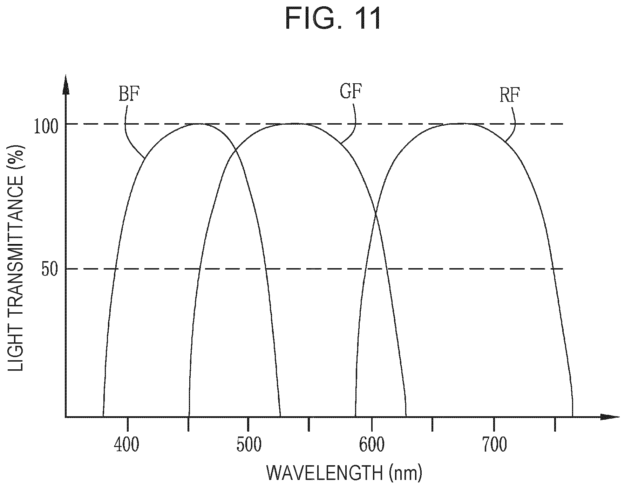

As illustrated in Fig. 11, a B color filter BF mainly allows light in a blue range, specifically, light the wavelength range of which is 380 to 560 nm (in a blue transmittance range) to pass therethrough. A peak wavelength at which the light transmittance is the maximum is close to 460 to 470 nm. A G color filter GF mainly allows light in a green range, specifically, light the wavelength range of which is 450 to 630 nm (a green transmittance range) to pass therethrough. A R color filter RF mainly allows light in a red range, specifically, light at 580 to 760 nm (a red transmittance range) to pass therethrough.

The image pick-up sensor 43 can be a charge coupled device (CCD) image pick-up sensor or a complementary metal-oxide semiconductor (CMOS) image pick-up sensor. A complementary color image pick-up sensor that includes a complementary color filter for C (cyan dye), M (magenta), Y (yellow), and G (green) may be used instead of the image pick-up sensor 43 for the primary colors. In the case where the complementary color image pick-up sensor is used, an image signal for four colors of CMYG is outputted. Accordingly, the image signal for the four colors of CMYG is converted into an image signal for three colors of RGB by complementary-color-to-primary-color conversion, and consequently, the image signal for the colors of RGB can be obtained as in the image pick-up sensor 43. The image pick-up sensor 43 is driven and controlled by an image pick-up control section 44.

The image pick-up sensor 43 senses the reflection light from the object to be observed with which the normal light is irradiated and outputs the normal light image signals (a Bc image signal, a Gc image signal, and a Rc image signal). In addition, the reflection light from the object to be observed with which the substantially white light is irradiated is sensed, and the substantially white light image signals (a B1 image signal, a G1 image signal, and a R1 image signal) are outputted. In addition, the reflection light from the object to be observed with which the calculation illumination light is irradiated is sensed, and calculation image signals (a B2 image signal, a G2 image signal, and a R2 image signal) are outputted. In addition, the reflection light from the object to be observed with which the correction illumination light is irradiated is sensed, and correction image signals (a B3 image signal, a G3 image signal, and a R3 image signal) are outputted. The image pick-up control section 44 implements control such that the image pick-up sensor 43 outputs the normal light image signals in the case of the normal mode, outputs the substantially white light image signals and the calculation image signals in the case of the observation mode, and outputs the substantially white light image signals, the calculation image signals, and the correction image signals in the case of the correction mode.

That is, as illustrated in Table 1, in the case where the normal light image signals are obtained in the normal mode, the light source control unit 21 implements control such that the V-LED 20a, the BS-LED 20b, the G-LED 20d, and the R-LED 20e are turned on, and the normal light is emitted in the normal light illumination period, the image pick-up control section 44 performs image pick-up for every frame, and the image pick-up sensor 43 is controlled such that the Bc image signals are outputted from the B pixels of the image pick-up sensor 43, the Gc image signals are outputted from the G pixels, and the Rc image signals are outputted from the R pixels.

Table 1

| Illumination period | Illumination light | Light source to be turned on (illumination light) | Image signal |
|---|---|---|---|
| Normal light illumination period | Normal light | V-LED (purple light)<br>BS-LED (first blue light)<br>R-LED (red light)<br>G-LED (green light) | Normal light image signal (Bc, Gc, Rc) |

As illustrated in Table 2, the substantially white light image signals and the calculation image signals are obtained in the observation mode. In the case where the substantially white light image signals are obtained, the light source control unit 21 implements control such that the BS-LED 20b, the G-LED 20d, and the R-LED 20e are turned on, the substantially white light WL is emitted in the substantially white light illumination period P1, the image pick-up control section 44 controls the image pick-up sensor 43 such that image pick-up is performed for every frame, and the B1 image signals are outputted from the B pixels of the image pick-up sensor 43, the G1 image signals are outputted from the G pixels, and the R1 image signals are outputted from the R pixels. In the case where the calculation image signals are obtained, the light source control unit 21 implements control such that the BL-LED 20c, the G-LED 20d, and the R-LED 20e are turned on, and the calculation illumination light OL is emitted in the calculation illumination period P2. The image pick-up control section 44 controls the image pick-up sensor 43 such that image pick-up is performed for every frame, and the B2 image signals are outputted from the B pixels of the image pick-up sensor 43, the G2 image signals are outputted from the G pixels, and the R2 image signals are outputted from the R pixels.

Table 2

| Illumination period | Illumination light | Light source to be turned on (illumination light) | Image signal |
|---|---|---|---|
| Substantially white light illumination period | Substantially white light | BS-LED (first blue light)<br>R-LED (red light)<br>G-LED (green light) | Substantially white light image signal (B1, G1, R1) |

(continued)

| Illumination period | Illumination light | Light source to be turned on (illumination light) | Image signal |
|---|---|---|---|
| Calculation illumination period | Calculation illumination light | BL-LED (second blue light) R-LED (red light) G-LED (green light) | Calculation image signal (B2, G2, R2) |

As illustrated in Table 3, the substantially white light image signals, the calculation image signals, and the correction image signals are obtained in the correction mode. In the case where the substantially white light image signals or the calculation image signals are obtained in the correction mode, the same control as in the observation mode is implemented. In the case where the correction image signals are obtained, the light source control unit 21 implements control such that the G-LED 20d is turned on, the correction illumination light is emitted, and the image pick-up control section 44 controls the image pick-up sensor 43 such that image pick-up is performed for every frame, the B3 image signals are outputted from the B pixels of the image pick-up sensor 43, the G3 image signals are outputted from the G pixels, and the R3 image signals are outputted from the R pixels. Exposure control under control of the image pick-up control section 44 will be described later. The correction illumination light is not limited to light in a single color as illustrated in Table 3 but may be illumination light in multiple colors.

Table 3

| Illumination period | Illumination light | Light source to be turned on (illumination light) | Image signal |
|---|---|---|---|
| Substantially white light illumination period | Substantially white light | BS-LED (first blue light) R-LED (red light) G-LED (green light) | Substantially white light image signal (B1, G1, R1) |
| Calculation illumination period | Calculation illumination light | BL-LED (second blue light) R-LED (red light) G-LED (green light) | Calculation image signal (B2, G2, R2) |
| Correction illumination period | Correction illumination light | G-LED (green light) | Correction image signal (B3, G3, R3) |

A correlated double sampling/automatic gain control (CDS/AGC) circuit 45 implements correlated double sampling (CDS) or automatic gain control (AGC) on an analog image signal obtained from the image pick-up sensor 43. The image signal via the CDS/AGC circuit 45 is converted into a digital image signal by using an analog/digital (A/D) convertor 46. Consequently, the digital image signal after A/D conversion is inputted into the processor device 14.

The processor device 14 includes the central control unit 50, an image signal obtaining section 60, a digital signal processor (DSP) 61, a noise reducing section 62, an image process change section 63, a normal light image generation section 70, a tissue oxygen saturation image generation section 80, a display control unit 100, and an exposure control unit 110 (see Fig. 2). As for the processor device 14, programs related to processes are installed in a program memory (not illustrated). The central control unit 50 that includes a processor runs a program in the program memory, and this fulfills the functions of the image signal obtaining section 60, the DSP 61, the noise reducing section 62, the image process change section 63, the normal light image generation section 70, the tissue oxygen saturation image generation section 80, the display control unit 100, and the exposure control unit 110. This leads to the fulfillment of the functions of a substantially white light image generation section 81, a base image generation section 82, a signal ratio calculation section 83, an oxygen saturation calculation section 84, an oxygen saturation image generation section 85, a correction oxygen saturation calculation section 86, and a correction oxygen saturation image generation section 87 that are included in the tissue oxygen saturation image generation section 80 and that will be described later and a exposure control signal generation section 120, a control amount output section 130, a brightness calculation section 140, an analysis region setting section 141, a region brightness calculation section 142, and a brightness difference calculation section 150 that are included in the exposure control unit 110.

The image signal obtaining section 60 receives the image signals (the normal light image signals, the substantially white light image signals, the calculation image signals, and the correction image signals) that are inputted from the endoscope 12 and transmits the received image signals to the DSP 61. The DSP 61 performs various signal processes such as a defect correction process, an offset process, a gain correction process, a linear matrix process, a gamma conversion process, a demosaicing process, and a YC conversion process on the received image signals. In the defect correction process, the signal of a defect pixel of the image pick-up sensor 43 is corrected. In the offset process, a dark current component is removed from the image signals on which the defect correction process is performed, and an accurate zero level is set. In the gain correction process, the image signals for the colors after the offset process is multiplied by a specific gain, and consequently, the signal level of each image signal is adjusted. The linear matrix process to increase color reproducibility is performed on the image signals for the colors after the gain correction process.

Subsequently, in the gamma conversion process, the brightness and saturation of each image signal are adjusted. The demosaicing process (also referred to as an isotropic process, or demosaicing) is performed on the image signals after the linear matrix process, and a signal for missing color in each pixel is generated by an interpolation. Through the demosaicing process, all of the pixels have the signals for the colors of RGB. The DSP 61 performs the YC conversion process on the image signals after the demosaicing process and outputs a brightness signal Y, a color difference signal Cb, and a color difference signal Cr to the noise reducing section 62.

The noise reducing section 62 performs the noise reducing process on the image signals on which the DSP 61 performs, for example, the demosaicing process by using, for example, a moving average method or a median filter method. The image signals in which a noise is reduced are inputted into the image process change section 63 and the exposure control unit 110 described later.

The image process change section 63 transmits the image signals from the noise reducing section 62 to the normal light image generation section 70 in the case of the normal mode or to the tissue oxygen saturation image generation section 80 in the case of the observation mode or the correction mode. The normal light image generation section 70 performs a color conversion process such as a $3 \times 3$ matrix process, a gradation transformation process, or a three-dimensional look-up-table (LUT) process on the Bc image signal, the Gc image signal, or the Rc image signal that is inputted for a single frame. Subsequently, various color emphasis processes are performed on RGB image data after the color conversion process. A structure emphasis process such as spatial frequency emphasis is performed on the RGB image data after the color emphasis process. The RGB image data on which the structure emphasis process is performed is transmitted as the normal light image to the display control unit 100.

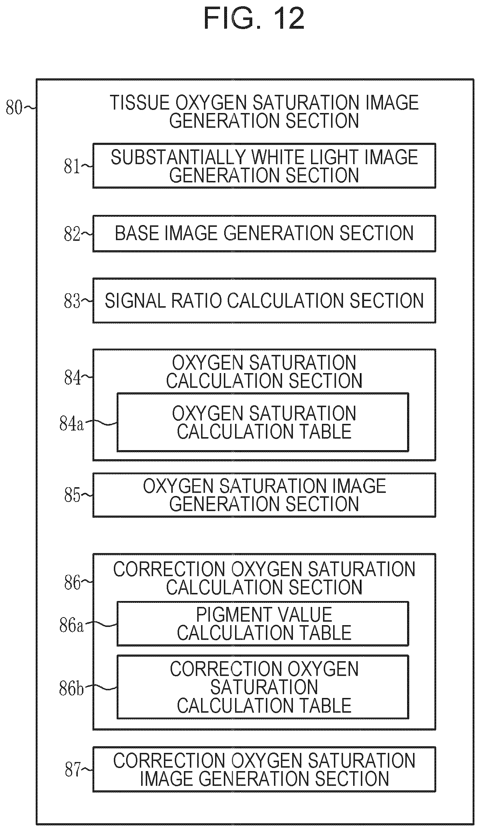

As illustrated in Fig. 12, the tissue oxygen saturation image generation section 80 includes the substantially white light image generation section 81, the base image generation section 82, the signal ratio calculation section 83, the oxygen saturation calculation section 84, the oxygen saturation image generation section 85, the correction oxygen saturation calculation section 86, and the correction oxygen saturation image generation section 87.

The substantially white light image generation section 81 performs the color conversion process such as the $3 \times 3$ matrix process, the gradation transformation process, or the three-dimensional LUT process on the B1 image signal, the G1 image signal, or the R1 image signal that is inputted for a single frame. Subsequently, various color emphasis processes are performed on the RGB image data after the color conversion process. The structure emphasis process such as the spatial frequency emphasis is performed on the RGB image data after the color emphasis process. The RGB image data on which the structure emphasis process is performed is transmitted as the substantially white light image to the display control unit 100. The substantially white light image corresponds to the normal light image obtained in the case of the normal mode but differs from the normal light image in that the hue is different because the spectrum of the illumination light with which the object to be observed is irradiated is different.

The base image generation section 82 performs various signal processes on the image signal inputted for a single frame, such as the B1 image signal, the G1 image signal, or the R1 image signal and generates a base image for generating the oxygen saturation image or the correction oxygen saturation image. The image signal inputted into the base image generation section 82 is not limited to the B1 image signal, the G1 image signal, or the R1 image signal but may be an image signal for generating any image that the user is to use as the base image. The base image is transmitted to the oxygen saturation image generation section 85 in the case of the observation mode or to the correction oxygen saturation image generation section 87 in the case of the correction mode.

The oxygen saturation calculation section 84 calculates the oxygen saturation, based on the substantially white light image signals and the calculation image signals in the observation mode. A method of calculating the oxygen saturation will be described later. Information about the calculated oxygen saturation is transmitted to the oxygen saturation image generation section 85. The oxygen saturation image generation section 85 generates the oxygen saturation image in which the information about the oxygen saturation is represented as the pseudo color in the base image. For example, the base image is obtained by adjusting the saturation of the substantially white light image or the substantially white light image. The use of the substantially white light image in which the saturation is reduced as the base image brings an advantage to improve visibility in the case where a region in which the oxygen saturation is displayed is narrow. An endoscope image based on an image signal obtained by using another illumination light may be used as the base image. The normal light image may be the base image. The oxygen saturation image is transmitted to the display control unit 100.

The correction oxygen saturation calculation section 86 calculates the correction oxygen saturation, based on the substantially white light image signals, the calculation image signals, and the correction image signals in the correction mode. A method of calculating the correction oxygen saturation will be described later. Information about the calculated correction oxygen saturation is transmitted to the correction oxygen saturation image generation section 87. The correction oxygen saturation image generation section 87 generates the oxygen saturation image in which the information about the correction oxygen saturation is superimposed as the pseudo color on the base image. The correction oxygen saturation image is transmitted to the display control unit 100.

The display control unit 100 converts the normal light image from the normal light image generation section 70 and the substantially white light image, the oxygen saturation image, and the correction oxygen saturation image from the tissue oxygen saturation image generation section 80 into video signals that enable the display 15 to make display in full color. The video signals after conversion are inputted into the display 15. Consequently, the display 15 displays the normal light image, the substantially white light image, the oxygen saturation image and/or the correction oxygen saturation image. Multiple displays 15 may be connected to the processor device 14, and the display control unit 100 may cause the normal light image, the substantially white light image, the oxygen saturation image, and the correction oxygen saturation image to be displayed on the different displays.

The method of calculating the oxygen saturation will be described. The signal ratio calculation section 83 of the tissue oxygen saturation image generation section 80 calculates calculation values for oxygen saturation calculation in a signal ratio process based on the B2 image signal, the G1 image signal, and the R1 image signal. Specifically, the signal ratio calculation section 83 calculates, as the calculation values for oxygen saturation calculation that are used to calculate the oxygen saturation, a signal ratio B2/G1 between the B2 image signal and the G1 image signal and a signal ratio R1/G1 between the R1 image signal and the G1 image signal. The signal ratio B2/G1 and the signal ratio R1/G1 are preferably logarithms (ln). Hue H, saturation S, or a color difference signal Cr or Cb calculated from the B2 image signal, the G1 image signal, and the R1 image signal may be used as the calculation value for oxygen saturation calculation.

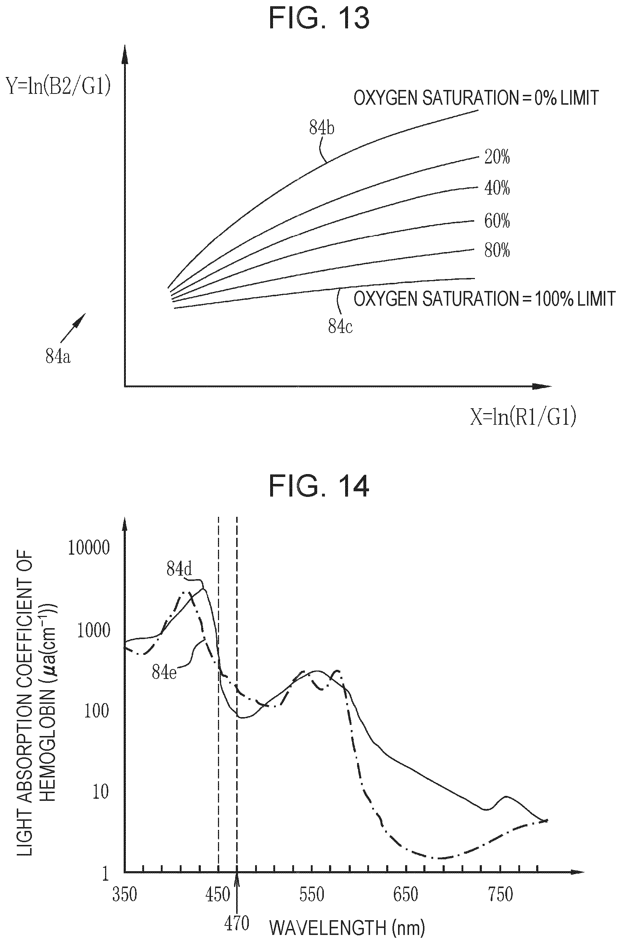

The oxygen saturation calculation section 84 refers an oxygen saturation calculation table 84a and calculates the oxygen saturation by using the calculation values for oxygen saturation calculation such as the signal ratio B2/G1 and the signal ratio R1/G1. The oxygen saturation calculation table 84a stores correlations among the oxygen saturation and the logarithms of the signal ratios B2/G1 and R1/G1 obtained by using a simulation or an endoscope image obtained by performing image pick-up of multiple phantoms that imitate an organism and that correspond to the oxygen saturation having multiple stages. In the case where the correlations are expressed in a two-dimensional space defined by using X (X = ln(R1/G1)) that is the value of the logarithm of the signal ratio R1/G1 in the direction of an X-axis and Y (Y = ln(B2/G1)) that is the value of the logarithm of the signal ratio B2/G1 in the direction of a Y-axis, as illustrated in Fig. 13, a contour that connects portions at which the oxygen saturation is the same extends substantially in the direction of the X-axis (the lateral axis). An isovalue line is lowered in the direction of the Y-axis (the vertical axis) as the oxygen saturation increases. For example, an isovalue line 84b when the oxygen saturation is 100% is lower than an isovalue line 84c when the oxygen saturation is 0%.

The correlations among the signal ratios B2/G1 and R1/G1 and the oxygen saturation have a close relationship with light absorption characteristics and light scattering characteristics of oxyhemoglobin (a graph 84d) and deoxyhemoglobin (a graph 84e) illustrated in Fig. 14. As illustrated in Fig. 14, the amount of absorbed light changes due to the oxygen saturation of hemoglobin in a wavelength range in which a difference in light absorption coefficient between oxyhemoglobin and deoxyhemoglobin is large such as a wavelength range of $470 \pm 10$ nm of the second blue light BL, and accordingly, the information about the oxygen saturation is easy to be handled. The light absorption coefficient of hemoglobin is relatively high in a wavelength range of $540 \pm 20$ nm of the green light G, and accordingly, the amount of absorbed light is likely to change due to blood concentration in the wavelength range.

As for the B2 image signal obtained in a manner in which the object to be observed is irradiated with the calculation illumination light that includes the second blue light BL, the signal value changes particularly greatly depending on the oxygen saturation. As for the G1 image signal obtained in a manner in which the object to be observed is irradiated with the substantially white light, the signal value changes particularly greatly depending on the blood concentration. As for the R1 image signal obtained in a manner in which the object to be observed is irradiated with the substantially white light, the signal value changes moderately depending on the oxygen saturation. As for the B2 image signal, the G1 image signal, and the R1 image signal, the signal values change depending on the oxygen saturation, the blood concentration, and the brightness. The "blood concentration" represents the concentration of hemoglobin (a hemoglobin content) in the blood.

The use of the signal ratio B2/G1 that mainly depends on the oxygen saturation and the signal ratio R1/G1 that mainly depends on the blood concentration in order to remove the influence of the brightness from the B2 image signal, the G1 image signal, and the R1 image signal enables the oxygen saturation to be calculated in consideration of the influence of the blood concentration. The signal ratio B2/G1 has a value that moderately depends on the blood concentration. The signal ratio R1/G1 has a value that moderately depends on the oxygen saturation.

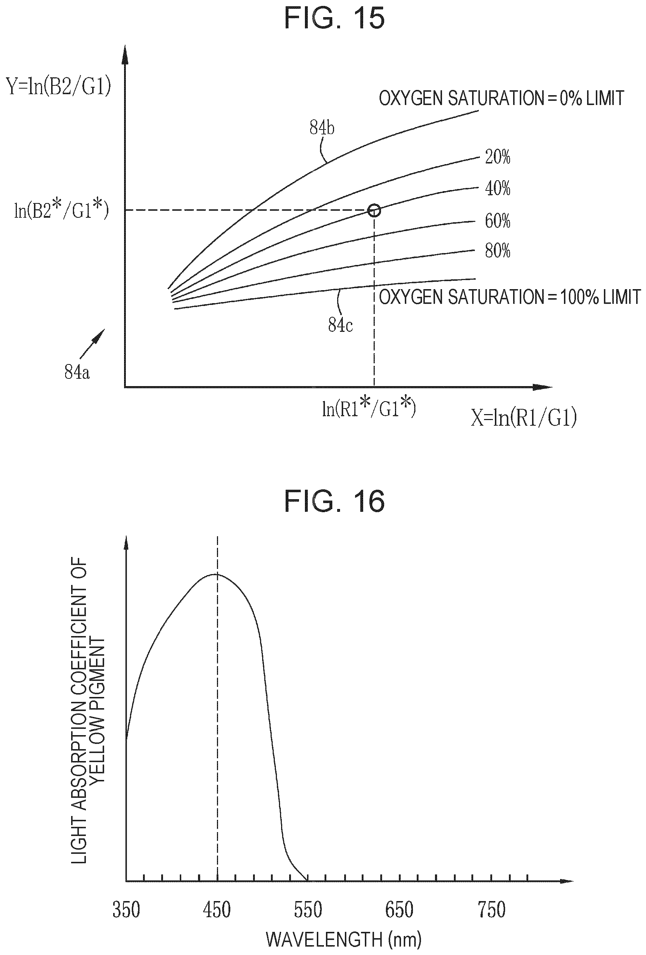

The oxygen saturation calculation section 84 refers the oxygen saturation calculation table 84a and calculates the oxygen saturation corresponding to the signal ratios B2/G1 and R1/G1 for every pixel. For example, as illustrated in Fig. 15, the oxygen saturation corresponding to the signal ratios B2*/G1* and R1*/G1* of a specific pixel is "40%". Accordingly, the oxygen saturation of the specific pixel that the oxygen saturation calculation section 84 calculates is "40%".

The signal ratios B2/G1 and R1/G1 scarcely become too high or too low. That is, a combination of the values of the signal ratios B2/G1 and R1/G1 is scarcely plotted below the isovalue line 84c (see Fig. 13) of the upper limit of 100% oxygen saturation or plotted above the isovalue line 84b (see Fig. 13) of the lower limit of 0% oxygen saturation. In the case where the combination is plotted above the isovalue line 84c of the upper limit, the oxygen saturation is 100%, and in the case where the combination is plotted below the isovalue line 84b of the lower limit, the oxygen saturation calculation section 84 sets the oxygen saturation to 0%. In the case where points corresponding to the signal ratios B2/G1 and R1/G1 are not plotted between the isovalue line 84c of the upper limit and the isovalue line 84b of the lower limit, it may be displayed that the reliability of the oxygen saturation of the pixel is low, and the oxygen saturation may not be calculated.

The oxygen saturation image generation section 85 generates the oxygen saturation image obtained by imaging the oxygen saturation by using the oxygen saturation calculated by the oxygen saturation calculation section 84. Specifically, the oxygen saturation image generation section 85 obtains the B2 image signal, the G1 image signal, and the R1 image signal and applies oxygen saturation image generation gains depending on the oxygen saturation to the image signals for every pixel. The RGB image data is generated by using the B2 image signal, the G1 image signal, and the R1 image signal to which the oxygen saturation image generation gains are applied and is superimposed on the base image into the oxygen saturation image.

For example, the oxygen saturation image generation gains are multiplied such that gains for a B channel and a G channel are relatively increased, and a gain for a R channel is relatively reduced. Specifically, the oxygen saturation image generation section 85 multiplies the B2 image signal, the G1 image signal, and the R1 image signal equally by an oxygen saturation image generation gain of "1" for a pixel the oxygen saturation of which is 60% or more. The B2 image signal is multiplied by an oxygen saturation image generation gain of "1" or more, and the G1 image signal and the R1 image signal are multiplied by an oxygen saturation image generation gain of less than "1" for a pixel the oxygen saturation of which is less than 60%. The oxygen saturation image corresponds to the RGB image data that is generated by using the B2 image signal, the G1 image signal, and the R1 image signal after the process with the oxygen saturation image generation gains is performed and that is superimposed as the pseudo color on the base image. The image signals to which the oxygen saturation image generation gains are applied to generate the oxygen saturation image are not limited to the image signals described above by way of example.

As for the oxygen saturation image generated by the oxygen saturation image generation section 85, for example, a high-oxygen region (a region in which the oxygen saturation is 60 to 100%) is preferably represented in the same color as the base image, and a low-oxygen region (a region in which the oxygen saturation is 0 to 60%) in which the oxygen saturation is less than a specific value is preferably represented in color (the pseudo color) that differs from that of the base image. In this case, the oxygen saturation image generation section 85 represents the low-oxygen region by multiplying the low-oxygen region by the oxygen saturation image generation gains for representation in the pseudo color.

The high-oxygen region may be represented by multiplying the high-oxygen region by the oxygen saturation image generation gains depending on the oxygen saturation. A color map in which the oxygen saturation is represented by using the gradation of the pseudo color may be generated as the oxygen saturation image. The low-oxygen region and the high-oxygen region are divided when the oxygen saturation is 60%, but the boundary thereof is freely determined. For example, a stepwise specific variable may be provided, and a color map divided into the low-oxygen region, a medium-oxygen region, and the high-oxygen region may be generated. The oxygen saturation image may be generated such that the oxygen saturation is represented by using information about characters such as "A%".

The method of calculating the correction oxygen saturation will now be described. The signal ratio calculation section 83 of the tissue oxygen saturation image generation section 80 calculates a calculation value for correction oxygen saturation calculation in the signal ratio process based on the B2 image signal, the G1 image signal, the R1 image signal, the B3 image signal, and the G3 image signal. Specifically, the signal ratio calculation section 83 calculates, as the calculation value for oxygen saturation calculation that is used to calculate the oxygen saturation, the signal ratio B2/G1 between the B2 image signal and the G1 image signal, the signal ratio R1/G1 between the R1 image signal and the G1 image signal, and a signal ratio B3/G3 between the B3 image signal and the G3 image signal. The signal ratios B2/G1, R1/G1, and B3/G3 are preferably logarithms (ln). The hue H, the saturation S, or the color difference signal Cr or Cb calculated from the B2 image signal, the G1 image signal, the R1 image signal, the B3 image signal, and the G3 image signal may be used as the calculation value for oxygen saturation calculation. A signal ratio B1/G3 between the B1 image signal and the G3 image signal may be used instead of the signal ratio B3/G3. In this case, a pigment value calculation table 86a that uses the signal ratio B1/G3 described later is referred.

In some cases, the object to be observed includes the specific pigment that differs from oxyhemoglobin and deoxyhemoglobin and that affects the calculation of the oxygen saturation. Examples of the specific pigment include yellow pigment. As illustrated in Fig. 16, the light absorption coefficient of the yellow pigment has a peak highest near a wavelength of $450 \pm 10$ nm. Accordingly, the signal value of the B2 image signal obtained based on light after the observation illumination light including the second blue light BL the central wavelength of which is $470$ nm $\pm 10$ nm passes through the B color filter BF highly depends on the amount of the yellow pigment, greatly changes depending on the amount of the yellow pigment, and accordingly affects the calculation of the oxygen saturation.

Specifically, as the amount of the yellow pigment increases, the signal value of the B2 image signal reduces.

Accordingly, the value of the signal ratio B2/G1 reduces, and the oxygen saturation seemingly increases. For this reason, in the case where the object to be observed includes the yellow pigment, image signals (such as the B3 image signal and the G3 image signal) that less depend on the amount of the yellow pigment are obtained, and this enables the correction oxygen saturation that corresponds to the tissue oxygen saturation in consideration of the influence of the yellow pigment to be calculated. The degree of dependence of the G1 image signal on the yellow pigment is a low degree to a moderate degree. The degree of dependence of the R1 image signal on the yellow pigment is a low degree.

The signal value of the B3 image signal obtained in a manner in which the object to be observed is irradiated with the correction illumination light that includes the green light G depends on the brightness, less depends on the oxygen saturation, and highly depends on the blood concentration, and the degree of dependence on the amount of the yellow pigment is a moderate degree. The signal value of the G3 image signal obtained in a manner in which the object to be observed is irradiated with the correction illumination light that includes the green light G depends on the brightness, less depends on the oxygen saturation, and highly depends on the blood concentration, and the degree of dependence on the amount of the yellow pigment is a low degree to a moderate degree.

The correction oxygen saturation calculation section 86 uses the signal ratios B2/G1, R1/G1, and B3/G3 of the B2 image signal, the G1 image signal, the R1 image signal, the B3 image signal, and the G3 image signal from which the influence of the brightness is removed, refers the pigment value calculation table 86a and a correction oxygen saturation calculation table 86b, and obtains the correction oxygen saturation depending on the amount of the yellow pigment. The pigment value calculation table 86a stores correlations among the signal ratio B2/G1, the signal ratio R1/G1, the signal ratio B3/G3, and the pigment value depending on the amount of the specific pigment. The pigment value is obtained in advance by using a simulation or an endoscope image obtained by performing image pick-up of multiple phantoms that imitate an organism and that correspond to the oxygen saturation having multiple stages.

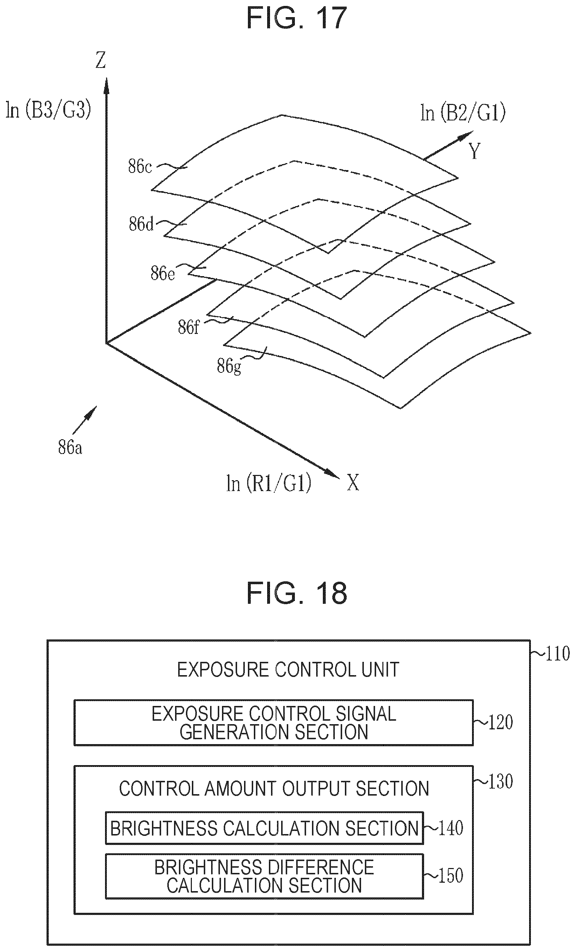

In the case where the correlations are represented in a three-dimensional space defined by using $X = \ln(R1/G1)$ that is a value in the direction of an X-axis, $Y = \ln(B2/G1)$ that is a value in the direction of a Y-axis, and $Z = \ln(B3/G3)$ that is a value in the direction of a Z-axis, as illustrated in Fig. 17, curved surfaces 86c to 86g for respective multiple stages of a pigment value are formed. The curved surfaces 86c to 86g are contours for the tissue oxygen saturation the pigment value of which is constant. In Fig. 17, the curved surface 86c the pigment value of which is "0", the curved surface 86d the pigment value of which is "1", the curved surface 86e the pigment value of which is "2", the curved surface 86f the pigment value of which is "3", and the curved surface 86g the pigment value of which is "4" are formed depending on the signal ratios $\ln(R1/G1)$, $\ln(B2/G1)$, and $\ln(B3/G3)$. That is, the curved surfaces (the correction oxygen saturation calculation table 86b) that are used to calculate the correction oxygen saturation are determined depending on the values of the signal ratios $\ln(R1/G1)$, $\ln(B2/G1)$, and $\ln(B3/G3)$. In the pigment value calculation table 86a illustrated in Fig. 17, as the amount of the yellow pigment increases, $Y = \ln(B2/G1)$ reduces, $X = \ln(R1/G1)$ increases, and $Z = \ln(B3/G3)$ reduces.

The correction oxygen saturation calculation section 86 refers the correction oxygen saturation calculation table 86b depending on the pigment value and calculates the correction oxygen saturation. The correction oxygen saturation calculation table 86b stores the correlations (the two-dimensional space defined by using $X = \ln(R1/G1)$ that is the value in the direction of the X-axis and $Y = \ln(B2/G1)$ that is the value of the direction of the Y-axis illustrated in Fig. 13) among the signal ratios B2/G1, the signal ratio R1/G1, and the correction oxygen saturation as in the oxygen saturation calculation table 84a.

The correction oxygen saturation calculation table 86b may not be stored in advance. In this case, the correction oxygen saturation calculation section 86 creates the correction oxygen saturation calculation table 86b that corresponds to correlations among the signal ratio B2/G1, the signal ratio R1/G1, and the correction oxygen saturation depending on the pigment value every time. The correction oxygen saturation calculation table 86b created may be used as the oxygen saturation calculation table 84a in the observation mode.

The correction oxygen saturation in consideration of the influence of the yellow pigment may be obtained by using the correction oxygen saturation calculation table 86b that stores correlations among the signal ratios R1/G1, B2/G1, and B3/G3, and the tissue oxygen saturation in advance instead of the pigment value calculation table 86a.

The correction oxygen saturation image generation section 87 generates the correction oxygen saturation image obtained by imaging the correction oxygen saturation by using the correction oxygen saturation calculated by the correction oxygen saturation calculation section 86. Specifically, the correction oxygen saturation image generation section 87 obtains the B2 image signal, the G1 image signal, and the R1 image signal and applies correction oxygen saturation image generation gains depending on the oxygen saturation on the image signals for every pixel. The RGB image data is generated by using the B2 image signal, the G1 image signal, and the R1 image signal to which the correction oxygen saturation image generation gains are applied and is superimposed as the pseudo color on the base image into the correction oxygen saturation image. The image signals to which the correction oxygen saturation image generation gains are applied to generate the correction oxygen saturation image are not limited to the image signals described above by way of example.

As for the correction oxygen saturation image, the low-oxygen region (the region in which the oxygen saturation is 0 to 60%) may be represented in the pseudo color as in the oxygen saturation image, and the high-oxygen region (the region in which the oxygen saturation is 60 to 100%) and the low-oxygen region (the region in which the oxygen saturation is 0 to 60%) may be represented in different colors (pseudo colors). A color map depending on the correction oxygen saturation may be generated as the correction oxygen saturation image, and the correction oxygen saturation image may be generated such that the correction oxygen saturation is represented by using information about characters such as "B%".

In the case where the degree of the image signal brightness of the image signals to which tissue oxygen saturation image generation gains (the oxygen saturation image generation gains or the correction oxygen saturation image generation gains) are applied is extremely low in a region, for example, in the case where the object to be observed is largely separated from the tip portion 12d of the endoscope 12, or in the case where the object to be observed is not sufficiently irradiated with the illumination light, for example, because the amount of light is insufficient, the oxygen saturation image generation section 85 or the correction oxygen saturation image generation section 87 may generate the tissue oxygen saturation image in which the region is filled in black. In the case where the degree of the image signal brightness of the image signals to which the tissue oxygen saturation image generation gains are applied is extremely high in a region, for example, in the case where the amount of specular reflection light included in the reflection light from the object to be observed is large, the tissue oxygen saturation image in which the region is filled with white may be generated.

The exposure control according to the present embodiment will now be described. Based on the image signals that are obtained in the first exposure period and the second exposure period, the exposure control unit 110 generates different exposure control signals and implements control (the exposure control) depending on the exposure control signals. As illustrated in Fig. 18, the exposure control unit 110 includes the exposure control signal generation section 120 and the control amount output section 130. The control amount output section 130 includes the brightness calculation section 140 and the brightness difference calculation section 150. An exposure period is a period from a timing with which various kinds of the illumination light are emitted to a timing with which the image pick-up sensor 43 senses the reflection light from the object to be observed and outputs the image signal. Under the exposure control, a unit operated in the exposure period such as the light source unit 20 or the image pick-up optical system 40 is controlled.

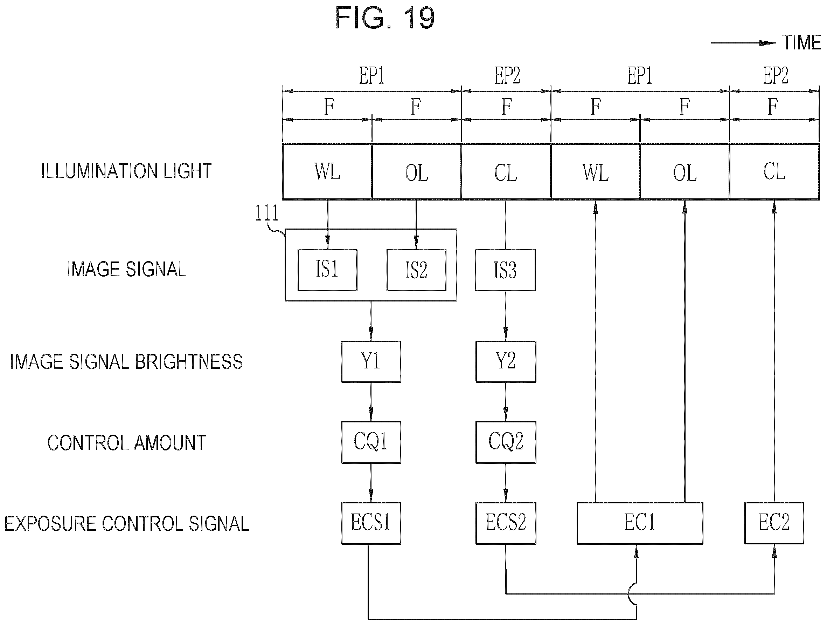

A specific example in which the different exposure control signals are generated in the exposure periods will be described with reference to Fig. 19. In Fig. 19, the exposure control is implemented in the case where various kinds of the illumination light are emitted in the second light emission pattern, but the light emission pattern is not limited thereto. The exposure control unit 110 receives an observation image signal 111 that is a substantially white light image signal IS1 or a calculation image signal IS2 obtained in the first exposure period EP1 from the noise reducing section 62. In addition, a correction image signal IS3 obtained in the second exposure period EP2 is received.

Subsequently, the brightness calculation section 140 of the control amount output section 130 calculates observation image signal brightness Y1 from the observation image signal 111 and calculates correction image signal brightness Y2 from the correction image signal IS3. The brightness calculation section 140 outputs the image signal brightness from some of the different image signals depending on the kinds of the received image signals. The reason is that the image signal brightness for outputting a control amount is calculated from some of the different image signals, and consequently, the exposure control can be more appropriately implemented on the image signals of the observation image signal and the correction image signal. A specific method of calculating the image signal brightness from some of the obtained image signals will be described later. The image signal brightness is information about brightness that the various image signals have.

Subsequently, the control amount output section 130 outputs a first control amount CQ1, based on the observation image signal 111 and outputs a second control amount CQ2, based on the correction image signal IS3. The control amounts have a value that changes depending on the image signal brightness. The reason is that the appropriate exposure amount changes depending on the kinds of the image signals to be obtained and changes depending on the image signals that are obtained for every frame even when the same kind of the illumination light is emitted.

The reason why the control amounts differ from each other depending on the kinds of the image signals to be obtained will be described. The white light and the calculation illumination light are illumination light obtained by combining light in multiple colors and have similar spectrums (see Fig. 3 and Fig. 4), and accordingly, the control amounts that are outputted for the exposure control have values close to each other. The spectrum of the correction illumination light greatly differs from that of the observation illumination light (see Fig. 3 to Fig. 5), and accordingly, the control amount based on the correction image signal outputted for the exposure control differs from the control amount based on the observation image signal.

The exposure control signal generation section 120 generates a first exposure control signal ECS1, based on the first control amount CQ1 and generates a second exposure control signal ECS2, based on the second control amount CQ2. The exposure control signals are transmitted from the exposure control unit 110 to the central control unit 50, and first exposure control EC1 depending on the first exposure control signal ECS1 and second exposure control EC2 depending on the second exposure control signal ECS2 are implemented on the light source unit 20 and the image pick-up optical system 40. The first exposure control EC1 and the second exposure control EC2 are control on obtaining the image signal

**EP 4 461 202 B1**

and emitting the illumination light in a subsequent exposure period after an exposure period in which the image signal is obtained. The content of the exposure control will be described in detail later. The control amounts that determine the content of the exposure control signals differ from each other, and accordingly, the first exposure control EC1 and the second exposure control EC2 have different contents.

With the structure described above, the exposure control is implemented based on the image signals that are obtained in multiple kinds of frames and that differ from each other and based on the image signal brightness that the image signals have, brightness between the different kinds of the image signals is prevented from being insufficient and being excessive, and the image signals can be obtained by performing image pick-up in subsequent frames in more appropriate conditions based on the control amounts.

The control amounts are preferably outputted based on a difference between the image signal brightness calculated from the image signals and target brightness. The brightness calculation section 140 calculates the image signal brightness by using the image signals. The brightness difference calculation section 150 calculates a difference (a brightness difference) between the predetermined target brightness and the image signal brightness. The target brightness has a target value of predetermined information for various kinds of the image signals about the brightness that the various kinds of the image signals have. Subsequently, the control amount output section 130 outputs the control amounts, based on the brightness difference. A method of outputting the control amounts is not limited thereto. For example, the amount of light, aperture diaphragm value, or exposure time of each light source that emits light when the image signals are obtained may be stored, a difference between the value thereof and the target value may be calculated, and the control amounts may be outputted. With the structure described above, the appropriate exposure control can be sequentially implemented based on the image signal brightness obtained depending on the purpose of observation.

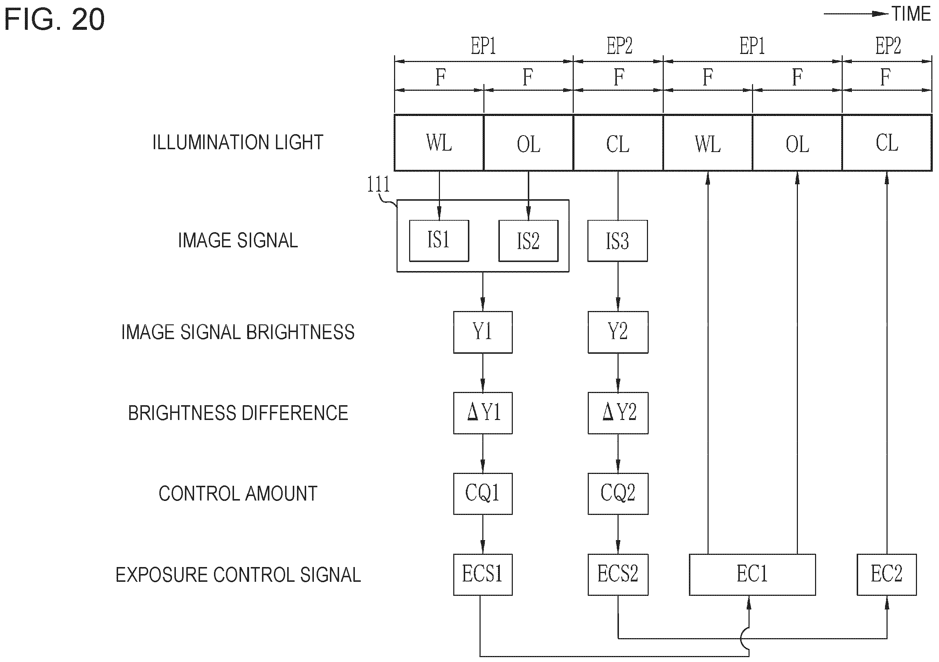

The brightness difference is preferably calculated from the image signals that are obtained depending on the exposure periods after the image signal brightness is calculated, the control amounts are preferably outputted from the brightness difference, and the exposure control signals based on the control amounts are preferably generated sequentially in subsequent frames. Specifically, as illustrated in Fig. 20, the brightness calculation section 140 calculates the observation image signal brightness (first image signal brightness) Y1 from the observation image signal 111 and calculates the correction image signal brightness (second image signal brightness) Y2 from the correction image signal IS3. The image signal brightness is calculated depending on the image signals that are obtained in each mode.

Subsequently, the brightness difference calculation section 150 calculates the difference between the predetermined target brightness and the image signal brightness. As for the target brightness, observation image signal target brightness (first target brightness) and correction image signal target brightness (second target brightness) are set. The first target brightness and the second target brightness may have the same value or different values. As illustrated in Fig. 20, an observation image signal brightness difference $\Delta Y1$ between the observation image signal brightness Y1 and the observation image signal target brightness is calculated. A correction image signal brightness difference $\Delta Y2$ between the correction image signal brightness Y2 and the correction image signal target brightness is calculated. The brightness difference is calculated in each mode.

Subsequently, the control amount output section 130 outputs the control amounts, based on the brightness differences. As illustrated in Fig. 20, the first control amount CQ1 is calculated based on the observation image signal brightness difference $\Delta Y1$. The second control amount CQ2 is calculated based on the correction image signal brightness difference $\Delta Y2$. Specifically, for example, the observation image signal brightness Y1 and the observation image signal brightness difference $\Delta Y1$ are substituted for a first control amount calculation function $f(Y1, \Delta Y1)$, and the first control amount CQ1 is calculated. The correction image signal brightness Y2 and the correction image signal brightness difference $\Delta Y2$ are substituted for a second control amount calculation function $f(Y2, \Delta Y2)$, and the second control amount CQ2 is calculated. A method of calculating the control amount is not limited thereto.

Subsequently, the exposure control signal generation section 120 generates the exposure control signals, based on the control amounts. As illustrated in Fig. 20, the first exposure control signal ECS1 is generated based on the first control amount CQ1, and the second exposure control signal ECS2 is generated based on the second control amount CQ2. The generated exposure control signals are transmitted to the central control unit 50, and the first exposure control EC1 depending on the first exposure control signal ECS1 and the second exposure control EC2 depending on the second exposure control signal ECS2 are implemented. With the structure described above, the exposure control suitable for the exposure period in which the observation illumination light is emitted and the exposure period in which the correction illumination light is emitted can be implemented.

The second control amount CQ2 is preferably larger than the first control amount CQ1. The reason is that the observation illumination light is bright illumination light obtained by combining light in multiple colors, but the correction illumination light is dark illumination light that includes light in a single color. The exposure amount for the image pick-up sensor 43 in the second exposure period in which the illumination light emitted is dark is smaller than the exposure amount in the first exposure period. Accordingly, an increase in the control amount enables the degree of the brightness of the image signals that are obtained in the first exposure period to be increased. Another reason is that the image signals that have a certain degree of the image signal brightness are preferably continuously obtained to accurately calculate the correction oxygen saturation. The image signal brightness obtained changes due to, for example, the magnification of image pick-up. As for the correction image signals that need a signal value more precise than the observation image signal, it is necessary to control the image signal brightness that is particularly necessary for every image pick-up scene with precision. Accordingly, when the second control amount is larger than the first control amount, the optimal exposure control for the image pick-up scene can be continuously implemented, and the precise signal value can be obtained.

The brightness calculation section 140 of the control amount output section 130 preferably outputs the image signal brightness by using the image signals (the specific color signals) that are obtained from the pixels for the specific color. For example, B image signals (B signals) are preferably used as the specific color signals. The reason is that among the obtained specific color signals, the degree of the image signal brightness of the B signals (the B1 image signal, the B2 image signal, and the B3 image signal) is relatively lower than that of a G image signal (a G signal) and a R image signal (a R signal), and accordingly, the exposure control suitable for the B signals enables the image signals that have a large degree of the image signal brightness to be obtained. In this case, the B1 image signal and/or the B2 image signal is used as the observation image signal, the B3 image signal is used as the correction image signal, the image signal brightness and the brightness differences are calculated, and the first control amount and the second control amount are outputted.

Specifically, in the case where the target brightness for each B signal is set as "B = 100", the brightness differences are calculated depending on the magnitude of the brightness of the B signal (the B1 image signal, the B2 image signal, or the B3 image signal). The target brightness for the specific color signals may be changed depending on the respective image signals. For example, the target brightness for the B1 image signal and the B2 image signal may be set as "B = 50", and the target brightness for the B3 image signal may be set as "B = 100". The specific color signals may be the G signals, the R signals, or image signals for four colors of CMYG. The target brightness can be freely set depending on the specific color signals. The specific color signals that are used to calculate the image signal brightness may be changed depending on the kinds of the image signals. For example, as for the observation image signal, the G1 image signal may be used, and as for the correction image signal, the B3 image signal may be used.

In the case where the G pixels of the image pick-up sensor 43 are divided into Gb pixels and Gr pixels, Gb image signals are obtained from the Gb pixels, Gr image signals are obtained from the Gr pixels, and the image signal brightness is calculated by using an expression of $Y = (K1*Gb + K2*Gr + K3*B + K4*R + K5) / 256$ (Gb, Gr, B, and R are pixel values, and K1, K2, K3, K4, and K5 are freely set coefficients), the brightness differences may be calculated after the coefficients are changed depending on the kinds of the image signals, and the control amounts may be outputted.

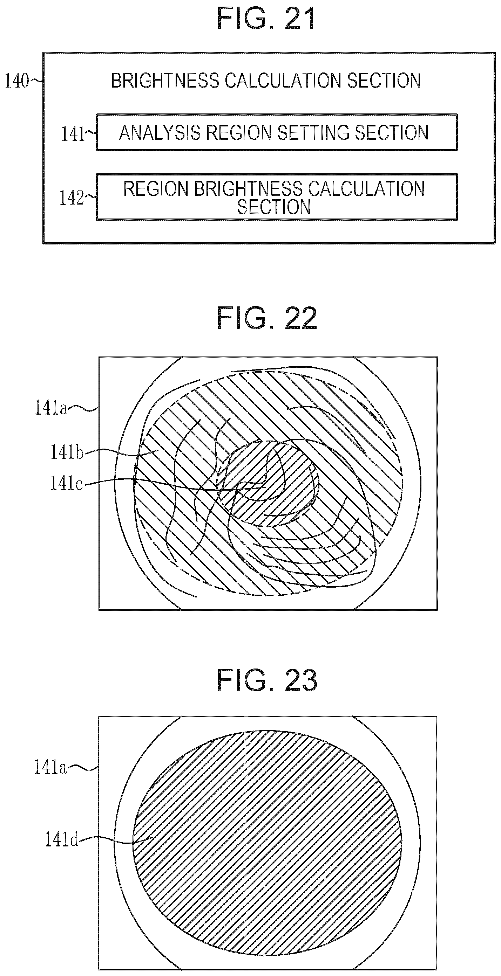

The control amount output section 130 preferably calculates the image signal brightness by using image signals in a region of an analysis image generated from the image signals. In this case, as illustrated in Fig. 21, the analysis region setting section 141 and the region brightness calculation section 142 are preferably provided in the brightness calculation section 140. The analysis region setting section 141 generates the analysis image from the image signals and extracts a specific region from the analysis image. The region brightness calculation section 142 calculates the brightness from an image signal in a specific image region.

The specific region is preferably a region in which the reliability of the signal value of the image signal and analysis precision are high. As illustrated in Fig. 22, in the case where observation is made such that a portion behind a lumen is visible, an endoscope image 141a generated such that the object to be observed is a lumen of a luminal organ such as the esophagus, stomach, small intestine, or large intestine is divided into a front region, that is, a region 141b that is near the tip portion 12d of the endoscope 12 and a rear region, that is, a region that is far from the tip portion 12d of the endoscope 12. As for a rear region 141c, the illumination light is unlikely to reach thereto, the brightness is not sufficient, and accordingly, the analysis precision is low. The front region 141b, however, is illuminated with a sufficient amount of light, is accordingly bright, and the analysis precision is high, which is suitable for the specific region. On the periphery of the endoscope image 141a, the degree of image distortion is typically high, and accordingly, the analysis precision is low. At the center of the endoscope image 141a, the degree of the image distortion is low, and the analysis precision is accordingly high, which is suitable for the specific region.

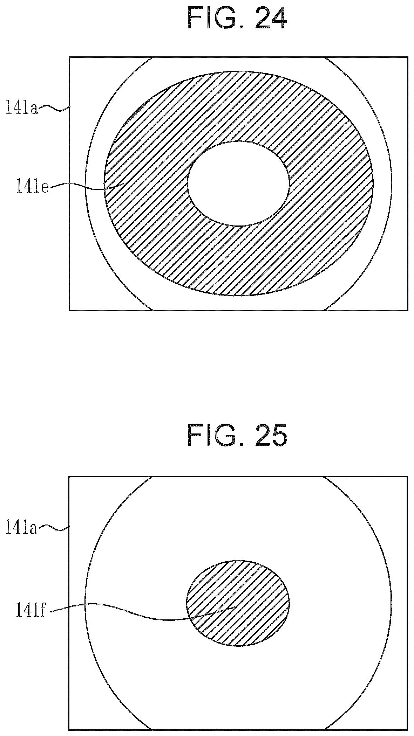

The specific region is preferably extracted so as to follow a specific shape that is set in advance depending on the kinds of the obtained image signals. The specific shape is set in consideration of the analysis precision. Examples include a shape 141d (an oblique line portion) obtained by removing outermost peripheral regions as illustrated in Fig. 23, a shape 141e (an oblique line portion) that has a donut shape obtained by removing regions near the periphery and a region near the center corresponding to the portion behind the lumen as illustrated in Fig. 24, and a shape 141f (an oblique line portion) obtained by extracting a region near the center as illustrated in Fig. 25. Luminance may be calculated from the image signals, and the specific region may be extracted based on the luminance. The case where a method of extraction into a predetermined shape is not appropriate, for example, the case where the portion behind the lumen is seen at the periphery of the endoscope image, or the case where the portion behind the lumen is not included in the endoscope image as a result of image pick-up of a mucosa at high magnification in a close range can be dealt with.

The analysis region setting section 141 extracts the specific region by using the substantially white light image signals or the calculation image signals that are the observation image signals. The specific region is extracted by using the correction image signals. In this case, an observation analysis image (a first analysis image) is generated by using the observation image signals, and an observation analysis region (a first region) is extracted as the specific region. A correction analysis image (a second analysis image) is generated by using the correction image signals, and a correction analysis region (a second region) is extracted as the specific region. Subsequently, the region brightness calculation section 142 calculates observation region brightness (first region brightness) as region brightness from the first region. Correction region brightness (second region brightness) is calculated from the second region.

Subsequently, the first region brightness and the second region brightness are transmitted to the brightness difference calculation section 150. The brightness difference calculation section 150 calculates an observation image signal region brightness difference as the brightness difference between the first region brightness and the first target brightness. A correction image signal region brightness difference is calculated as the brightness difference between the second region brightness and the second target brightness. The control amount output section 130 outputs a first region control amount, based on the observation image signal region brightness difference and outputs a second region control amount, based on the correction image signal region brightness difference.

The exposure control signal generation section 120 generates a first region exposure control signal, based on the first region control amount and generates a second region exposure control signal, based on the second region control amount. Finally, the first exposure control EC1 depending on the first region exposure control signal and the second exposure control EC2 depending on the second region exposure control signal are sequentially implemented, and consequently, the appropriate exposure control depending on the exposure periods is implemented.

A user such as a doctor preferably makes a diagnosis by using the substantially white light image (or the normal light image) and the oxygen saturation image in a wide range (such as a region included in the shape in Fig. 23 or Fig. 24) of the endoscope image except for regions near the periphery at which the degree of distortion is high and near the portion behind the lumen at which observation is difficult. When the correction oxygen saturation is calculated, an image signal of a region (such as a region of the shape in Fig. 25) near the center of the image at which the analysis precision is high is preferably used to calculate the correction oxygen saturation with high precision. For this reason, as for the observation image signal (the substantially white light image signal and/or the calculation image signal) and the correction image signal, the image signals that are used for the exposure control preferably differ from each other due to the use of different analysis regions.

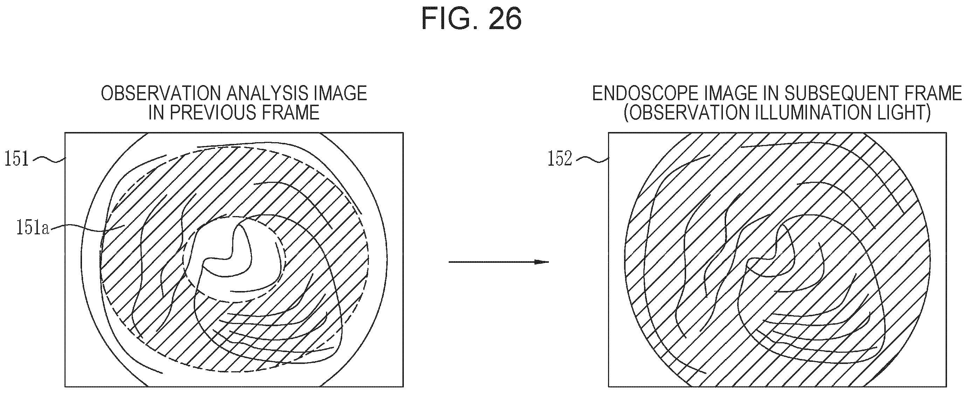

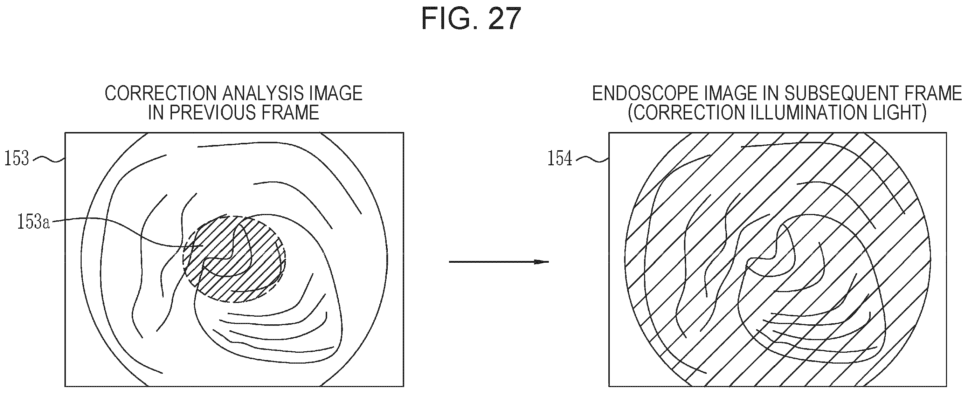

Specifically, in the case where the observation image signal 111 is obtained, as illustrated in Fig. 26, a specific region 151a (the first region) is extracted from a first analysis image 151 (the observation analysis image in a previous frame in Fig. 26), and the first region exposure control signal is generated by using the observation image signal of the first region. As a result, the brightness of an observation image signal 152 (the endoscope image (the observation illumination light) in a subsequent frame in Fig. 26) obtained in the subsequent first exposure period is adjusted. In the case where the correction image signal is obtained, as illustrated in, for example, Fig. 27, a specific region 153a (the second region) is extracted from a second analysis image 153 (the correction analysis image in a previous frame in Fig. 27), and the second region exposure control signal is generated by using the correction image signal of the second region. As a result, the brightness of a correction image signal 154 (the endoscope image (the correction illumination light) in a subsequent frame in Fig. 27) obtained in the subsequent second exposure period is adjusted.

In Fig. 26 and Fig. 27, the brightness of the image signals is illustrated by using the concentration of the oblique line portions. That is, in the case where the exposure control is implemented by using the first region 151a that is relatively bright and that is suitable for diagnosis in the observation image signal that is originally relatively bright, the control amounts are small, and the amount of change in the brightness from that of the image signal subsequently obtained is small. In the case where the exposure control is implemented by using the second region 153a that has high analysis precision and that is darker than the correction image signal that is originally dark, the control amounts are large, and the amount of change in the brightness from that of the image signal subsequently obtained is large. In this way, the image signals that are used for the exposure control can be changed depending on the purpose. With the structure described above, the image signals that have more appropriate brightness depending on the kinds of the image signals can be obtained.

In the case where the control amounts are outputted by using some of the obtained image signals, and the exposure control signals are generated, a method of using the specific color signals and a method of using the image signals in the specific region may be combined. For example, the G1 image signal included in the specific region illustrated in Fig. 23 or Fig. 24 may be used to calculate the observation image signal brightness, and the B3 image signal included in the specific region illustrated in Fig. 25 may be used to calculate the correction image signal brightness.

In the observation mode in which the observation image signals are obtained and the correction mode in which the correction image signals are obtained in addition to the observation image signals, different exposure control signals are preferably generated, and the exposure control is preferably implemented in different manners. In the observation mode and the correction mode, the obtained image signals differ from each other. Accordingly, as illustrated in, for example, Fig. 19, the exposure control signals that are consequently generated differ from each other. Accordingly, the exposure control can be implemented in different manners depending on the modes.

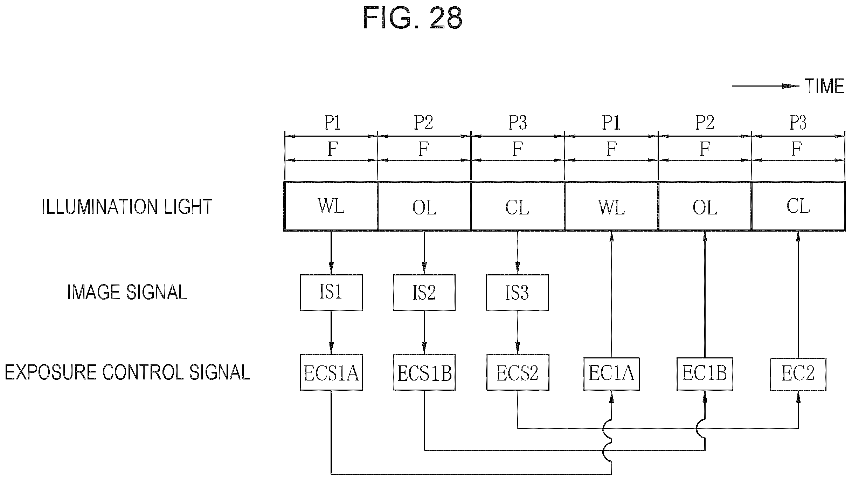

Based on three kinds of the image signals of the substantially white light image signal IS1, the calculation image signal IS2, and the correction image signal IS3 that are obtained, the generated exposure control signals preferably differ from each other, and the exposure control is preferably implemented in different manners. A specific example will be described with reference to Fig. 28. In this case, the exposure control signal generation section 120 generates a first A exposure control signal ECS1A, based on the substantially white light image signal IS1 obtained in the first illumination period. In addition, the exposure control signal generation section 120 generates a first B exposure control signal ECS1B, based on the calculation image signal IS2 obtained in the second illumination period. In addition, the exposure control signal generation section 120 generates the second exposure control signal ECS2, based on the correction image signal IS3 obtained in the third illumination period.

The exposure control unit 110 transmits the exposure control signals to the central control unit 50 and finally implements first A exposure control EC1A depending on the first A exposure control signal ECS1A, first B exposure control EC1B depending on the first B exposure control signal ECS1B, and second exposure control EC2 depending on the second exposure control signal ECS2. With the structure described above, the substantially white light image signal, the calculation image signal, and correction image signal that have more appropriate image signal brightness for the observation of the object to be observed or the calculation of the tissue oxygen saturation can be obtained.

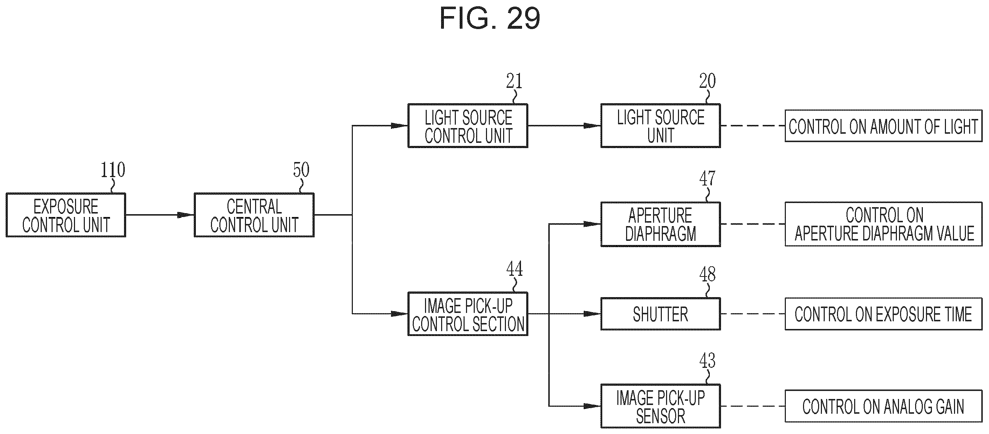

The exposure control implemented by the exposure control unit 110 preferably includes control on the amount of light for controlling the light source unit 20, control on the aperture diaphragm value of the aperture diaphragm 47 in the image pick-up optical system 40, control on the exposure time for controlling the shutter 48, or control on the gains for controlling the image pick-up sensor 43. As illustrated in Fig. 29, the exposure control unit 110 transmits the exposure control signals and consequently controls the light source control unit 21 or the image pick-up control section 44 by using the central control unit 50. In the case where the exposure control signals are transmitted to the light source control unit 21, the light source control unit 21 controls the light source unit 20 and adjusts the amount of light and the ratio of the amount of light.

In the case where the amount of light is controlled, the target amount of light may be set. In the case where the target amount of light is set, the exposure control unit 110 transmits, as the exposure control signal, a light amount control signal depending on the brightness difference and image signal brightness based on the image signals to the light source control unit 21 via the central control unit 50. The light source control unit 21 controls the amount of light of the light sources of the light source unit 20, based on the light amount control signal.

Specifically, the exposure control unit 110 generates the light amount control signal, based on the first control amount CQ1 outputted by using the observation image signal 111, and the light source control unit 21 adjusts the amount of light or the ratio of the amounts of light of the BS-LED 20b, the BL-LED 20c, the G-LED 20d, and the R-LED 20e. The light amount control signal is generated based on the second control amount CQ2 calculated by using the correction image signal IS3, and the light source control unit 21 adjusts the light of the G-LED 20d. With the structure described above, the illumination light in subsequent frames can be sequentially emitted in the amount of light suitable for the frames, and the appropriate exposure amount can be obtained. The control on the amount of light is not limited thereto.

In the case where the exposure control signals are transmitted to the image pick-up control section 44, the image pick-up control section 44 controls the aperture diaphragm 47, the shutter 48 and/or the image pick-up sensor 43. In the case where the exposure control is implemented such that the aperture diaphragm 47 is controlled, for example, the exposure control unit 110 sets a target aperture diaphragm value, generates the exposure control signals, controls the aperture diaphragm 47, and consequently adjusts the aperture diaphragm value. In the case where the exposure control is implemented such that the shutter 48 is controlled, for example, the exposure control unit 110 sets a target frame rate or a target shutter speed and generates the exposure control signals, controls the shutter 48, and adjusts the exposure time. In the case where the exposure control is implemented such that the image pick-up sensor 43 is controlled, for example, the exposure control unit 110 sets a target gain, controls the image pick-up sensor 43, and adjusts an analog gain. The control on the image pick-up optical system is not limited thereto.

A method for the exposure control is not limited to the above description. The exposure control may be set such that the methods described above are combined. With the structure described above, the appropriate method can be selected based on the image signals that are obtained in the exposure periods, and the exposure control can be implemented.

According to the present embodiment, the endoscope 12 is a soft endoscope. However, the present invention is suitable for the case of using a hard endoscope (a laparoscope) used for, for example, a surgery. In the case where a soft endoscope is used, the object to be observed is a mucosa in a surface layer viewed from a lumen of a luminal organ, and the endoscope image such as the tissue oxygen saturation image obtained by calculating the tissue oxygen saturation of the mucosa in the surface layer is displayed. In the case where a laparoscope is used, the object to be observed is an organ viewed from the serosa, and the endoscope image such as the tissue oxygen saturation image obtained by calculating the tissue oxygen saturation of the surface of the organ is displayed.

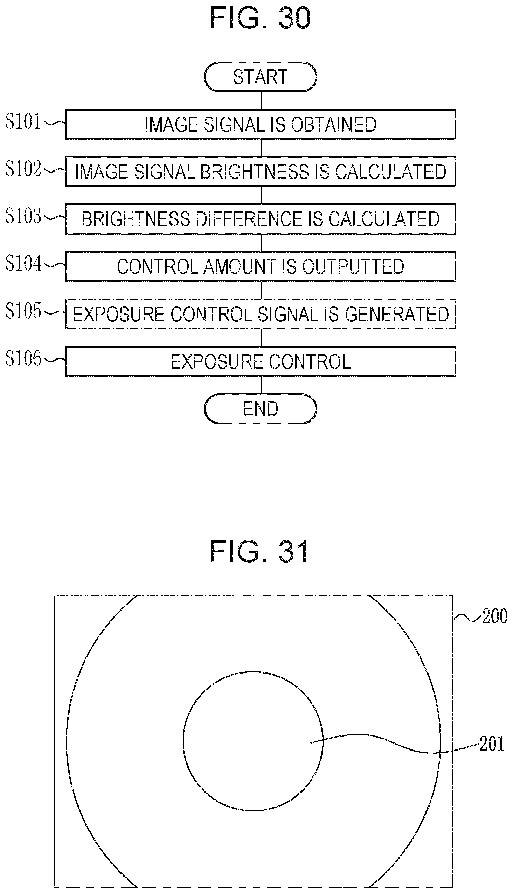

The series of flow regarding the exposure control for every frame according to the present embodiment will be described. As illustrated in Fig. 30, the image signal obtaining section 60 obtains an image signal (S101), the brightness calculation section 140 calculates the brightness of the image signal (S102), the brightness difference calculation section 150 calculates the brightness difference between the calculated brightness and the target brightness (S103), the control amount output section outputs the control amount (S104), the exposure control signal generation section 120 generates the exposure control signal, based on the control amount (S105), and the exposure control unit 110 implements the exposure control for a subsequent frame (S106).

In the case where the multiple kinds of the image signals are obtained, according to the embodiment described above, the image signals in the subsequent frames can be obtained in more appropriate conditions. The "multiple kinds of the image signals" are the normal light image signals, the substantially white light image signals, the calculation image signals, or the correction image signals illustrated in Table 1 to Table 3.

The exposure control according to the present embodiment is sequentially implemented in manners that differs from each other depending on the exposure periods in which the image signals are obtained, based on the multiple kinds of the image signals that are obtained for different purposes in a manner in which the object to be observed is irradiated with the illumination light with different spectrums. Accordingly, the brightness of the image signals can be prevented from being insufficient and being excessive due to the exposure control based on a single kind of an image signal, and the image signals can be obtained by performing image pick-up in more appropriate conditions.

When the correction oxygen saturation calculation table 86b (the curved surfaces 86c to 86g illustrated in Fig. 17) depending on the pigment value is referred, and the correction oxygen saturation is calculated in the correction mode, some of the image signals that are obtained in the correction mode may be used. In the following description, a process of selecting the correction oxygen saturation calculation table 86b depending on the pigment value is referred to as a "correction process". The some of the image signals are image signals in a correction specific region in a correction image described later (see Fig. 31 described later). In this case, the correction specific region is preferably a region in which the influence of a disturbance on the precision of calculation of the oxygen saturation is weak. The reliability of the image signals in the correction specific region is calculated to determine the degree of the influence of the disturbance in the correction specific region.

Example of the disturbance include halation, a dark region, bleeding, fat, and an adhering substance on the surface of the mucosa that can cause the precision of calculation of the oxygen saturation to reduce except for the specific pigment in the object to be observed in the endoscope image that is imaged by the endoscope 12. The halation and the dark region relate to the brightness of the endoscope image. The halation is a white region in the image due to the intense light that enters the image pick-up sensor 43. The dark region is a region that is dark in the image and that the illumination light is unlikely to reach due to the influence of a shadow of a structure such as a treatment tool in an organism such as a fold or a colic flexure or due to the portion behind the lumen.

Examples of bleeding include external bleeding outside the serosa (inside the abdominal cavity) or into the gastrointestinal lumen and internal bleeding inside the mucosa. Examples of fat include fat observed outside the serosa (inside the abdominal cavity) such as the greater omentum, the lesser omentum, or the mesentery and fat observed on the surface of the mucosa of the gastrointestinal lumen. Examples of the adhering substance on the surface of the mucosa include an adhering substance originated from an organism such as mucus, blood, or exudate, an adhering substance originated outside an organism such as a staining solution or water supplied from a water supply device, and an adhering substance that is a residue or a remaining liquid of a mixture of the adhering substances originated from the organism and outside the organism.

In the case where the correction process is performed in the correction mode by using the image signals in the correction specific region, the display 15 displays a correction image 200 illustrated in Fig. 31 with a timing with which the mode is switched to the correction mode. The display control unit 100 controls the display of the correction image 200. The correction image 200 includes a correction specific region 201 that is visible by the user.

The shape of the correction specific region 201 is not limited to a circular shape illustrated in Fig. 31. The position of the correction specific region 201 is not limited to a central portion of the image illustrated in Fig. 31. For example, the correction specific region may be a donut-shaped region except for the peripheral portion of the correction image 200 at which the influence of distortion increases due to a lens curvature and a central portion of the correction image 200 that corresponds to the portion behind the lumen and that is accordingly the dark portion. The correction image is preferably a color image (such as the substantially white light image) generated by using the B1 image signal, the G1 image signal, and the R1 image signal. The correction image may be generated by using another image signal.

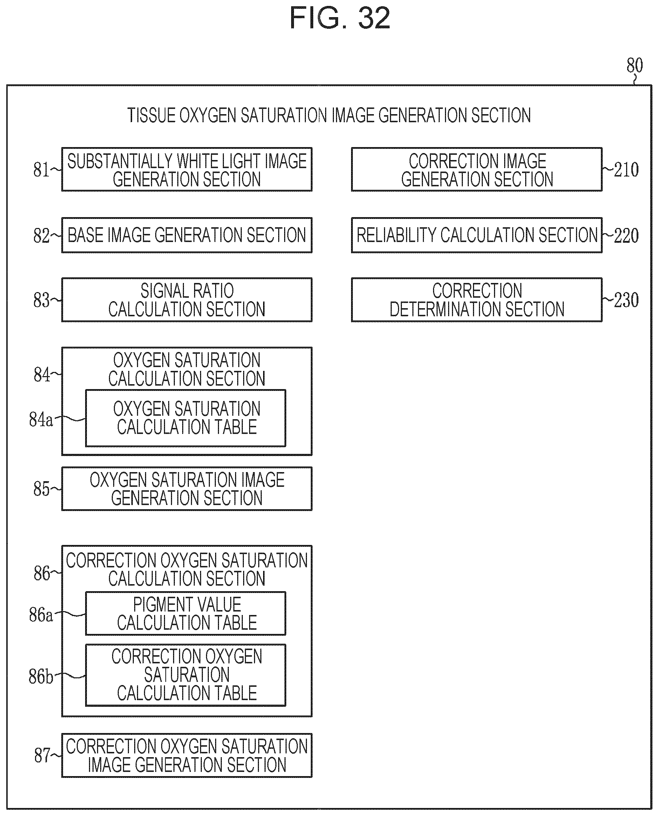

In the case where the correction image is displayed, and the reliability is calculated by using the image signals in the correction specific region, as illustrated in Fig. 32, the correction image is generated by a correction image generation section 210 of the tissue oxygen saturation image generation section 80. In this case, as illustrated in Fig. 32, the tissue oxygen saturation image generation section 80 further includes a reliability calculation section 220 and a correction determination section 230 in addition to the correction image generation section 210.

In the case where the display 15 displays the correction image 200 illustrated in Fig. 31, an instruction for calculating the reliability is inputted, and the reliability calculation section 220 subsequently calculates the reliability for every pixel included in the correction specific region 201, based on the image signals in the correction specific region 201. The instruction for calculating the reliability may be inputted in accordance with an instruction for an input via a user interface or may be automatically inputted with the same timing as the control for displaying the correction image 200.

The calculation of the reliability will now be described. Examples of the reliability include (1) reliability about the brightness of the endoscope image, (2) reliability due to the degree of bleeding included in the endoscope image, and (3) reliability due to the degree of fat included in the endoscope image.

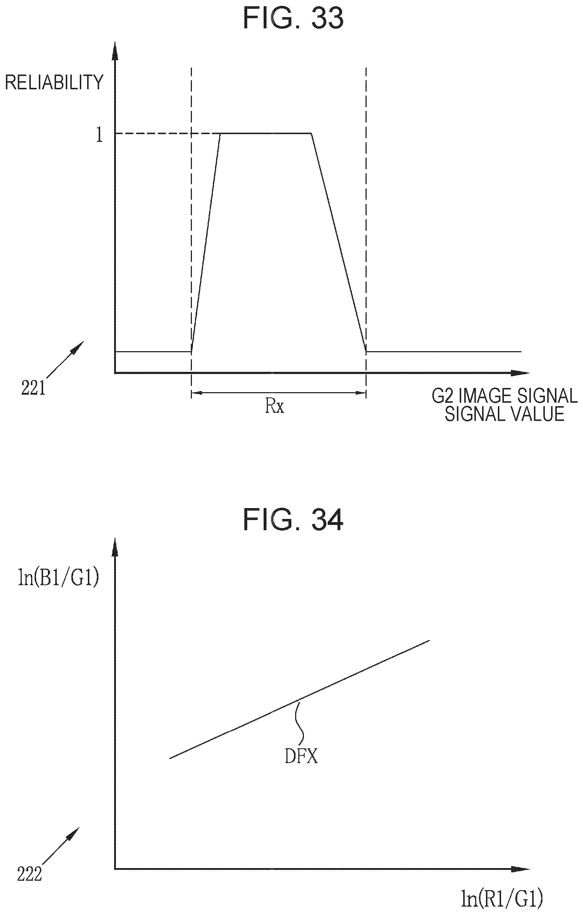

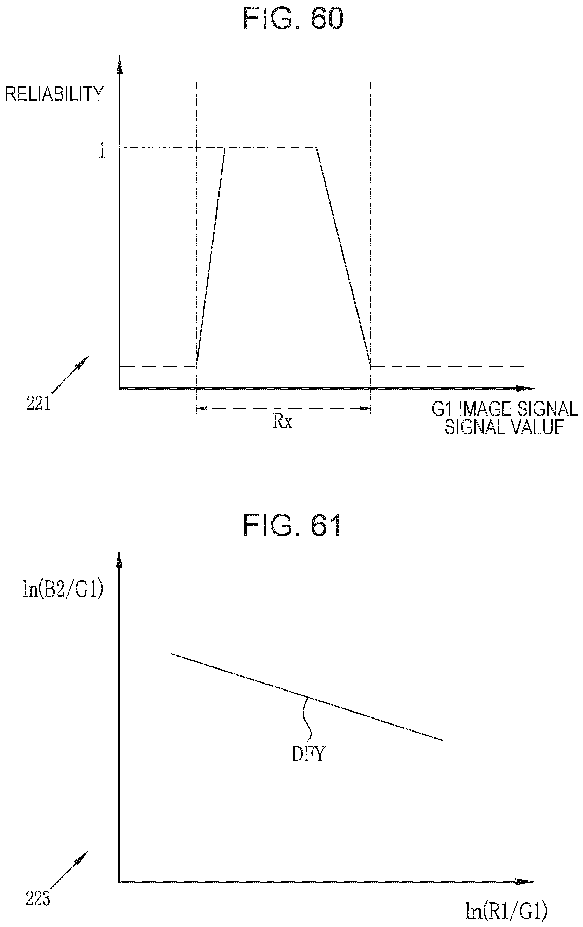

The calculation of the reliability about the brightness will be described. In this case, the reliability calculation section 220 calculates the reliability by referring a first reliability calculation table 221 illustrated in Fig. 33 by using the G2 image signal. The first reliability calculation table 221 represents a relationship between the signal value of the G2 image signal and the reliability and is generated in advance. For example, the signal value of the G2 image signal is a brightness value obtained by performing a conversion process by using the G2 image signal. In this case, the reliability is calculated as a value from 0 to 1. In the first reliability calculation table 221, as illustrated in Fig. 33, the reliability when the signal value of the G1 image signal is out of a certain range Rx is lower than the reliability when the brightness value of the G1 image signal is within the certain range Rx. Specific examples of the case where the signal value of the G1 image signal is out of the certain range Rx includes the case where the halation is included in the pixels, and consequently, the brightness value is large and the case where the dark region is included in the correction specific region, and consequently, the brightness value is very small. The reliability about the brightness may be calculated by using the G1 image signal instead of the G2 image signal (see Fig. 60 described later).

The calculation of the reliability due to the degree of bleeding will be described. In this case, the reliability calculation section 220 calculates the reliability by referring a second reliability calculation table 222 illustrated in Fig. 34 by using the signal ratio ln(R1/G1) and the signal ratio ln(B1/G1). As for the second reliability calculation table 222, a definition line DFX is plotted in a two-dimensional coordinate system where an X-axis represents ln(R1/G1), and a Y-axis represents the signal ratio ln(B1/G1). In this case, the calculation is made such that the reliability reduces as coordinates of $(X1, Y1) = (\ln(R1/G1), \ln(B1/G1))$ where the value of an X component is the signal value ln(R1/G1), and the value of a Y component is the signal ratio ln(B1/G1) are nearer to the lower right in the second reliability calculation table 222. In the case where the coordinates (X1, Y1) are located on an upper left region from the definition line DFX, the reliability due to the degree of bleeding is determined to be a fixed value that represents high reliability. The signal ratio ln(R1/G1) has a value obtained by standardizing the R1 image signal by using the G1 image signal and is a logarithm. The signal ratio ln(B1/G1) has a value obtained by standardizing the B1 image signal by using the G1 image signal and is a logarithm.

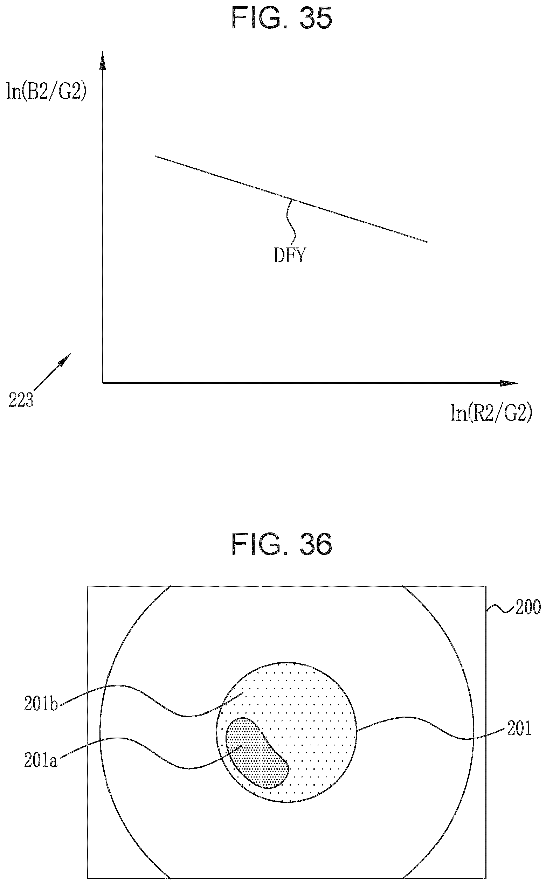

The calculation of the reliability due to the degree of fat will be described. In this case, the reliability calculation section 220 calculates the reliability by referring a third reliability calculation table 223 illustrated in Fig. 35 by using the signal value ln(R2/G2) and the signal ratio ln(B2/G2). As for the third reliability calculation table 223, a definition line DFY is plotted in a two-dimensional coordinate system where an X-axis is the ln(R2/G2) and a Y-axis is the signal ratio ln(B2/G2). In this case, the calculation is made such that the reliability reduces as coordinates $(X2, Y2) = (\ln(R2/G2), \ln(B2/G2))$ where the value of an X component is the signal value ln(R2/G2), and the value of a Y component is the signal ratio ln(B2/G2) are nearer to the lower left in the third reliability calculation table 223. In the case where the coordinates (X2, Y2) are located in an upper right region from the definition line DFY, the reliability due to the degree of fat is determined to be a fixed value that represents high reliability. The signal ratio ln(R2/G2) has a value obtained by standardizing the R2 image signal by using the G2 image signal and is a logarithm. The signal ratio ln(B2/G2) has a value obtained by standardizing the B2 image signal by using the G2 image signal and is a logarithm. The reliability due to the degree of fat may be calculated by using a position in the reliability calculation table regarding coordinates $(X2, Y2) = (\ln(R1/G1), \ln(B2/G1))$ where the value of an X component is the signal value ln(R1/G1), and the value of a Y component is the signal ratio ln(B2/G1) by using the G1 image signal instead of the G2 image signal and by using the R1 image signal instead of the R2 image signal (see Fig. 61 described later).

The reliability calculation section 220 calculates at least one or more of the reliability (first reliability) about the brightness, the reliability (second reliability) due to the degree of bleeding, or the reliability (third reliability) due to the degree of fat. The calculated reliability is used for a notification for preventing the correction specific region from including a region that has low reliability or in a process of weighting the signal values (the signal ratios ln(R1/G1), ln(B2/G1), and ln(B3/G3)) of the image signals that are used in the correction process.

In the case where the notification for preventing the correction specific region from including a region that has low reliability is given, the calculated reliability is transmitted to the correction determination section 230. The correction determination section 230 determines the reliability calculated for every pixel in the correction specific region by using a predetermined reliability determination threshold value and outputs a determination result that represents whether each pixel is a highly reliable pixel or an insufficiently reliable pixel.

For example, the correction determination section 230 determines that a pixel the reliability of which is equal to or more than the reliability determination threshold value is the highly reliable pixel, and a pixel the reliability of which is less than the reliability determination threshold value is the insufficiently reliable pixel. The correction determination section 230 transmits the determination result of determination of the reliability of each pixel to the display control unit 100. The display control unit 100 implements control such that the display form of the correction image 200 displayed on the display 15 is changed depending on the determination result.

As illustrated in, for example, Fig. 36, the display control unit 100 determines that the saturation of an insufficiently reliable region 201a is more than the saturation of a highly reliable region 201b in the correction specific region 201. The insufficiently reliable region is a pixel set of the insufficiently reliable pixels. The highly reliable region is a pixel set of the highly reliable pixels. The correction image 200 is displayed such that the display forms of the insufficiently reliable region 201a and the highly reliable region 201b differ from each other as illustrated in Fig. 36, and consequently, the user can be notified of the correction specific region that includes the insufficiently reliable region that has a relatively large amount of disturbances. A method of changing the display forms of the insufficiently reliable region 201a and the highly reliable region 201b from each other is not limited to a method of changing the saturation in each region. For example, luminance or color tone, for example, may be changed.

In the case of calculating multiple kinds of the reliability among the first reliability, the second reliability, and the third reliability, reliability used for determining the reliability is the minimum reliability among the first reliability, the second reliability, and the third reliability. The reliability determination threshold value may be set for every kind of the reliability. For example, a first reliability determination threshold value for the first reliability, a second reliability determination threshold value for the second reliability, and a third reliability determination threshold value for the third reliability may be set in advance, and in the case where the reliability is less than the reliability determination threshold value, the pixel the reliability of which is calculated may be determined to be the insufficiently reliable pixel.

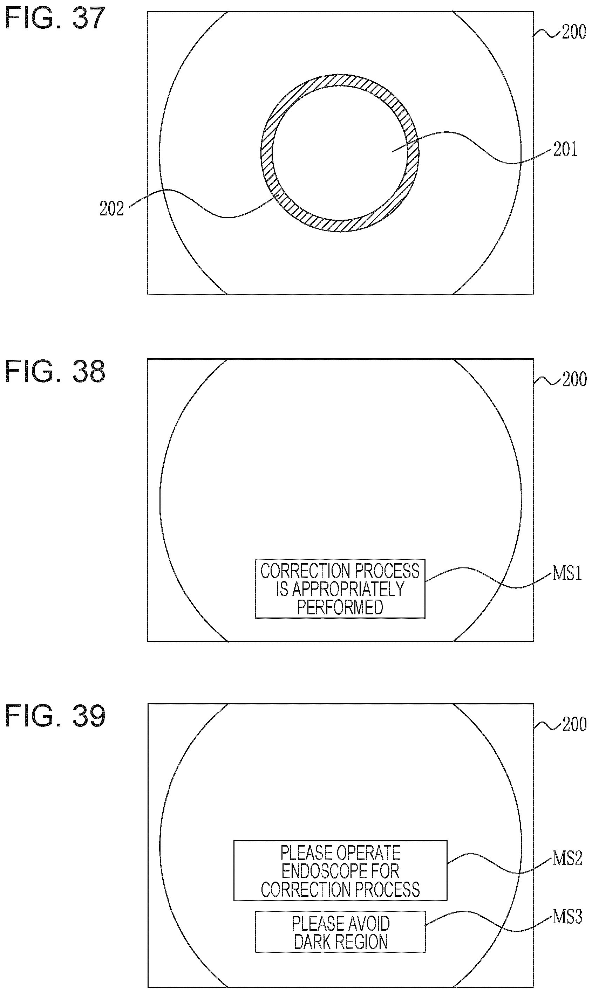

The correction determination section 230 may make a determination with respect to the number of the highly reliable pixels relative to the reliability calculated for every pixel. In this case, the display control unit 100 changes the display form of the correction specific region between the case where the number of the highly reliable pixels in the correction specific region is equal to or more than a highly reliable pixel number determination threshold value and the case where the number of the highly reliable pixels in the correction specific region is less than the highly reliable pixel number determination threshold value. For example, in the case where the number of the highly reliable pixels in the correction specific region is equal to or more than the highly reliable pixel number determination threshold value, as illustrated in Fig. 37, the correction image 200 is emphatically displayed such the correction specific region is surrounded by a border 202 in a first determination result color. The emphatical display such that the correction specific region is surrounded by the border in the first determination result color enables the user to be notified of the correction process that can be performed with the influence of the disturbance being weak.

In the case where the number of the highly reliable pixels in the correction specific region is less than the highly reliable pixel number determination threshold value, the correction image 200 may be emphatically displayed such that the correction specific region is surrounded by a border in a second determination result color that differs from the first determination result color. The emphatical display such that the correction specific region is surrounded by the border in the second determination result color enables the user to be notified of the number of pixels that are less affected by the disturbance being less than a certain value.

The correction determination section 230 may make a determination with respect to the number of the insufficiently reliable pixels, and consequently, the display control unit 100 may change the display form of the correction specific region between the case where the number of the insufficiently reliable pixels in the correction specific region is equal to or more than an insufficiently reliable pixel number determination threshold value and the case where the number of the insufficiently reliable pixels in the correction specific region is less than the insufficiently reliable pixel number determination threshold value. The display form of the correction image is thus changed depending on the number of the pixels that are highly or insufficiently reliable by using a reliability pixel number determination threshold value (the highly reliable pixel number determination threshold value or the insufficiently reliable pixel number determination threshold value), and this enables the user to be notified of a degree at which the disturbance is included in the correction specific region and to be prompted to operate the endoscope for appropriately performing the correction process.

In the case where the correction determination section 230 determines the reliability for every pixel in the correction specific region by using the reliability determination threshold value and/or the reliability pixel number determination threshold value and determines that the influence of the disturbance in the correction specific region is weak, a message that represents that the correction process can be appropriately performed is displayed in the correction image 200. As illustrated in, for example, Fig. 38, a message MS1 "CORRECTION PROCESS IS APPROPRIATELY PERFORMED" is superimposed and displayed on the correction image 200.

In the case where the correction determination section 230 determines the reliability for every pixel in the correction specific region by using the reliability determination threshold value and/or the reliability pixel number determination threshold value, and the insufficiently reliable region is included in the correction specific region, or the number of the insufficiently reliable pixels that are included is equal to or more than the reliability pixel number determination threshold value, a warning may be displayed. As illustrated in, for example, Fig. 39, a message MS2 "PLEASE OPERATE ENDOSCOPE FOR CORRECTION PROCESS" is superimposed and displayed on the correction image 200. In the case where it is determined that the reliability about the brightness is particularly greatly affected, as illustrated in Fig. 39, a message MS3 "PLEASE AVOID DARK REGION" may be superimposed and displayed on the correction image 200.

The display form regarding the correction image 200 is changed as described above, and consequently, the user can be notified of the correction specific region that includes the insufficiently reliable region that has a relatively large amount of disturbances or notified of the correction process that can be appropriately performed. In addition to the image displayed on the display 15, a notification may be given by using a voice.

The notification enables the user to be prompted to operate the endoscope 12 such that a region less affected by the disturbance is in the correction specific region 201. That is, the user can be prompted to operate the endoscope 12 such that no insufficiently reliable region is in the correction specific region, and no highly reliable region is therein as possible.

In the case where it is notified that the correction process can be appropriately performed, and the user inputs an operation of an instruction for performing the correction process, the correction process in the correction mode is performed. In the case where the reliability for every pixel in the correction specific region is determined by using the reliability determination threshold value and/or the reliability pixel number determination threshold value, and the influence of the disturbance on the correction specific region 201 is weak, the correction process may be automatically performed with the user inputting no operation of the instruction for performing the correction process.

The correction process may be performed by using the image signals in the correction specific region after the reliability in the correction specific region is calculated in internal processing of the processor device 14 without displaying the correction image 200 on the display 15, and the reliability for every pixel is determined.

In the case where the correction process is performed, a process of weighting the signal value of the B1 image signal, the G1 image signal, the R1 image signal, the B2 image signal, the B3 image signal, and/or the G3 image signal may be performed by using the reliability calculated for every pixel in the specific region, and the reliability may be reflected on the correction process. In the case where in the correction process, the signal ratio ln(R2/G2), the signal ratio ln(B1/G2), and the signal ratio ln(B3/G3) are calculated by using the averages of the signal values (average signal values) of the B1 image signal, the G1 image signal, the R1 image signal, the B2 image signal, the B3 image signal, and/or the G3 image signal in the correction specific region, weighted averages after the average signal values are weighted may be used, and the signal ratio thereof may be calculated.

Second Embodiment

According to a second embodiment, as for the light source unit 20, a wide-range light source 400 that emits wide-range light such as a white LED, a xenon lamp, or a halogen light source is used instead of the LEDs 20a to 20e for the colors described according to the first embodiment, and the illumination light with which the photographic subject is illuminated is light emitted from the light source device 13 by using a combination of the wide-range light source 400 and a rotation filter 410. In the following description, components that differ from those according to the first embodiment in the endoscope system 10 will be described, and the description for common components is omitted.

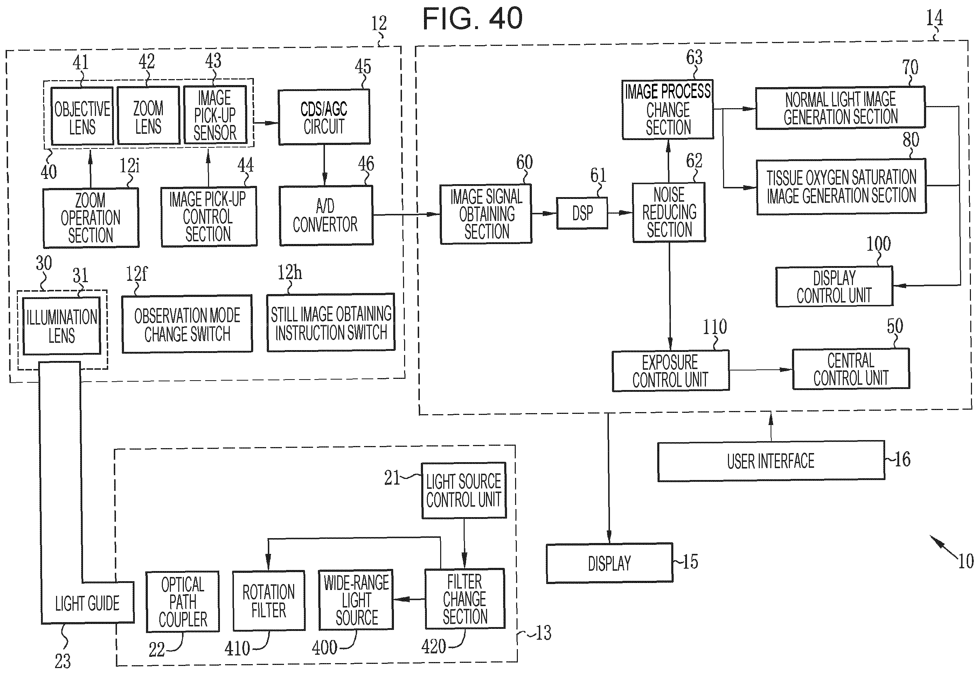

According to the second embodiment, as illustrated in Fig. 40, the light source device 13 of the endoscope system 10 includes the wide-range light source 400, the rotation filter 410, and a filter change section 420. The filter change section 420 is controlled by the light source control unit 21. The other structure is the same as that of the endoscope system 10 according to the first embodiment. According to the second embodiment, the image pick-up sensor 43 is preferably a monochrome image pick-up sensor.

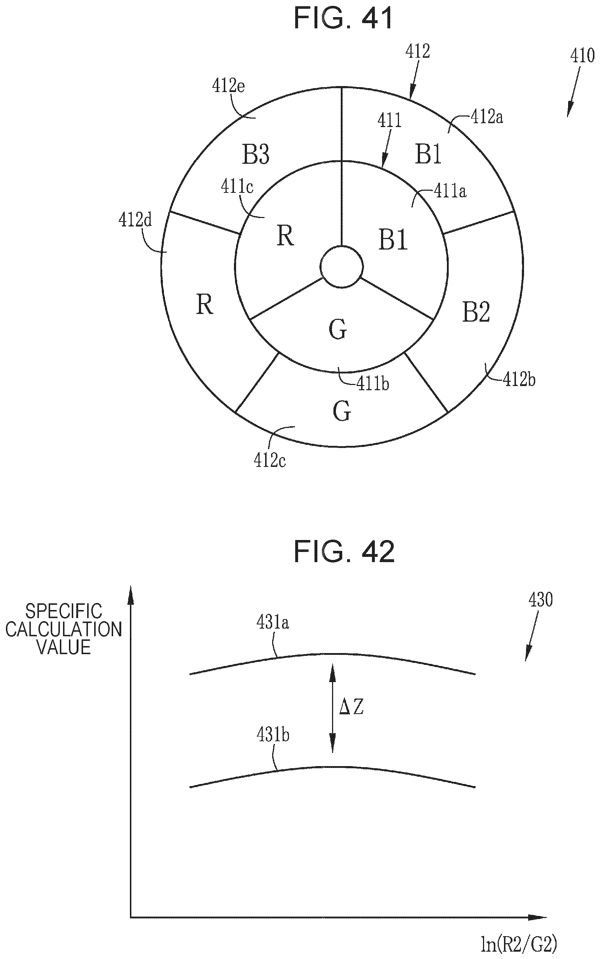

The wide-range light source 400 emits wide-range light that has the wavelength ranges of blue to red. An example of the wide-range light is white light. As illustrated in Fig. 41, the rotation filter 410 includes an inner filter 411 provided inside and an outer filter 412 provided outside. The filter change section 420 moves the rotation filter 410 in a radial direction. In the case of the normal mode, the filter change section 420 inserts the inner filter 411 of the rotation filter 410 into an optical path for the white light. In the case of the observation mode or the correction mode, the filter change section 420 inserts the outer filter 412 of the rotation filter 410 into the optical path for the white light.

As illustrated in Fig. 41, the inner filter 411 includes a B1 filter 411a that allows light in the wavelength ranges that the purple light V and the first blue light BS have in the white light to pass therethrough, a G filter 411b that allows light in the wavelength range that the green light G has in the white light to pass therethrough, and a R filter 411c that allows light in the wavelength range that the red light R has in the white light to pass therethrough in a circumferential direction. Accordingly, in the case of the normal mode, illumination light that has the wavelength ranges of the purple light V and the first blue light BS, illumination light that has the wavelength range of the green light G, and illumination light that has the wavelength range of the red light R are emitted from the light source device 13 so as to follow the rotation of the rotation filter 410.

As illustrated in Fig. 41, the outer filter 412 includes a B1 filter 412a that allows light in the wavelength range that the second blue light BL has in the white light to pass therethrough, a B2 filter 412b that allows light in the wavelength range that the first blue light BS has in the white light to pass therethrough, a G filter 412c that allows light in the wavelength range that the green light G has in the white light to pass therethrough, a R filter 412d that allows light in the wavelength range that the red light R has in the white light to pass therethrough, and a B3 filter 412e that allows blue green light BG in the white light to pass therethrough in the circumferential direction. The blue green light BG is light in the wavelength range that passes in the case where the correction illumination light passes through the B color filter BF of the image pick-up sensor 43 that is a color image pick-up sensor (see Fig. 11). Accordingly, in the case of the observation mode or the correction mode, illumination light that has the wavelength ranges of the first blue light BS, the second blue light BL, the green light G, the red light R, and the blue green light BG is emitted from the light source device 13 so as to follow the rotation of the rotation filter 410.

As for the endoscope system 10 in the normal mode, reflection light obtained in a manner in which the photographic subject is illuminated with the illumination light that has the wavelength ranges of the purple light V and the first blue light BS is imaged by a monochrome image pick-up sensor, and consequently, the Bc image signal is outputted. In addition, reflection light obtained in a manner in which the photographic subject is illuminated with the illumination light that has the wavelength range of the green light G is imaged by the monochrome image pick-up sensor, and consequently, the Gc image signal is outputted. In addition, reflection light obtained in a manner in which the photographic subject is illuminated with the illumination light that has the wavelength range of the red light R is imaged by the monochrome image pick-up sensor, and consequently, the Rc image signal is outputted. Subsequently, the normal light image is generated based on the Bc image signal, the Gc image signal, and the Rc image signal in the same manner as in the first embodiment.

In the case of the observation mode or the correction mode, reflection light obtained in a manner in which the photographic subject is illuminated with the illumination light that has the wavelength range of the first blue light BS is imaged by the monochrome image pick-up sensor, and consequently, the B1 image signal is outputted. In addition, reflection light obtained in a manner in which the photographic subject is illuminated with the illumination light that has the wavelength range of the green light G is imaged by the monochrome image pick-up sensor, and consequently, the G1 image signal is outputted. In addition, reflection light obtained in a manner in which the photographic subject is illuminated with the illumination light that has the wavelength range of the red light R is imaged by the monochrome image pick-up sensor, and consequently, the R1 image signal is outputted. In addition, reflection light obtained in a manner in which the photographic subject is illuminated with the illumination light that has the wavelength range of the second blue light BL is imaged by the monochrome image pick-up sensor, and consequently, the B2 image signal is outputted. In addition, reflection light obtained in a manner in which the photographic subject is illuminated with the illumination light that has the wavelength range of the blue green light BG is imaged by the monochrome image pick-up sensor, and consequently, the B3 image signal is outputted. Subsequently, the oxygen saturation image is generated based on the B1 image signal, the B2 image signal, the G2 image signal, the R2 image signal, and the B3 image signal in the same manner as in the first embodiment. In addition, the correction process is performed, and consequently, the correction oxygen saturation image is generated. According to the second embodiment, however, the signal ratio ln(B3/G1) obtained by standardizing the B3 image signal by using the G1 image signal is used instead of the signal ratio ln(B3/G3).

As for the correction process regarding the calculation of the correction oxygen saturation in the correction mode, the process of selecting the correction oxygen saturation calculation table 86b depending on the pigment value may be performed, and a calculated value correction process of adding or subtracting a correction value obtained from a specific calculation value to or from the oxygen saturation calculated by referring the oxygen saturation calculation table 84a may be performed, and the correction oxygen saturation may be calculated.

In the case where the calculated value correction process is performed, a two-dimensional coordinate system 430 illustrated in Fig. 42 is referred to, and consequently, the correction value used for correcting the oxygen saturation is calculated. The vertical axis of the two-dimensional coordinate system 430 represents a specific calculation value obtained based on the B1 image signal, the G1 image signal, the R1 image signal, the B2 image signal, and the B3 image signal, and the lateral axis thereof represents ln(R1/G1). The specific calculation value is defined as an expression A):

$$\text{Expression A)} \quad B2/G1 \times \cos\varphi - B3/G1 \times \sin\varphi.$$

As for the two-dimensional coordinate system 430, a reference line 431a that represents the distribution of predetermined reference baseline information and an actual measurement line 431b that represents the distribution of actual measurement baseline information obtained by imaging the actual object to be observed are illustrated. A difference value $\Delta Z$ between the reference line 431a and the actual measurement line 431b is calculated as the correction value. The reference baseline information is obtained with no specific pigment being present and is defined as information that does not depend on the oxygen saturation. Specifically, the reference baseline information is a value obtained by adjusting $\varphi$ such that the expression A) described above is constant even when the oxygen saturation changes.

Third Embodiment

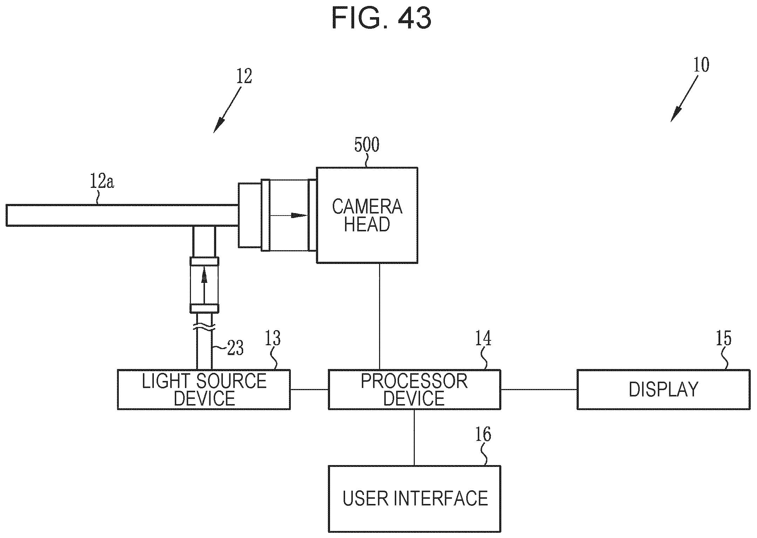

According to a third embodiment, as illustrated in Fig. 43, the endoscope 12 is a hard endoscope that includes a camera head 500 at the proximal end portion of the insertion section 12a. In the case where the endoscope 12 is a laparoscope, the insertion section 12a is inserted into the abdominal cavity of a person to be inspected. The camera head 500 includes the image pick-up optical system 40. According to the first embodiment and the second embodiment, the image pick-up optical system 40 that includes the objective lens 41 and the image pick-up sensor 43 is provided at a tip portion of the endoscope 12. According to the third embodiment, the camera head 500 includes the image pick-up sensor in the image pick-up optical system 40 not at the tip portion. The camera head 500 images the reflection light guided from the tip portion of the endoscope 12. An image signal of an image that is imaged by the camera head 500 is transmitted to the processor device 14. In Fig. 43, the angle knob 12e, the mode change switch 12f, the still image obtaining instruction switch 12h, and the zoom operation section 12i are omitted. In the following description, components that differ from those according to the first embodiment and the second embodiment in the endoscope system 10 will be described, and the description for common components is omitted.

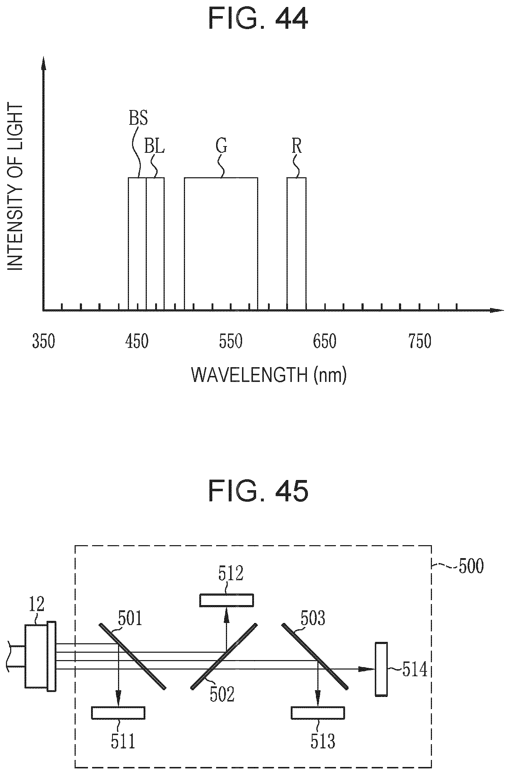

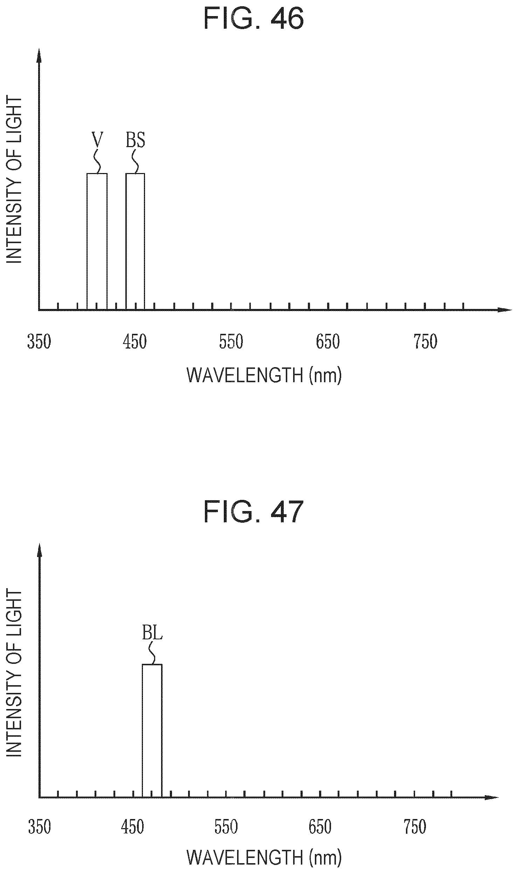

In the case of the normal mode, the light source device 13 emits the normal light that includes the purple light V, the first blue light BS, the green light G, and the red light R. In the case of the observation mode and correction mode, as illustrated in Fig. 44, the light source device 13 emits the illumination light (first mixed light) that is mixed light including the first blue light BS, the second blue light BL, the green light G, and the red light R. In Fig. 44 and in Fig. 46, Fig. 47, Fig. 48, and Fig. 49 described later, the illustration of the spectrum of the illumination light is simplified such that the intensity of light of the illumination light in the colors is constant for simplification, but the intensity of light of the illumination light in the colors is not limited to a constant value.

As illustrated in Fig. 45, the camera head 500 includes dichroic mirrors 501, 502, and 503 and image pick-up sensors 511, 512, 513, and 514 that are monochrome image pick-up sensors. The dichroic mirror 501 reflects light in the wavelength ranges that the purple light V and the first blue light BS have in the reflection light from the photographic subject and allows light in the wavelength ranges that the second blue light BL, the green light G, and the red light R have to pass therethrough. As illustrated in Fig. 46, the light that is reflected by the dichroic mirror 501 and that enters the image pick-up sensor 511 has the wavelength range of the purple light V or the first blue light BS. The image pick-up sensor 511 outputs the Bc image signal in the normal mode and outputs the oxygen saturation or the B1 image signal in the correction mode.

The dichroic mirror 502 reflects light in the wavelength range that the second blue light BL has in the light that passes through the dichroic mirror 501 and allows light in the wavelength ranges that the green light G and the red light R have to pass therethrough. As illustrated in Fig. 47, the light that is reflected by the dichroic mirror 502 and that enters the image pick-up sensor 512 has the wavelength range of the second blue light BL. The image pick-up sensor 512 stops outputting the image signals in the normal mode and outputs the B2 image signal in the observation mode or the correction mode.

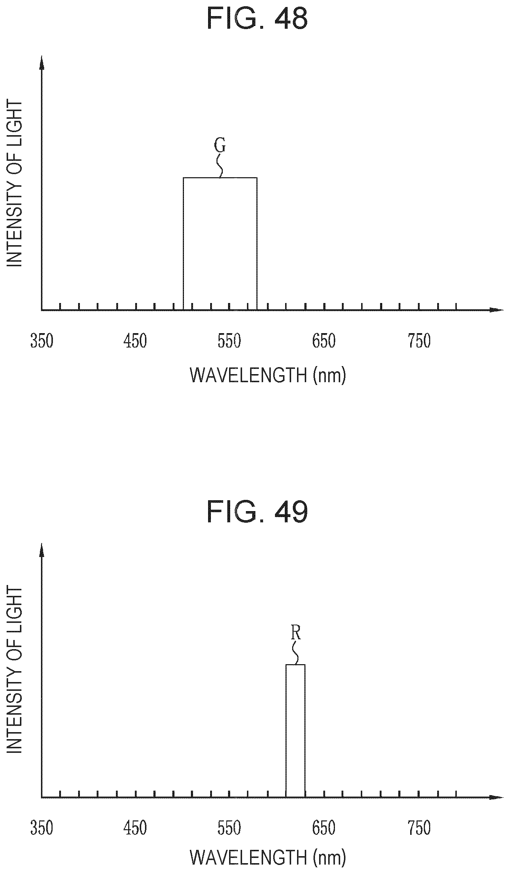

The dichroic mirror 503 reflects light in the wavelength range that the green light G has in the light that passes through the dichroic mirror 502 and allows light in the wavelength range that the red light R has to pass therethrough. As illustrated in Fig. 48, the light that is reflected by the dichroic mirror 503 and that enters the image pick-up sensor 513 has the wavelength range of the green light G. The image pick-up sensor 513 outputs the Gc image signal in the normal mode and outputs the oxygen saturation or the G1 image signal in the correction mode.

As illustrated in Fig. 49, the light that passes through the dichroic mirror 503 and that enters the image pick-up sensor 514 has the wavelength range of the red light R. The image pick-up sensor 514 outputs the Rc image signal in the normal mode and outputs the R1 image signal in the observation mode or the correction mode.

That is, according to the third embodiment, the Bc image signal, the Gc image signal, and the Rc image signal are outputted from the camera head in the normal mode, and the B1 image signal, the G1 image signal, the R1 image signal, and the B2 image signal are outputted therefrom in the observation mode or the correction mode. The image signals that are outputted from the image pick-up sensors 511, 512, 513, and 514 are transmitted to the processor device 14.

Fourth Embodiment

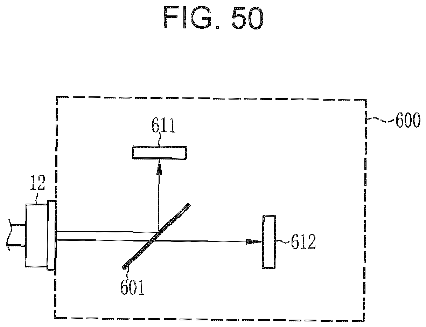

According to a fourth embodiment, the endoscope 12 is a hard endoscope that includes a camera head at the proximal end portion of the insertion section 12a as in the third embodiment. In the following description, components that differ from those according to the first embodiment, the second embodiment, and the third embodiment will be described, and the description for common components is omitted. According to the fourth embodiment, a camera head 600 illustrated in Fig. 50 is included instead of the camera head 500 according to the third embodiment.

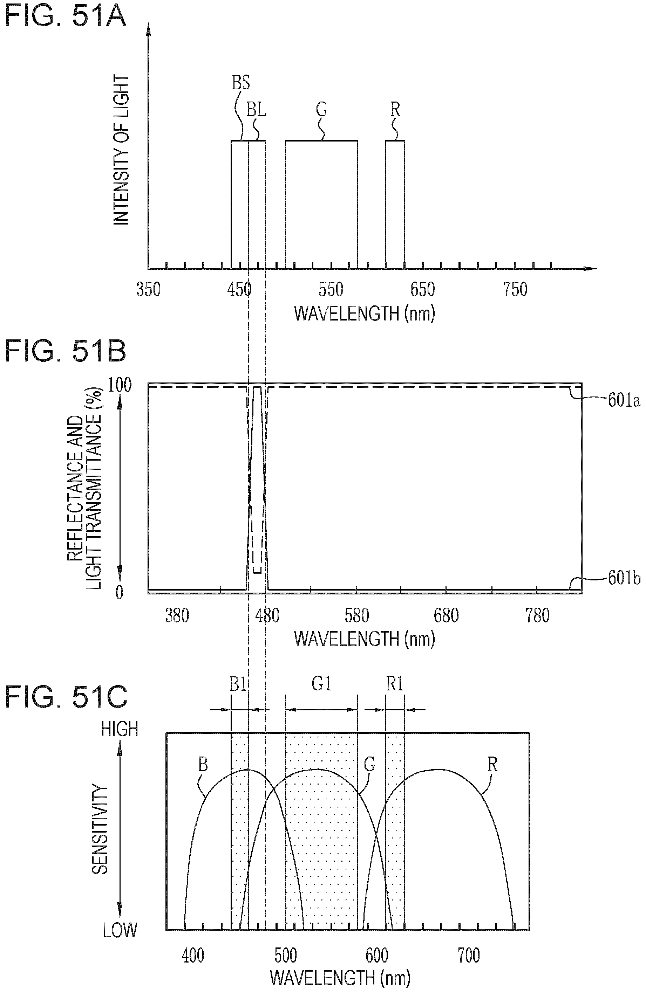

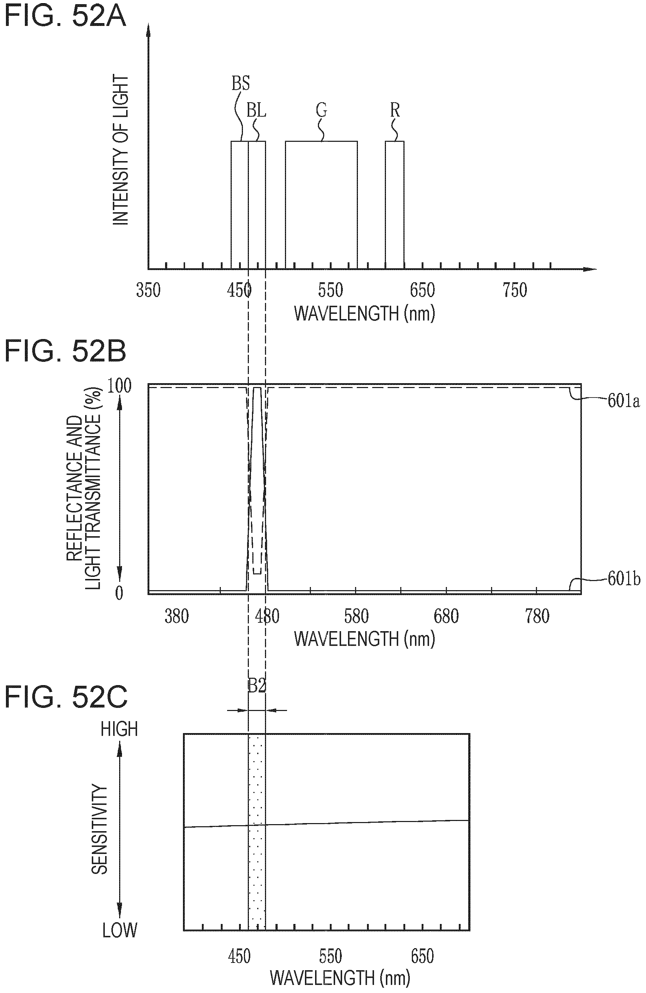

As illustrated in Fig. 50, the camera head 600 includes a dichroic mirror 601 and image pick-up sensors 611 and 612 The dichroic mirror 601 reflects light in the wavelength ranges that the purple light V, the first blue light BS, the green light G, and the red light R have in the reflection light from the photographic subject and allows light in the wavelength range that the second blue light BL has to pass therethrough (see Fig. 51B and Fig. 52B described later).

The image pick-up sensor 611 that receives the light reflected by the dichroic mirror 601 is a color image pick-up sensor in which each B pixel includes the B color filter BF, each G pixel includes the G color filter GF, and each R pixel includes the R color filter RF. The image pick-up sensor 612 that receives the light that passes through the dichroic mirror 601 is a monochrome image pick-up sensor.

In the case of the normal mode, the white light is emitted from the light source device 13, the image pick-up sensor 611 that is a color image pick-up sensor receives the reflection light reflected by the dichroic mirror 601 from the photographic subject, and consequently, the Bc image signal, the Gc image signal, and the Rc image signal are outputted from the image pick-up sensor 611. In the normal mode, the image pick-up sensor 612 that is a monochrome image pick-up sensor stops outputting the image signals.

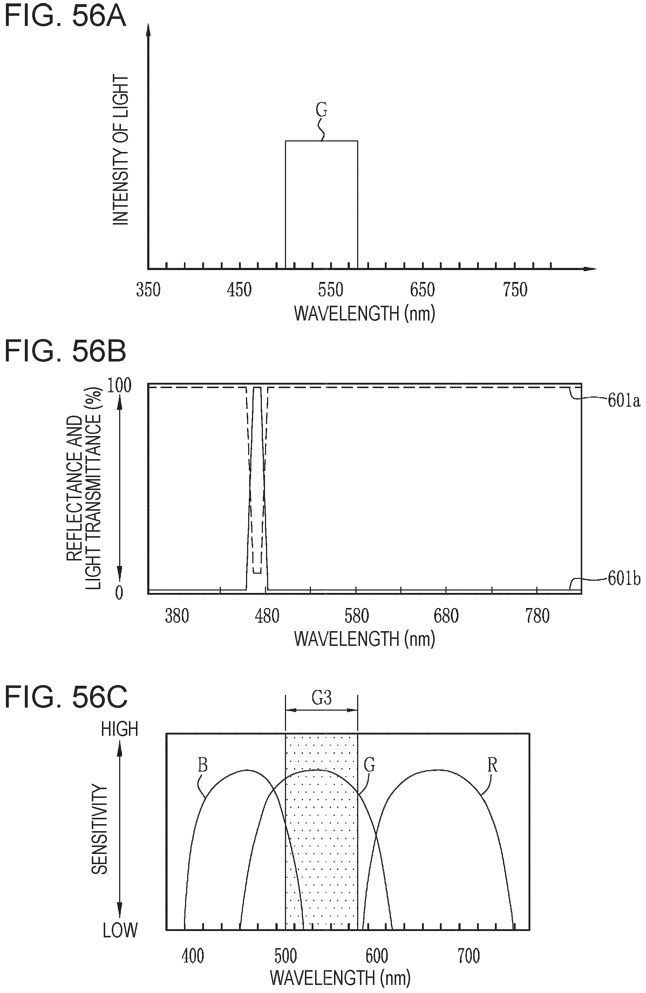

In the case of the observation mode, as illustrated in Fig. 51A, illumination light (referred to below as second mixed light) that includes the first blue light BS, the second blue light BL, the green light G, and the red light R is emitted from the light source device 13. The dichroic mirror 601 separates the reflection light from the photographic subject illuminated with the second mixed light by reflecting the light and allowing the light to pass therethrough. Fig. 51B illustrates a relationship between the reflectance (a dashed line 601a) of the light that enters the dichroic mirror 601 and the wavelength of light and between the light transmittance (a solid line 601b) and the wavelength of light. In Fig. 51A and in Fig. 52A, and Fig. 56A described later, the illustration of the spectrum of the illumination light is simplified such that the intensity of light of the illumination light in the colors is constant for simplification, but the intensity of light of the illumination light in the colors is not limited to a constant value.

The light reflected by the dichroic mirror 601 in the reflection light from the photographic subject illuminated with the second mixed light is received by the image pick-up sensor 611 that is a color image pick-up sensor. The sensitivity of a B pixel B, a G pixel G, and a R pixel R of the image pick-up sensor 611 and the wavelength of light have a relationship illustrated in Fig. 51C. For this reason, the B pixel B of the image pick-up sensor 611 senses light in a wavelength range B1 that the first blue light BS has and consequently outputs the B1 image signal. The G pixel G of the image pick-up sensor 611 senses light in a wavelength range G1 that the green light G has and consequently outputs the G1 image signal. The R pixel R of the image pick-up sensor 611 senses light in a wavelength range R1 that the red light R has and outputs the R1 image signal.

The light that passes through the dichroic mirror 601 in the reflection light from the photographic subject illuminated with the second mixed light is received by the image pick-up sensor 612 that is a monochrome image pick-up sensor. The sensitivity of the image pick-up sensor 612 and the wavelength of light have a relationship illustrated in Fig. 52C. For this reason, the image pick-up sensor 612 senses light in a wavelength range B2 that the second blue light BL has that passes through the dichroic mirror 601 and consequently outputs the B2 image signal. Fig. 52A illustrates the wavelength range of light included in the second mixed light as in Fig. 51A. Fig. 52B illustrates a relationship between the reflectance (the dashed line 601a) of light that enters the dichroic mirror 601 and the wavelength of light and between the light transmittance (the solid line 601b) and the wavelength of light as in Fig. 51B.

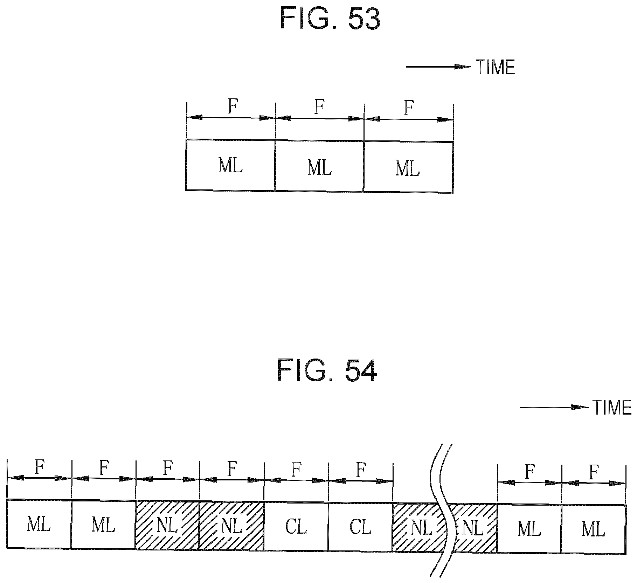

In the observation mode according to the fourth embodiment, as illustrated in Fig. 53, a light emission pattern is repeated such that second mixed light ML is emitted once for every frame F. Accordingly, in the observation mode according to the fourth embodiment, the B1 image signal, the G1 image signal, and the R1 image signal are outputted from the image pick-up sensor 611 that is a color image pick-up sensor for every frame, and the B2 image signal is outputted from the image pick-up sensor 612 that is a monochrome image pick-up sensor. The B1 image signal, the G1 image signal, the R1 image signal, and the B2 image signal that are outputted from the image pick-up sensor 611 or the image pick-up sensor 612 in the observation mode are transmitted to the processor device 14. A method of calculating the oxygen saturation in the observation mode is the same as that according to the first embodiment.

In the case where the second mixed light ML is emitted in the correction mode according to the fourth embodiment, as illustrated in Fig. 54, the light emission pattern is such that an instruction for changing light emission is inputted by a user operation, the correction illumination light CL is emitted for two frames F through a non-light-emission state for the two frames F, and the second mixed light ML is emitted for two frames F through the non-light-emission state NL for multiple frames. The frames in the non-light-emission state NL correspond to periods for switching between the second mixed light ML and the correction illumination light during which no illumination light is emitted. The instruction for changing light emission may be inputted by operating an illumination light change switch (not illustrated) provided in the endoscope 12 or the user interface or may be inputted by a toggle operation on the mode change switch 12f.

In the correction mode, the B1 image signal, the G1 image signal, and the R1 image signal are outputted from the image pick-up sensor 611, and the B2 image signal is outputted from the image pick-up sensor 612 in the frames in which the second mixed light ML is emitted as in the observation mode.

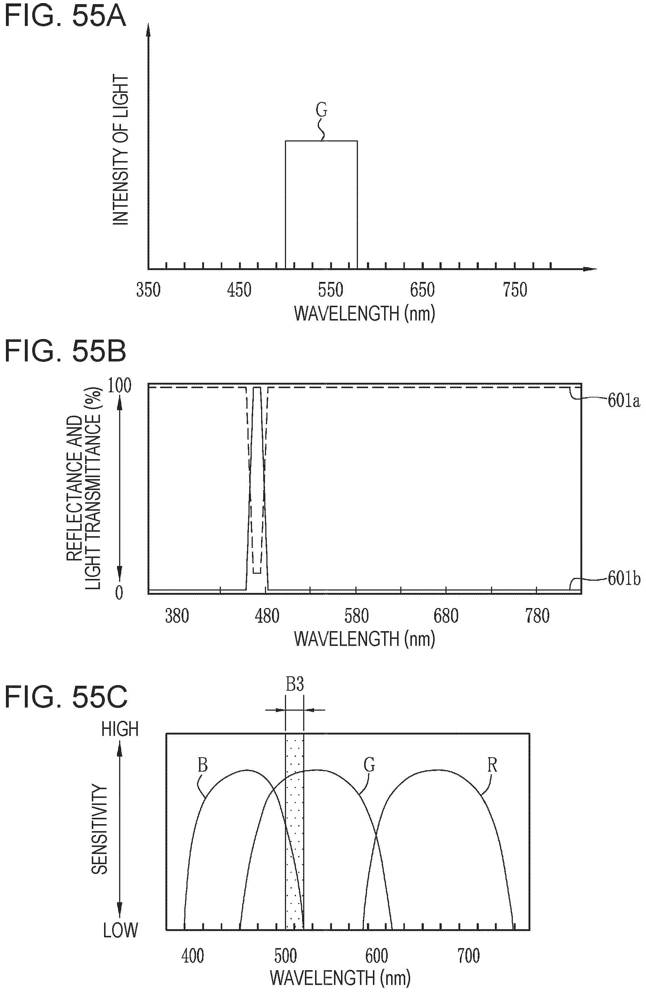

In the correction mode, the correction illumination light (the correction illumination light) that includes the green light G illustrated in Fig. 55A is emitted from the light source device 13 in the frames in which the correction illumination light CL is emitted. The image pick-up sensor 611 that is a color image pick-up sensor receives the reflection light (see Fig. 55B) reflected by the dichroic mirror 601 from the photographic subject. As a result, as illustrated in Fig. 55C, the B pixel B of the image pick-up sensor 611 senses light in a wavelength range B3 that the green light G has and consequently outputs the B3 image signal. In addition, as illustrated in Fig. 56C, the G pixel G of the image pick-up sensor 611 senses light in a wavelength range G3 that the green light G has and consequently outputs the G3 image signal. The R pixel R of the image pick-up sensor 611 senses light in the wavelength range that the green light G has and consequently outputs the R3 image signal (not illustrated).

Fig. 56A illustrates the wavelength range of light included in the correction illumination light as in Fig. 55A. In Fig.

55B and Fig. 56B, a relationship between the reflectance (the dashed line 601a) of light that enters the dichroic mirror 601 and the wavelength of light and between the light transmittance (the solid line 601b) and the wavelength of light is illustrated as in Fig. 51B. In Fig. 55C and Fig. 56C, a relationship between the sensitivity of the B pixel B, the G pixel G, and the R pixel R of the image pick-up sensor 611 and the wavelength of light as in Fig. 51C.

The B1 image signal, the G1 image signal, and the R1 image signal, the B2 image signal, the B3 image signal, and the G3 image signal that are outputted from the image pick-up sensor 611 or the image pick-up sensor 612 in the correction mode are transmitted to the processor device 14. The calculation of the reliability according to the fourth embodiment will now be described.

In an example described according to the first embodiment, the correction image 200 is displayed, and regarding the correction specific region 201 included in the correction image 200, the reliability for every pixel included in the correction specific region 201 is calculated. According to the fourth embodiment, the reliability is calculated for every correction region by using the images (the substantially white light image and the second blue light image) obtained in each frame in which the second mixed light ML is emitted and the image (the correction illumination light image) obtained in each frame in which the correction illumination light CL is emitted unlike the first embodiment. The correction region corresponds to the correction specific region according to the first embodiment. The term "correction region" is used as a term that represents a "set of multiple divided small regions" or a "small region itself (a N-th correction region, N is a natural number of 1 or more)" as described later.

The substantially white light image is an endoscope image generated by using the B1 image signal, the G1 image signal, and the R1 image signal outputted in each frame in which the second mixed light ML is emitted. The second blue light image is an endoscope image generated by using the B2 image signal outputted in each frame in which the second mixed light ML is emitted. The correction illumination light image is an endoscope image generated by using the B3 image signal, the G3 image signal, and the R3 image signal outputted in each frame in which the correction illumination light CL is emitted. The substantially white light image, the second blue light image, and the correction illumination light image are generated by the DSP 61.

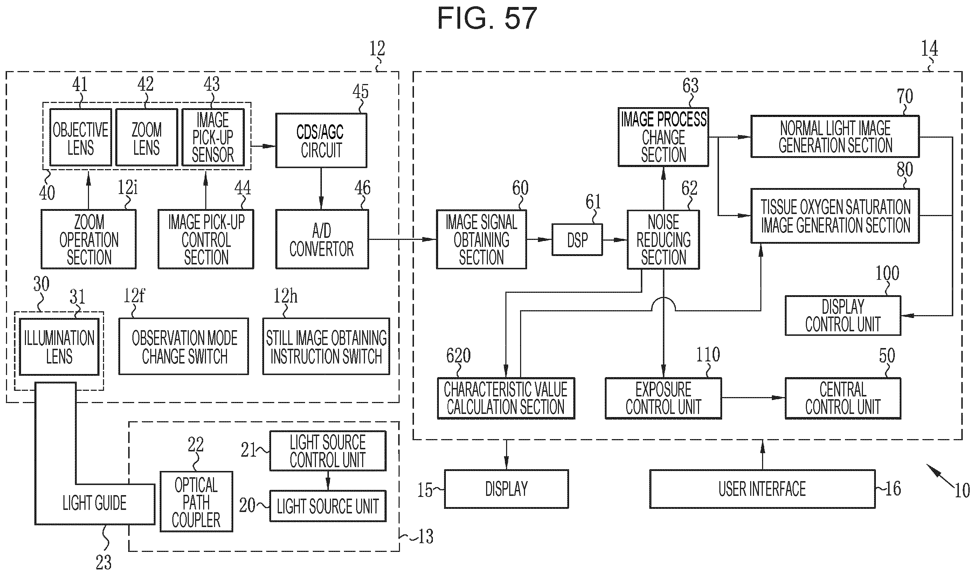

The substantially white light image, the second blue light image, and the correction illumination light image are transmitted to a characteristic value calculation section 620 of the processor device 14 illustrated in Fig. 57. The characteristic value calculation section 620 preferably includes a processor that differs from the central control unit 50 in the processor device 14. For example, the characteristic value calculation section 620 preferably includes a field programmable gate array (FPGA).

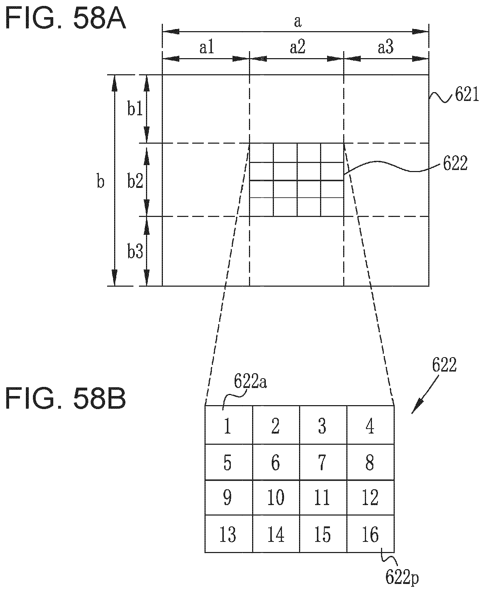

The characteristic value calculation section 620 calculates region characteristic values regarding multiple correction regions illustrated in Fig. 58A and Fig. 58B in the substantially white light image, the second blue light image, and the correction illumination light image. The region characteristic values will be described later. As illustrated in Fig. 58A and Fig. 58B, correction regions 622 are multiple divided small regions in a substantially white light image 621. In an example illustrated in Fig. 58A and Fig. 58B, the correction regions 622 are regions obtained such that a lateral length a of the substantially white light image 621 is equally divided into three of a1, a2, and a3, a vertical length b is equally divided into three of b1, b2, and b3, and a region at which the column of a2 and the row of b2 intersect with each other is divided into 16 regions (see Fig. 58A). The positions of the correction regions 622 and the number of times of the division of the region into the correction regions 622 are not limited thereto. For example, the correction regions 622 may be equally divided 9 regions or equally divided 25 regions.

Fig. 58B is an enlarged view of one of the correction regions 622 illustrated in Fig. 58A. As illustrated in Fig. 58B, the correction region 622 corresponds to the 16 divided regions ranging from a first correction region 622a to a sixteenth correction region 622p. In Fig. 58B, the 16 regions ranging from the first correction region 622a to the sixteenth correction region 622p into which the correction region 622 is divided are designated by using numbers from 1 to 16.

The characteristic value calculation section 620 determines whether pixels in the correction regions are effective pixels regarding the correction regions such as the N-th correction region (in the case of the example illustrated in Fig. 58A and Fig. 58B, the first correction region to the sixteenth correction region). Whether the pixels are effective pixels is determined in a manner in which the lower limit and upper limit of channel threshold values regarding channels (the B channel, the G channel, and the R channel) of the pixels are provided.

As for the B channel, a B channel lower limit threshold value and a B channel upper limit threshold value are provided. As for the G channel, a G channel lower limit threshold value and a G channel upper limit threshold value are provided. As for the R channel, a R channel lower limit threshold value and a R channel upper limit threshold value are provided.

As for the substantially white light image, the second blue light image, and the correction illumination light image, in the case where the pixel values of the channels for all colors of each pixel in the correction regions are equal to or more than the channel lower limit threshold values for the colors and less than the channel upper limit threshold values, the characteristic value calculation section 620 determines that the pixel is an effective pixel.

As for the substantially white light image and the correction illumination light image, in the case where the pixel value of the B channel of each pixel that forms the substantially white light image and the correction illumination light image is equal to or more than the B channel lower limit threshold value and less than the B channel upper limit threshold value, the pixel value of the G channel is equal to or more than the G channel lower limit threshold value and less than the G channel upper limit threshold value, and the pixel value of the R channel is equal to or more than the R channel lower limit threshold value and less than the R channel upper limit threshold value, it is determined that the pixel is an effective pixel.

As for the second blue light image, in the case where the pixel value of each pixel is equal to or more than a monochrome image channel lower limit value and less than a monochrome image channel upper limit value, it is determined that the pixel is an effective pixel.

Subsequently, the characteristic value calculation section 620 calculates the region characteristic values regarding the correction regions ranging from the first correction region to the sixteenth correction region. Examples of the region characteristic values include the number of the effective pixels, the sum of the pixel values of the effective pixels, the sum of the squares of the pixel values of the effective pixels, and the variance of the pixel values of the effective pixels.

That is, the characteristic value calculation section 620 calculates the region characteristic values regarding the correction regions of the channels in the substantially white light image. In addition, the region characteristic values regarding the correction regions of the channels in the correction illumination light image are calculated. In addition, the region characteristic values regarding the correction regions in the second blue light image are calculated. The region characteristic values of the correction regions of the channels in the endoscope image that are calculated by the characteristic value calculation section 620 are transmitted to the reliability calculation section 220 of the tissue oxygen saturation image generation section 80.

According to the fourth embodiment, the reliability calculation section 220 calculates the reliability for determining the degree of the influence of the disturbance in the correction regions. The reliability calculation section 220 calculates the pigment value for determining the degree of movement of the endoscope 12. The degree of movement of the endoscope 12 is a degree for determining whether the endoscope 12 is moved while the illumination light in the correction mode according to the fourth embodiment is changed (that is, the non-light-emission state NL). In some cases where the endoscope 12 moves in the non-light-emission state NL, the object to be observed that is seen in the endoscope image also moves, and accordingly, the correction process is not appropriately performed. For this reason, the degree of movement of the endoscope 12 in each correction region is calculated, and consequently, a determination regarding the movement of the endoscope 12 is made by using the degree of movement of the endoscope 12 as described later. In the case where the degree of movement of the endoscope 12 is high, a notification can be given to inhibit the user from moving the endoscope 12.

According to the fourth embodiment, the correction determination section 230 of the tissue oxygen saturation image generation section 80 determines the degree of the influence of the disturbance by using the reliability and/or determines the degree of movement of the endoscope 12 by using the pigment value.

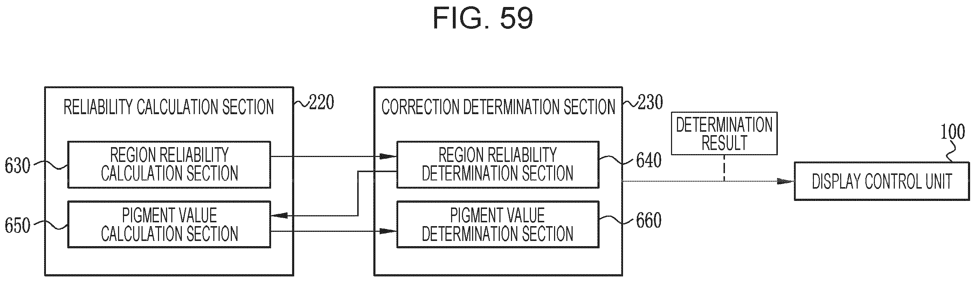

A method of determining the degree of the influence of the disturbance and a method of determining the degree of movement of the endoscope 12 according to the fourth embodiment will now be described. According to the fourth embodiment, as illustrated in Fig. 59, the reliability calculation section 220 includes a region reliability calculation section 630 and a pigment value calculation section 650. The correction determination section 230 includes a region reliability determination section 640 and a pigment value determination section 660.

The region reliability calculation section 630 of the reliability calculation section 220 calculates region reliability by using the region characteristic values of the correction regions of the channels in the substantially white light image, the second blue light image, and the correction illumination light image that are generated from the image signals that are outputted in the frames. Examples of the region reliability include the average of the pixel values in the correction regions, the standard deviation of the pixel values in the correction regions, the ratio of the effective pixels in the correction regions, reliability regarding the brightness in the correction regions, reliability due to the degree of bleeding included in the correction regions, and reliability due to the degree of fat included in the correction regions. The region reliability corresponds to an aspect of the "reliability" according to the first embodiment.

The average of the pixel values in the correction regions is calculated by using the number of the pixels in the correction regions and the pixel values of the effective pixels in the correction regions. The standard deviation of the pixel values in the correction regions is calculated by using the number of the pixels in the correction regions and the variance of the pixel values of the effective pixels. The ratio of the effective pixels in the correction regions is calculated by using the number of the pixels in the correction regions and the number of the effective pixels. The reliability regarding the brightness in the correction regions is calculated by applying the average of the G1 image signals in the correction regions (that is, the pixel value in the correction region of the G channel in the substantially white light image) to the first reliability calculation table 221 where the lateral axis of the first reliability calculation table 221 (see Fig. 33) illustrated in Fig. 60 represents the signal values of the G1 image signals. The average of the G1 image signals is preferably the average of the brightness values that are obtained by performing the conversion process by using the G1 image signals.

The reliability due to the degree of bleeding included in the correction regions is calculated in a manner in which a region average signal ratio ln(R1/G1) and a region average signal ratio ln(B1/G1) are calculated by using the average of the B1 image signals in the correction regions in the substantially white light image, the average of the G1 image signals, and the average of the R1 image signals (that is, the average of the pixel values in the correction regions of the channels for the colors in the substantially white light image), and the signal ratio thereof is applied to the second reliability calculation table 222 (see Fig. 34).

The reliability due to the degree of fat included in the correction regions is calculated in a manner in which the region average signal ratio ln(R1/G1) and the region average signal ratio ln(B2/G1) are calculated by using the average of the G1 image signals and the average of the R1 image signals in the correction regions in the substantially white light image (that is, the average of the pixel values in the correction regions of the G channel and the R channel in the substantially white light image) and the average of the B2 image signals in the correction regions in the second blue light image, and the signal ratio thereof is applied to the third reliability calculation table 223 where the vertical axis of the third reliability calculation table 223 (see Fig. 35) illustrated in Fig. 61 represents the signal ratio ln(B2/G1). The region reliability calculated by the region reliability calculation section 630 is transmitted to the region reliability determination section 640 of the correction determination section 230 (see Fig. 59).

A method of determining the degree of the influence of the disturbance and giving a notification according to the fourth embodiment will now be described. The region reliability determination section 640 outputs the determination result that represents whether the correction regions in the substantially white light image, the second blue light image, and the correction illumination light image are highly reliable correction regions or insufficiently reliable correction regions by using a predetermined region reliability determination threshold value.

The region reliability determination threshold value may be set depending on the kind of the region reliability. For example, in the case where a first region reliability determination threshold value is set for the "average of the pixel values in a correction region", and the "average of the pixel values in the correction region" is equal to or more than the first region reliability determination threshold value, the correction region is determined to be the "highly reliable correction region". In the case where the "average of the pixel values in a correction region" is less than the first region reliability determination threshold value, the correction region is determined to be the "insufficiently reliable correction region".

Similarly, a second region reliability determination threshold value is set for the "standard deviation of the pixel values in a correction region", a third region reliability determination threshold value is set for the "ratio of the effective pixels in a correction region", a fourth region reliability determination threshold value is set for the "reliability regarding the brightness in a correction region", and a fifth region reliability determination threshold value is set for the "reliability due to the degree of fat included in a correction region", and the determination result is outputted.

The region reliability determination section 640 determines the reliability in the substantially white light image, the second blue light image, and the correction illumination light image by using the determination result that represents whether the correction regions are the highly reliable correction regions or the insufficiently reliable correction regions. In this case, an image determination result is outputted depending on the number of the correction regions that are determined as the "insufficiently reliable correction regions" among all of the correction regions in the substantially white light image, the second blue light image, and the correction illumination light image. For example, a first image determination result output threshold value is set in advance, and consequently, the image determination result is outputted.

For example, in the case where the first image determination result output threshold value is "10", and in the case where the number of the correction regions in the substantially white light image is 16, when the number of the insufficiently reliable correction regions in the substantially white light image is 10 or more, the reliability in the correction regions as a whole is high, that is, the influence of the disturbance is weak, and the image determination result that represents that the correction process can be appropriately performed is outputted. When the number of the insufficiently reliable correction regions in the substantially white light image is less than 10, the reliability in the correction regions as a whole is weak, that is, the influence of the disturbance is exerted, and the image determination result that represents that the correction process cannot be appropriately performed is outputted.

As for all of the substantially white light image, the second blue light image, and the correction illumination light image, or as for some of the substantially white light image, the second blue light image, and the correction illumination light image, the region reliability may be calculated, and the image determination result may be outputted. The reason is that a calculation process is quickly performed.

The image determination result outputted by the region reliability determination section 640 is transmitted to the display control unit 100. The display control unit 100 preferably changes the display form on the display 15 depending on the image determination result. For example, in the case where the image determination result that represents the "reliability in the correction regions as a whole is low" is outputted, the display control unit 100 causes the display 15 to display a message that represents that the correction process can be appropriately performed (see Fig. 38). In the case where the image determination result that represents that the "reliability in the correction regions as a whole is high" is outputted, the display 15 displays a warning message that represents that "PLEASE OPERATE ENDOSCOPE FOR CORRECTION PROCESS" (see Fig. 39). The message may be superimposed and displayed on the substantially white light image displayed on the display.

The region reliability determination section 640 may calculate image determination average reliability by using the correction regions in the substantially white light image, the second blue light image, and the correction illumination light image. The image determination average reliability is calculated, for example, in a manner in which the sum of the reliability in all of the correction regions in the substantially white light image is divided by the number of the correction regions. In this case, the region reliability determination section 640 sets a second image determination result output threshold value for the image determination average reliability in advance and outputs the image determination result that represents that the "reliability in the correction regions as a whole is high" when the image determination average reliability is equal to or more than the second image determination result output threshold value. When the image determination average reliability is less than the second image determination result output threshold value, the image determination result that represents that the "reliability in the correction regions as a whole is low" is outputted. Also in this case, the display control unit 100 preferably changes the display form on the display 15 depending on the image determination result.

A method of determining the degree of movement of the endoscope 12 and giving a notification according to the fourth embodiment will now be described. In this case, the result of determination that is outputted by the region reliability determination section 640 and that represents whether the correction regions are the highly reliable correction regions or the insufficiently reliable correction regions in the substantially white light image, the second blue light image, and the correction illumination light image is transmitted to the pigment value calculation section 650 (see Fig. 59).

The pigment value calculation section 650 preferably performs an exclusion process of excluding a correction region that is determined as the "insufficiently reliable correction region" among the correction regions in the substantially white light image, the second blue light image, and the correction illumination light image from the calculation of the pigment value.

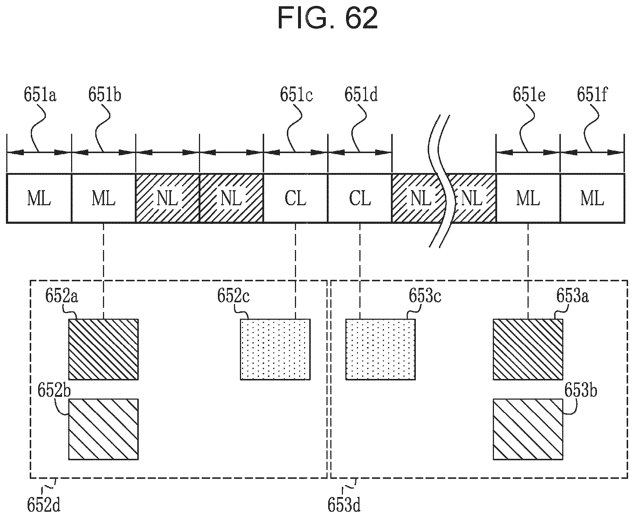

The pigment value calculation section 650 preferably determines some of the substantially white light image, the second blue light image, and the correction illumination light image as images on which the exclusion process is performed. Specific examples include a substantially white light image 652a and a second blue light image 652b that are generated based on the image signals outputted in a frame 651b, a correction illumination light image 652c that is generated based on the image signals outputted in a frame 651c, a correction illumination light image 653c that is generated based on the image signals outputted in a frame 651d, and a substantially white light image 653a and a second blue light image 653b that are generated based on the image signals outputted in a frame 651e among a frame 651a, the frames 651b, 651c, 651d, and 651e, and a frame 651f in which the second mixed light ML or the correction illumination light CL is emitted in the light emission pattern in the correction mode according to the fourth embodiment as illustrated in Fig. 62 (see Fig. 54).

Among these, the substantially white light image 652a, the second blue light image 652b, and the correction illumination light image 652c are referred to as a first image set 652d. The substantially white light image 653a, the second blue light image 653b, and the correction illumination light image 653c are referred to as a second image set 653d. The pigment value calculation section 650 preferably performs the exclusion process on the images that are included in the first image set 652d and the images that are included in the second image set 653d. A correction region that is determined as the "highly reliable correction region" for which no exclusion process is performed is referred to below as an effective region. A correction region that is determined as the "insufficiently reliable correction region" for which the exclusion process is performed is referred to as an exclusion region.

The pigment value calculation section 650 performs the exclusion process such that the position of the exclusion region and the position of the effective region that is included in the first image set 652d match each other among the substantially white light image 652a, the second blue light image 652b, and the correction illumination light image 652c.

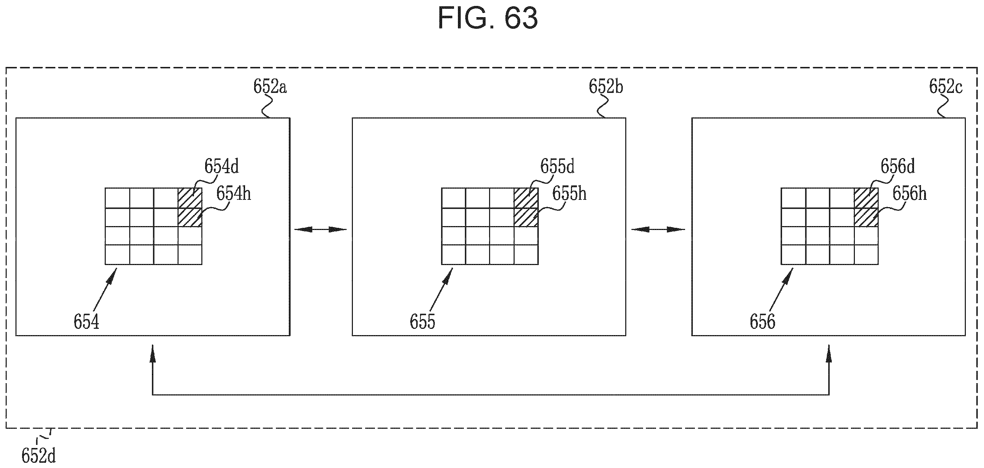

Specifically, as illustrated in for example, Fig. 63, the exclusion regions in the substantially white light image 652a are correction regions 654d and 654h, and correction regions other than the correction regions 654d and 654h in a correction region 654 as a whole are the effective regions. Similarly, the exclusion regions in the second blue light image 652b are correction regions 655d and 655h, and correction regions other than the correction regions 655d and 655h in a correction region 655 as a whole are the effective regions. The exclusion regions in the correction illumination light image 652c are correction regions 656d and 656h, and correction regions other than the correction regions 656d and 656h in a correction region 656 as a whole are the effective regions. The exclusion process is thus performed, and consequently, the positions of the effective regions can match each other among the images that are included in the first image set 652d.

A method of the exclusion process will be described. The pigment value calculation section 650 performs the exclusion process on the image sets by using predetermined exclusion process threshold values. The exclusion process threshold values are set as multiple values for calculating the region reliability of the correction regions such that the region reliability is evaluated in five stages of "1" to "5". The exclusion process threshold values are preferably set depending on the kind of the region reliability.

The pigment value calculation section 650 first calculates region determination reliability in the five stages regarding the correction regions in the substantially white light image, the second blue light image, and the correction illumination light image that are included in the image sets. Subsequently, among the corresponding correction regions in the substantially white light image, the second blue light image, and the correction illumination light image that are included in the image sets, a correction region that has the minimum region determination reliability is selected.

Subsequently, the region reliability determination threshold value for determining the "highly reliable correction region" or the "insufficiently reliable correction region" is applied to the correction region that has the minimum region determination reliability, and a correction region that is determined as the "insufficiently reliable correction region" is determined as the exclusion region. In this case, all of the correction regions in the substantially white light image, the second blue light image, and the correction illumination light image that correspond to the correction region that is determined as the "insufficiently reliable correction region" are determined as the exclusion regions.

The pigment value calculation section 650 calculates the pigment value from the first image set 652d and the second image set 653d. The calculation of the pigment value will now be described in detail. In the case where the pigment value of the first image set 652d is calculated, the region average signal ratio ln(R1/G1), the region average signal ratio ln(B2/G1), and the region average signal ratio ln(B3/G3) are calculated for every effective region, based on the signal values in the effective regions that are located at corresponding positions in the substantially white light image 652a, the second blue light image 652b, and the correction illumination light image 652c.

The region average signal ratio ln(R1/G1) is calculated by using the average of the R1 image signals in the effective regions in the substantially white light image 652a and the average (that is, the average of the pixel values in the effective regions of the R channel in the substantially white light image and the average of the pixel values in the effective regions of the G channel) of the G1 image signals in the effective regions.

The region average signal ratio ln(B2/G1) is calculated by using the average (that is, the average of the pixel values in the effective regions in the second blue light image) of the B1 image signals in the effective regions in the second blue light image 652b and the average of the G1 image signals in the effective regions in the substantially white light image 652a.

The region average signal ratio ln(B3/G3) is calculated by using the average of the B3 image signals in the effective regions in the correction illumination light image 652c and the average (that is, the average of the pixel values in the effective regions of the B channel in the correction illumination light image and the average of the pixel values in the effective regions of the G channel) of the G3 image signals in the effective regions.

The pigment value calculation section 650 calculates the region average signal ratio ln(R1/G1), the region average signal ratio ln(B2/G1), and the region average signal ratio ln(B3/G3) regarding the corresponding effective regions of the first image set 652d by referring the pigment value calculation table 86a (see Fig. 17). In the pigment value calculation table 86a, curved surfaces CV0 to CV4 distribute depending on the concentration of the yellow pigment in a three-dimensional coordinate system where an X-axis represents the signal ratio ln(R1/G1), a Y-axis represents the signal ratio ln(B2/G1), and the Z-axis represents the signal ratio ln(B3/G3).

The pigment value calculation section 650 refers the pigment value calculation table 86a that is a three-dimensional coordinate system and calculates the pigment value by using the curved surface that overlaps coordinates (X3, Y3, Z3) = (the region average signal ratio ln(R1/G1), the region average signal ratio ln(B2/G1), and the region average signal ratio ln(B3/G3)) or that has the minimum distance among the curved surfaces CV0 to CV4. The curved surfaces CV0 to CV4 represent that the pigment value is "0" to "4". For example, in the case where the coordinates (X3, Y3, Z3) overlap the curved surface CV2, the pigment value that is calculated is "2". The pigment value calculation section 650 calculates the pigment value for every effective region of the first image set 652d. Similarly, the pigment value of the second image set 653d is calculated for every effective region.

The pigment value calculated for every effective region of the first image set 652d and the pigment value calculated for every effective region of the second image set 653d are transmitted to the pigment value determination section 660 of the correction determination section 230. The value of an X component, the value of a Y component, and the value of a Z component of the coordinates (X3, Y3, Z3) that are calculated for every effective region of the first image set 652d and the value of an X component, the value of a Y component, and the value of a Z component of the coordinates (X3, Y3, Z3) that are calculated for every effective region of the second image set 653d are preferably transmitted to the pigment value determination section 660.

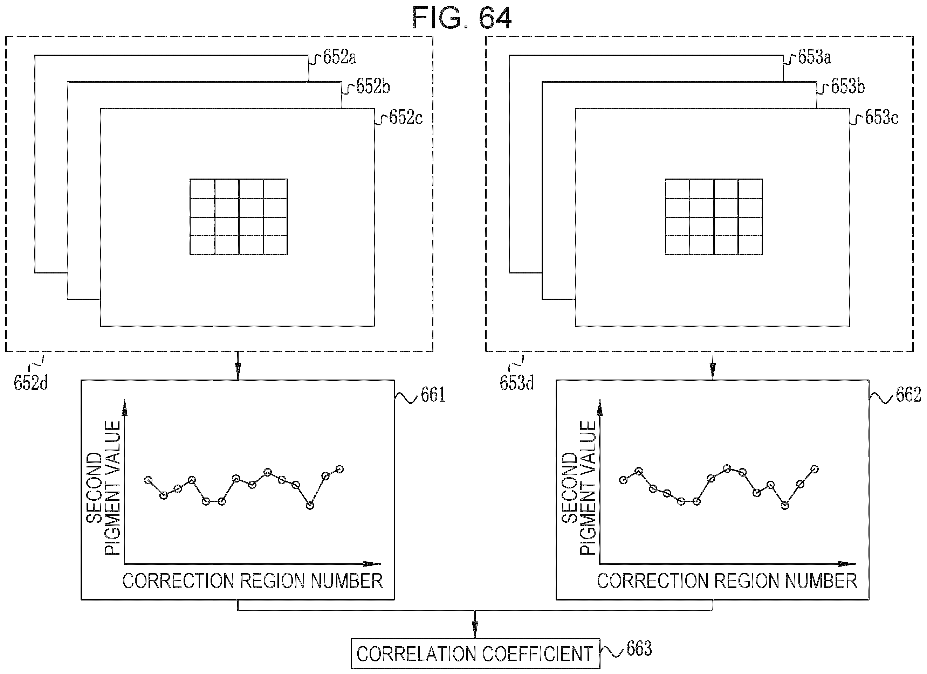

As illustrated in Fig. 64, the pigment value determination section 660 obtains a correlation coefficient 663 between a pigment value 661 calculated for every effective region of the first image set 652d and a pigment value 662 calculated for every effective region of the second image set 653d. In Fig. 64, as for the pigment values 661 and 662 that are calculated for every effective region, the numbers of the correction regions (that is, the "N" number of the N-th correction region) with a vertical axis assigned to the pigment values and a lateral axis assigned to the effective region for description.

In the case where the correlation coefficient is less than a predetermined movement determination threshold value, the pigment value determination section 660 determines that the "degree of movement of the endoscope is high". In the case where the correlation coefficient is more than the movement determination threshold value, it is determined that the "degree of movement of the endoscope is low ". In this case, the pigment value determination section 660 outputs and transmits the determination result that represents that the "the degree of movement of the endoscope is high" or the "degree of movement of the endoscope is low" as a movement determination result to the display control unit 100.

**EP 4 461 202 B1**

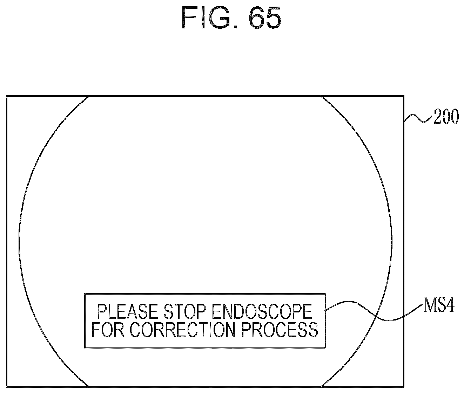

The display control unit 100 preferably changes the display form on the display 15 depending on the movement determination result. For example, in the case where the movement determination result that represents that the "degree of movement of the endoscope is low" is outputted, the display control unit 100 causes the display 15 to display a message that represents that the correction process can be appropriately performed (see Fig. 38). In the case where the movement determination result that represents that the "degree of movement of the endoscope is high" is outputted, the display 15 displays a warning message MS4 that represents "PLEASE STOP ENDOSCOPE FOR CORRECTION PROCESS" illustrated in Fig. 65.

In some cases where the instruction for changing light emission is manually inputted, and the illumination light is changed, the movement of the endoscope 12 increases during changing. In these cases, there is a possibility that the correction process depending on the influence of the concentration of the specific pigment cannot be appropriately performed. In view of this, the degree of movement of the endoscope 12 is determined, a notification is given to the user in the case where the degree of movement is high, and consequently, the user is prompted not to move the endoscope 12. As a result, in the case where the degree of movement of the endoscope 12 is low, the correction process can be appropriately performed.

The image determination result of determination of the degree of the influence of the disturbance and/or the movement determination result of determination of the degree of movement of the endoscope 12 is reported as described above, and consequently, the user can be prompted to input an operation for appropriately performing the correction process. In the correction process, the pigment value is preferably obtained by using a robust estimation method, based on the pigment value in each correction region that is calculated by using the first image set and the second image set to perform a table correction process depending on the pigment value, and the correction process is preferably performed to select the correction oxygen saturation calculation table 86b corresponding to the pigment value. In the case where the movement determination result that represents that the "degree of movement of the endoscope is low" is outputted and in the case where the image determination result that represents that the "reliability in the correction regions as a whole is high", the pigment value may be obtained by using the region average signal ratio in the correction region that is determined as the "highly reliable correction region", and consequently, the correction process may be performed.

According to the embodiment described above, the hardware structures of processors (processing units) that perform various processes such as the central control unit 50, the image signal obtaining section 60, the DSP 61, the noise reducing section 62, the image process change section 63, the normal light image generation section 70, the tissue oxygen saturation image generation section 80, the display control unit 100, and the exposure control unit 110 correspond to various processors described below. The various processors include a central processing unit (CPU) that is a generic processor that runs software (a program) and that functions as various processing units, a graphical processing unit (GPU), a programmable logic device (PLD) that is a processor that can change a circuit structure after manufacture such as a FPGA, and a dedicated electric circuit that is a processor that has a circuit structure that is dedicatedly designed to perform various processes.

A single processing unit may include a single processor among the various processors or may include a combination of the same kind or different kinds of two or more processors (for example, multiple FPGAs, a combination of a CPU and a FPGA, and a combination of a CPU and a GPU). Multiple processing units may include a single processor. A first example in which the multiple processing units include a single processor is a single processor that includes a combination of one or more CPUs and software as represented by a computer such as a client or a server and that functions as the multiple processing units. A second example is a processor that fulfills the function of the entire system including the multiple processing units by using a single integrated circuit (IC) chip as represented by a system on chip (SoC). The hardware structures of the various processing units use one or more processors of the various processors described above.

More specifically, the hardware structures of the various processors correspond to an electric circuit (circuitry) obtained by combining circuit elements such as semiconductor elements. The hardware structure of a storage unit corresponds to a storage device such as a hard disc drive (HDD) or a solid state drive (SSD).

Reference Signs List

10 endoscope system
12 endoscope
12a insertion section
12b operation section
12c bending portion
12d tip portion
12e angle knob

12f mode change switch
12h still image obtaining instruction switch
12i zoom operation section
12j forceps port
13 light source device
14 processor device
15 display
16 user interface
20 light source unit
20a V-LED
20b BS-LED
20c BL-ELD
20d G-LED
20e R-LED
21 light source control unit
22 optical path coupler
23 light guide
30 illumination optical system
31 illumination lens
40 image pick-up optical system
41 objective lens
42 zoom lens
43,511,512,513,514, 611, 612 image pick-up sensor
44 image pick-up control section
45 CDS/AGC circuit
46 A/D convertor
47 aperture diaphragm
48 shutter
50 central control unit
60 image signal obtaining section
61 DSP
62 noise reducing section
63 image process change section
70 normal light image generation section
80 tissue oxygen saturation image generation section
81 substantially white light image generation section
82 base image generation section
83 signal ratio calculation section
84 oxygen saturation calculation section
84a oxygen saturation calculation table
86b correction oxygen saturation calculation table
84b, 84c contour
84d, 84e light absorption coefficient graph
85 oxygen saturation image generation section
86 correction oxygen saturation calculation section
86a pigment value calculation table
86c, 86d, 86e, 86f, 86g curved surface
87 correction oxygen saturation image generation section
100 display control unit
111 observation image signal
110 exposure control unit
120 exposure control signal generation section
130 control amount output section
140 brightness calculation section
141 analysis region setting section
141a endoscope image
141b, 141c region in the endoscope image
141d, 141e, 141f region in an analysis image

142 region brightness calculation section
150 brightness difference calculation section
151 first analysis image
151a first region
152 observation image signal
153 second analysis image
153a second region
154 correction image signal
200 correction image
201 correction specific region
201a insufficiently reliable region
201b highly reliable region
202 border
210 correction image generation section
220 reliability calculation section
221 first reliability calculation table
222 second reliability calculation table
223 third reliability calculation table
230 correction determination section
400 wide-range light source
410 rotation filter
411 inner filter
411a, 412a B1 filter
411b, 412c G filter
411c, 412d R filter
412 outer filter
412b B2 filter
412e B3 filter
420 filter change section
430 two-dimensional coordinate system
431a reference line
431b actual measurement line
500, 600 camera head
501,502,503, 601 dichroic mirror
620 characteristic value calculation section
621, 652a, 653a substantially white light image
622, 654, 654d, 654h, 655, 655d, 655h, 656, 656d, 656h correction region
622a first correction region
622p sixteenth correction region
630 region reliability calculation section
640 region reliability determination section
650 pigment value calculation section
601a reflectance
601b light transmittance
651a, 651b, 651c, 651d, 651e, 651fframe
652b, 653b second blue light image
652c, 653c correction illumination light image
652d first image set
653d second image set
660 pigment value determination section
661, 662 pigment value
663 correlation coefficient

**Claims**

1. An endoscope system that illuminates an object to be observed and that performs image pick-up of reflection light from the object to be observed, comprising:

a light source device (13) that causes at least one or more light sources to produce light and that emits observation illumination light and correction illumination light having a spectrum that differs from a spectrum of the observation illumination light toward the object to be observed;

an image pick-up optical system (40) that performs image pick-up of the reflection light; and

a processor (14),

wherein the processor is configured to:

obtain image signals of multiple kinds different from each other in a first exposure period in which the observation illumination light is emitted and in a second exposure period in which the correction illumination light is emitted;

**characterized in that** the processor is further configured to:

calculate image signal brightness from image signals obtained with the observation light and calculate image signal brightness from image signals obtained with the correction light;

output a control amount for the image signals obtained with the observation light that changes depending on the image signal brightness and output a control amount for the image signals obtained with the correction light that changes depending on the image signal brightness;

generate exposure control signals for the image signals obtained with the observation light and for for the image signals obtained with the correction light having the control amounts different from each other; and

control the at least one or more light sources and the image pick-up optical system depending on the exposure control signals.

2. The endoscope system according to claim 1,
wherein the control amount is outputted based on a difference between the image signal brightness and predetermined target brightness.

3. The endoscope system according to claim 2,
wherein the processor is configured to:

obtain observation image signals as the image signals in the first exposure period;

obtain correction image signals as the image signals in the second exposure period;

output a first control amount as the control amount, based on a difference between first image signal brightness that is the image signal brightness calculated by using the observation image signals and first target brightness that is the target brightness;

output a second control amount as the control amount that differs from the first control amount, based on a difference between second image signal brightness that is the image signal brightness calculated by using the correction image signals and second target brightness that is the target brightness;

generate first exposure control signals as the exposure control signals based on the first control amount; and

generate second exposure control signals as the exposure control signals based on the second control amount.

4. The endoscope system according to claim 3,
wherein the second control amount is larger than the first control amount.

5. The endoscope system according to claim 3 or 4,
wherein the processor is configured to calculate the image signal brightness by using a specific color signal among the image signals.

6. The endoscope system according to claim 5,
wherein the specific color signal is a B signal.

7. The endoscope system according to claim 3 or 4,
wherein the processor is configured to:

generate an analysis image by using the image signals;

extract a specific region from the analysis image; and

calculate the image signal brightness by using the image signals in the specific region.

8. The endoscope system according to claim 7,
wherein the specific region is extracted so as to follow a specific shape.

**9.** The endoscope system according to claim 8,
wherein the processor is configured to:

extract a first region as the specific region from a first analysis image that is the analysis image generated by using the observation image signals;
output a first region control amount as the control amount, based on a difference between first region brightness calculated as the image signal brightness by using the first region and the first target brightness;
extract a second region as the specific region from a second analysis image that is the analysis image generated by using the correction image signals;
output a second region control amount as the control amount, based on a difference between second region brightness calculated as the image signal brightness by using the second region and the second target brightness;
generate first region exposure control signals as the exposure control signals based on the first region control amount; and
generate second region exposure control signals as the exposure control signals based on the second region control amount.

**10.** The endoscope system according to claim 3,
wherein the processor is configured to:

switch between an observation mode in which the observation image signals are obtained and a correction mode in which the observation image signals and the correction image signals are obtained;
generate the first exposure control signals in the observation mode; and
generate the first exposure control signals and the second exposure control signals in the correction mode.

**11.** The endoscope system according to claim 3,

wherein the first exposure period includes a first illumination period in which substantially white light included in the observation illumination light is emitted and a second illumination period in which calculation illumination light included in the observation illumination light is emitted,
wherein the second exposure period includes a third illumination period in which the correction illumination light is emitted, and
wherein the processor is configured to:

automatically switch between the first illumination period, the second illumination period, and the third illumination period;
obtain substantially white light image signals as the image signals in the first illumination period;
obtain calculation image signals as the image signals in the second illumination period;
obtain the correction image signals in the third illumination period;
generate first A exposure control signals as the exposure control signals based on the substantially white light image signals;
generate first B exposure control signals as the exposure control signals based on the calculation image signals;
generate the second exposure control signals based on the correction image signals; and
control the at least one or more light sources and the image pick-up optical system depending on the first A exposure control signals, the first B exposure control signals, and the second exposure control signals.

**12.** The endoscope system according to claim 1,

wherein control on the at least one or more light sources includes control on an amount of light, and
wherein control on the image pick-up optical system includes control on an aperture diaphragm value, control on an exposure time, and control on a gain.

**Patentansprüche**

**1.** Endoskopsystem, das ein zu beobachtendes Objekt beleuchtet und das Bildaufnahme von Reflexionslicht von dem zu beobachtenden Objekt durchführt, umfassend:

eine Lichtquellenvorrichtung (13), die mindestens eine oder mehrere Lichtquellen veranlasst, Licht zu produzieren, und die Beobachtungsbeleuchtungslicht und Korrekturbeleuchtungslicht, das ein Spektrum aufweist, das sich von einem Spektrum des Beobachtungsbeleuchtungslichts unterscheidet, zu dem zu beobachtenden Objekt hin emittiert;

ein optisches Bildaufnahmesystem (40), das Bildaufnahme des Reflexionslichts durchführt; und

einen Prozessor (14),

wobei der Prozessor so konfiguriert ist, dass er:

Bildsignale von mehreren Arten, die sich voneinander unterscheiden, in einer ersten Belichtungsperiode, in der das Beobachtungsbeleuchtungslicht emittiert wird, und in einer zweiten Belichtungsperiode, in der das Korrekturbeleuchtungslicht emittiert wird, erhält;

**dadurch gekennzeichnet, dass** der Prozessor ferner so konfiguriert ist, dass er:

Bildsignalhelligkeit aus Bildsignalen, die mit dem Beobachtungslicht erhalten werden, berechnet und Bildsignalhelligkeit aus Bildsignalen, die mit dem Korrekturlicht erhalten werden, berechnet;

eine Steuergröße für die mit dem Beobachtungslicht erhaltenen Bildsignale, die sich in Abhängigkeit von der Bildsignalhelligkeit ändert, ausgibt und eine Steuergröße für die mit dem Korrekturlicht erhaltenen Bildsignale, die sich in Abhängigkeit von der Bildsignalhelligkeit ändert, ausgibt;

Belichtungssteuersignale für die mit dem Beobachtungslicht erhaltenen Bildsignale und für die mit dem Korrekturlicht erhaltenen Bildsignale, die die Steuergrößen aufweisen, die sich voneinander unterscheiden, erzeugt;

und

die mindestens eine oder mehreren Lichtquellen und das optische Bildaufnahmesystem in Abhängigkeit von den Belichtungssteuersignalen steuert.

2. Endoskopsystem nach Anspruch 1,
wobei die Steuergröße auf der Grundlage einer Differenz zwischen der Bildsignalhelligkeit und vorbestimmter Zielhelligkeit ausgegeben wird.

3. Endoskopsystem nach Anspruch 2,
wobei der Prozessor so konfiguriert ist, dass er:

Beobachtungsbildsignale als die Bildsignale in der ersten Belichtungsperiode erhält;

Korrekturbildsignale als die Bildsignale in der zweiten Belichtungsperiode erhält;

eine erste Steuergröße als die Steuergröße auf der Grundlage einer Differenz zwischen erster Bildsignalhelligkeit, die die Bildsignalhelligkeit ist, die durch Verwenden der Beobachtungsbildsignale berechnet wird, und erster Zielhelligkeit, die die Zielhelligkeit ist, ausgibt;

eine zweite Steuergröße als die Steuergröße, die sich von der ersten Steuergröße unterscheidet, auf der Grundlage einer Differenz zwischen zweiter Bildsignalhelligkeit, die die Bildsignalhelligkeit ist, die durch Verwenden der Korrekturbildsignale berechnet wird, und zweiter Zielhelligkeit, die die Zielhelligkeit ist, ausgibt;

erste Belichtungssteuersignale als die Belichtungssteuersignale auf der Grundlage der ersten Steuergröße erzeugt; und

zweite Belichtungssteuersignale als die Belichtungssteuersignale auf der Grundlage der zweiten Steuergröße erzeugt.

4. Endoskopsystem nach Anspruch 3,
wobei die zweite Steuergröße größer als die erste Steuergröße ist.

5. Endoskopsystem nach Anspruch 3 oder 4,
wobei der Prozessor so konfiguriert ist, dass er die Bildsignalhelligkeit durch Verwenden eines spezifischen Farbsignals unter den Bildsignalen berechnet.

6. Endoskopsystem nach Anspruch 5,
wobei das spezifische Farbsignal ein B-Signal ist.

7. Endoskopsystem nach Anspruch 3 oder 4,
wobei der Prozessor so konfiguriert ist, dass er:

ein Analysebild durch Verwenden der Bildsignale erzeugt;

einen spezifischen Bereich aus dem Analysebild extrahiert; und

die Bildsignalhelligkeit durch Verwenden der Bildsignale in dem spezifischen Bereich berechnet.

8. Endoskopsystem nach Anspruch 7,

wobei der spezifische Bereich so extrahiert wird, dass er einer spezifischen Form folgt.

9. Endoskopsystem nach Anspruch 8,

wobei der Prozessor so konfiguriert ist, dass er:

einen ersten Bereich als den spezifischen Bereich aus einem ersten Analysebild, das das Analysebild ist, das durch Verwenden der Beobachtungsbildsignale erzeugt wird, extrahiert;

eine Steuergröße für ersten Bereich als die Steuergröße auf der Grundlage einer Differenz zwischen Helligkeit von erstem Bereich, die als die Bildsignalhelligkeit durch Verwenden des ersten Bereichs berechnet wird, und der ersten Zielhelligkeit ausgibt;

einen zweiten Bereich als den spezifischen Bereich aus einem zweiten Analysebild, das das Analysebild ist, das durch Verwenden der Korrekturbildsignale erzeugt wird, extrahiert;

eine Steuergröße für zweiten Bereich als die Steuergröße auf der Grundlage einer Differenz zwischen Helligkeit von zweitem Bereich, die als die Bildsignalhelligkeit durch Verwenden des zweiten Bereichs berechnet wird, und der zweiten Zielhelligkeit ausgibt;

Belichtungssteuersignale für ersten Bereich als die Belichtungssteuersignale auf der Grundlage der Steuergröße für ersten Bereich erzeugt; und

Belichtungssteuersignale für zweiten Bereich als die Belichtungssteuersignale auf der Grundlage der Steuergröße für zweiten Bereich erzeugt.

10. Endoskopsystem nach Anspruch 3,

wobei der Prozessor so konfiguriert ist, dass er:

zwischen einem Beobachtungsmodus, in dem die Beobachtungsbildsignale erhalten werden, und einem Korrekturmodus, in dem die Beobachtungsbildsignale und die Korrekturbildsignale erhalten werden, umschaltet;

die ersten Belichtungssteuersignale in dem Beobachtungsmodus erzeugt; und

die ersten Belichtungssteuersignale und die zweiten Belichtungssteuersignale in dem Korrekturmodus erzeugt.

11. Endoskopsystem nach Anspruch 3,

wobei die erste Belichtungsperiode eine erste Beleuchtungsperiode, in der im Wesentlichen weißes Licht, das in dem Beobachtungsbeleuchtungslicht enthalten ist, emittiert wird, und eine zweite Beleuchtungsperiode, in der Berechnungsbeleuchtungslicht, das in dem Beobachtungsbeleuchtungslicht enthalten ist, emittiert wird, enthält,

wobei die zweite Belichtungsperiode eine dritte Beleuchtungsperiode, in der das Korrekturbeleuchtungslicht emittiert wird, enthält, und

wobei der Prozessor so konfiguriert ist, dass er:

zwischen der ersten Beleuchtungsperiode, der zweiten Beleuchtungsperiode und der dritten Beleuchtungsperiode automatisch umschaltet;

Bildsignale von im Wesentlichen weißem Licht als die Bildsignale in der ersten Beleuchtungsperiode erhält;

Berechnungsbildsignale als die Bildsignale in der zweiten Beleuchtungsperiode erhält; Korrekturbildsignale in der dritten Beleuchtungsperiode erhält;

erste A-Belichtungssteuersignale als die Belichtungssteuersignale auf der Grundlage der Bildsignale von im Wesentlichen weißem Licht erzeugt;

erste B-Belichtungssteuersignale als die Belichtungssteuersignale auf der Grundlage der Berechnungsbildsignale erzeugt;

die zweiten Belichtungssteuersignale auf der Grundlage der Korrekturbildsignale erzeugt; und

die mindestens eine oder mehreren Lichtquellen und das optische Bildaufnahmesystem in Abhängigkeit von den ersten A-Belichtungssteuersignalen, den ersten B-Belichtungssteuersignalen und den zweiten Belichtungssteuersignalen steuert.

12. Endoskopsystem nach Anspruch 1,

**EP 4 461 202 B1**

wobei Steuerung der mindestens einen oder mehreren Lichtquellen Steuerung einer Lichtmenge enthält, und wobei Steuerung des optischen Bildaufnahmesystems Steuerung eines Blendenwerts, Steuerung einer Belichtungszeit und Steuerung einer Verstärkung enthält.

**Revendications**

1. Système d'endoscope qui éclaire un objet à observer et qui effectue une prise d'images de la lumière de réflexion par l'objet à observer, comprenant :

   un dispositif de source de lumière (13) qui amène au moins une ou plusieurs sources de lumière à produire de la lumière et qui émet une lumière d'éclairage d'observation et une lumière d'éclairage de correction ayant un spectre qui diffère d'un spectre de la lumière d'éclairage d'observation vers l'objet à observer ;
   un système optique de prise d'images (40) qui effectue une prise d'images de la lumière de réflexion ; et
   un processeur (14),
   dans lequel le processeur est configuré pour :

      obtenir des signaux d'images de plusieurs types différents les uns des autres lors d'une première période d'exposition durant laquelle la lumière d'éclairage d'observation est émise et lors d'une deuxième période d'exposition durant laquelle la lumière d'éclairage de correction est émise ;
      **caractérisé en ce que** le processeur est en outre configuré pour :

         calculer une luminosité de signal d'images à partir de signaux d'images obtenus avec la lumière d'observation et calculer une luminosité de signal d'images à partir de signaux d'images obtenus avec la lumière de correction ;
         produire une quantité de contrôle pour les signaux d'images obtenus avec la lumière d'observation qui change en fonction de la luminosité de signal d'images et produire une quantité de contrôle pour les signaux d'images obtenus avec la lumière de correction qui change en fonction de la luminosité de signal d'images ;
         générer des signaux de contrôle d'exposition pour les signaux d'images obtenus avec la lumière d'observation et pour les signaux d'images obtenus avec la lumière de correction ayant les quantités de contrôle différentes les unes des autres ;
         et
         contrôler l'au moins une ou plusieurs sources de lumière et le système optique de prise d'images en fonction des signaux de contrôle d'exposition.

2. Système d'endoscope selon la revendication 1,
   dans lequel la quantité de contrôle est produite sur la base d'une différence entre la luminosité de signal d'images et une luminosité cible prédéterminée.

3. Système d'endoscope selon la revendication 2,
   dans lequel le processeur est configuré pour :

      obtenir des signaux d'images d'observation en tant que signaux d'images lors de la première période d'exposition ;
      obtenir des signaux d'images de correction en tant que signaux d'images lors de la deuxième période d'exposition ;
      produire une première quantité de contrôle en tant que quantité de contrôle, sur la base d'une différence entre une première luminosité de signal d'images qui est la luminosité de signal d'images calculée en utilisant les signaux d'images d'observation et une première luminosité cible qui est la luminosité cible ;
      produire une deuxième quantité de contrôle en tant que quantité de contrôle qui diffère de la première quantité de contrôle, sur la base d'une différence entre une deuxième luminosité de signal d'images qui est la luminosité de signal d'images calculée en utilisant les signaux d'images de correction et une deuxième luminosité cible qui est la luminosité cible ;
      générer des premiers signaux de contrôle d'exposition en tant que signaux de contrôle d'exposition sur la base de la première quantité de contrôle ; et
      générer des deuxièmes signaux de contrôle d'exposition en tant que signaux de contrôle d'exposition sur la base de la deuxième quantité de contrôle.

**4.** Système d'endoscope selon la revendication 3,
dans lequel la deuxième quantité de contrôle est supérieure à la première quantité de contrôle.

**5.** Système d'endoscope selon la revendication 3 ou la revendication 4,
dans lequel le processeur est configuré pour calculer la luminosité de signal d'images en utilisant un signal de couleur spécifique parmi les signaux d'images.

**6.** Système d'endoscope selon la revendication 5,
dans lequel le signal de couleur spécifique est un signal B.

**7.** Système d'endoscope selon la revendication 3 ou la revendication 4,
dans lequel le processeur est configuré pour :

générer une image d'analyse en utilisant les signaux d'images ;
extraire une région spécifique de l'image d'analyse ; et
calculer la luminosité de signal d'images en utilisant les signaux d'images dans la région spécifique.

**8.** Système d'endoscope selon la revendication 7,
dans lequel la région spécifique est extraite de manière à suivre une forme spécifique.

**9.** Système d'endoscope selon la revendication 8,
dans lequel le processeur est configuré pour :

extraire une première région en tant que région spécifique à partir d'une première image d'analyse qui est l'image d'analyse générée en utilisant les signaux d'images d'observation ;
produire une quantité de contrôle de première région en tant que quantité de contrôle, sur la base d'une différence entre une luminosité de première région calculée en tant que luminosité de signal d'images en utilisant la première région et la première luminosité cible ;
extraire une deuxième région en tant que région spécifique à partir d'une deuxième image d'analyse qui est l'image d'analyse générée en utilisant les signaux d'images de correction ;
produire une quantité de contrôle de deuxième région en tant que quantité de contrôle, sur la base d'une différence entre une luminosité de deuxième région calculée en tant que luminosité de signal d'images en utilisant la deuxième région et la deuxième luminosité cible ;
générer des signaux de contrôle d'exposition de première région en tant que signaux de contrôle d'exposition sur la base de la quantité de contrôle de première région ; et
générer des signaux de contrôle d'exposition de deuxième région en tant que signaux de contrôle d'exposition sur la base de la quantité de contrôle de deuxième région.

**10.** Système d'endoscope selon la revendication 3,
dans lequel le processeur est configuré pour :

commuter entre un mode d'observation dans lequel les signaux d'images d'observation sont obtenus et un mode de correction dans lequel les signaux d'images d'observation et les signaux d'images de correction sont obtenus ;
générer les premiers signaux de contrôle d'exposition dans le mode d'observation ; et
générer les premiers signaux de contrôle d'exposition et les deuxièmes signaux de contrôle d'exposition dans le mode de correction.

**11.** Système d'endoscope selon la revendication 3,

dans lequel la première période d'exposition inclut une première période d'éclairage durant laquelle une lumière sensiblement blanche incluse dans la lumière d'éclairage d'observation est émise et une deuxième période d'éclairage durant laquelle une lumière d'éclairage de calcul incluse dans la lumière d'éclairage d'observation est émise,
dans lequel la deuxième période d'exposition inclut une troisième période d'éclairage durant laquelle la lumière d'éclairage de correction est émise, et
dans lequel le processeur est configuré pour :

commuter automatiquement entre la première période d'éclairage, la deuxième période d'éclairage et la troisième période d'éclairage ;

obtenir des signaux d'images de lumière sensiblement blanche en tant que signaux d'images lors de la première période d'éclairage ;

obtenir des signaux d'images de calcul en tant que signaux d'images lors de la deuxième période d'éclairage ;

obtenir les signaux d'images de correction lors de la troisième période d'éclairage ;

générer des premiers signaux de contrôle d'exposition A en tant que signaux de contrôle d'exposition sur la base des signaux d'images de lumière sensiblement blanche ;

générer des premiers signaux de contrôle d'exposition B en tant que signaux de contrôle d'exposition sur la base des signaux d'images de calcul ;

générer les deuxièmes signaux de contrôle d'exposition sur la base des signaux d'images de correction ; et contrôler l'au moins une ou plusieurs sources de lumière et le système optique de prise d'images en fonction des premiers signaux de contrôle d'exposition A, des premiers signaux de contrôle d'exposition B et des deuxièmes signaux de contrôle d'exposition.

12. Système d'endoscope selon la revendication 1,

dans lequel le contrôle de l'au moins une ou plusieurs sources de lumière inclut un contrôle d'une quantité de lumière, et

dans lequel le contrôle du système optique de prise d'images inclut un contrôle d'une valeur de diaphragme d'ouverture, un contrôle d'un temps d'exposition et un contrôle d'un gain.

# FIG. 1

FIG. 2

FIG. 3

20 ~

| V-LED | ~20a |
| BS-LED | ~20b |
| BL-LED | ~20c |
| G-LED | ~20d |
| R-LED | ~20e |

FIG. 4

FIG. 5

# FIG. 6

CALCULATION ILLUMINATION LIGHT

INTENSITY OF LIGHT

BL

G

R

WAVELENGTH (nm)

# FIG. 7

CORRECTION ILLUMINATION LIGHT

INTENSITY OF LIGHT

G

WAVELENGTH (nm)

# FIG. 8

TIME →

| EP1 | | EP1 | | EP1 | |
|---|---|---|---|---|---|
| P1 | P2 | P1 | P2 | P1 | P2 |
| F | F | F | F | F | F |
| | | | | | |
| WL | OL | WL | OL | WL | OL |

# FIG. 9

TIME →

| EP1 | | EP2 | EP1 | | EP2 |
|---|---|---|---|---|---|
| P1 | P2 | P3 | P1 | P2 | P3 |
| F | F | F | F | F | F |
| | | | | | |
| WL | OL | CL | WL | OL | CL |

# FIG. 10

# FIG. 11

# FIG. 12

80~ TISSUE OXYGEN SATURATION IMAGE
GENERATION SECTION

81~ SUBSTANTIALLY WHITE LIGHT IMAGE
GENERATION SECTION

82~ BASE IMAGE GENERATION SECTION

83~ SIGNAL RATIO CALCULATION SECTION

84~ OXYGEN SATURATION
CALCULATION SECTION

84a~ OXYGEN SATURATION
CALCULATION TABLE

85~ OXYGEN SATURATION IMAGE
GENERATION SECTION

86~ CORRECTION OXYGEN SATURATION
CALCULATION SECTION

86a~ PIGMENT VALUE
CALCULATION TABLE

86b~ CORRECTION OXYGEN
SATURATION
CALCULATION TABLE

87~ CORRECTION OXYGEN SATURATION
IMAGE GENERATION SECTION

# FIG. 13

# FIG. 14

# FIG. 15

# FIG. 16

# FIG. 17

# FIG. 18

# FIG. 19

TIME

|  | EP1 | | EP2 | EP1 | | EP2 |
|---|---|---|---|---|---|---|
|  | F | F | F | F | F | F |
| ILLUMINATION LIGHT | WL | OL | CL | WL | OL | CL |

ILLUMINATION LIGHT — WL | OL | CL | WL | OL | CL

IMAGE SIGNAL — 111 — IS1 | IS2 | IS3

IMAGE SIGNAL BRIGHTNESS — Y1 | Y2

CONTROL AMOUNT — CQ1 | CQ2

EXPOSURE CONTROL SIGNAL — ECS1 | ECS2 | EC1 | EC2

EP 4 461 202 B1

FIG. 20

# FIG. 21

140 — BRIGHTNESS CALCULATION SECTION

141 — ANALYSIS REGION SETTING SECTION

142 — REGION BRIGHTNESS CALCULATION SECTION

# FIG. 22

141a

141b

141c

# FIG. 23

141a

141d

# FIG. 24

141a

141e

# FIG. 25

141a

141f

# FIG. 26

OBSERVATION ANALYSIS IMAGE
IN PREVIOUS FRAME

ENDOSCOPE IMAGE IN SUBSEQUENT FRAME
(OBSERVATION ILLUMINATION LIGHT)

EP 4 461 202 B1

FIG. 27

CORRECTION ANALYSIS IMAGE IN PREVIOUS FRAME

153

153a

ENDOSCOPE IMAGE IN SUBSEQUENT FRAME (CORRECTION ILLUMINATION LIGHT)

154

# FIG. 28

EP 4 461 202 B1

FIG. 29

EP 4 461 202 B1

## FIG. 30

START

S101 — IMAGE SIGNAL IS OBTAINED

S102 — IMAGE SIGNAL BRIGHTNESS IS CALCULATED

S103 — BRIGHTNESS DIFFERENCE IS CALCULATED

S104 — CONTROL AMOUNT IS OUTPUTTED

S105 — EXPOSURE CONTROL SIGNAL IS GENERATED

S106 — EXPOSURE CONTROL

END

## FIG. 31

# FIG. 32

80

## TISSUE OXYGEN SATURATION IMAGE GENERATION SECTION

81 — SUBSTANTIALLY WHITE LIGHT IMAGE GENERATION SECTION

82 — BASE IMAGE GENERATION SECTION

83 — SIGNAL RATIO CALCULATION SECTION

84 — OXYGEN SATURATION CALCULATION SECTION

84a — OXYGEN SATURATION CALCULATION TABLE

85 — OXYGEN SATURATION IMAGE GENERATION SECTION

86 — CORRECTION OXYGEN SATURATION CALCULATION SECTION

86a — PIGMENT VALUE CALCULATION TABLE

86b — CORRECTION OXYGEN SATURATION CALCULATION TABLE

87 — CORRECTION OXYGEN SATURATION IMAGE GENERATION SECTION

CORRECTION IMAGE GENERATION SECTION — 210

RELIABILITY CALCULATION SECTION — 220

CORRECTION DETERMINATION SECTION — 230

# FIG. 33

RELIABILITY

1

221

Rx

G2 IMAGE SIGNAL
SIGNAL VALUE

# FIG. 34

$\ln(B1/G1)$

DFX

222

$\ln(R1/G1)$

# FIG. 35

# FIG. 36

FIG. 37

200

201

202

FIG. 38

200

CORRECTION PROCESS
IS APPROPRIATELY
PERFORMED

MS1

FIG. 39

200

PLEASE OPERATE
ENDOSCOPE FOR
CORRECTION PROCESS

MS2

PLEASE AVOID
DARK REGION

MS3

FIG. 40

## FIG. 41

## FIG. 42

# FIG. 43

EP 4 461 202 B1

# FIG. 44

# FIG. 45

# FIG. 46

# FIG. 47

# FIG. 48

# FIG. 49

# FIG. 50

FIG. 51A

FIG. 51B

FIG. 51C

FIG. 52A

FIG. 52B

FIG. 52C

FIG. 53

TIME

| F | F | F |
|---|---|---|
| ML | ML | ML |

FIG. 54

TIME

| F | F | F | F | F | F | | | F | F |
|---|---|---|---|---|---|---|---|---|---|
| ML | ML | NL | NL | CL | CL | NL | NL | ML | ML |

**FIG. 55A**

**FIG. 55B**

**FIG. 55C**

FIG. 56A

FIG. 56B

FIG. 56C

# FIG. 57

EP 4 461 202 B1

FIG. 58A

FIG. 58B

FIG. 59

# FIG. 60

# FIG. 61

# FIG. 62

# FIG. 63

FIG. 64

# FIG. 65

200

PLEASE STOP ENDOSCOPE
FOR CORRECTION PROCESS

MS4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6412252 B

- JP 2013188365 A